(19) Europäisches Patentamt
European Patent Office
Office européen des brevets

(11) EP 0 491 243 B1

(12) EUROPÄISCHE PATENTSCHRIFT

(45) Veröffentlichungstag und Bekanntmachung des Hinweises auf die Patenterteilung:
**15.05.1996 Patentblatt 1996/20**

(21) Anmeldenummer: **91120914.6**

(22) Anmeldetag: **05.12.1991**

(51) Int Cl.[6]: **C07D 295/12**, C07C 217/28, C07C 217/42, C07C 323/26, C07C 323/32, A61K 31/13, A61K 31/535

(54) **Alkylaminoalkylamin- und -äther- Verbindungen sowie Verfahren und Zwischenprodukte zu ihrer Herstellung und diese Verbindungen enthaltende Arzneimittel**

Alkylaminoalkylamino and -ether compounds as well as processes and intermediates for their preparation, and medicaments containing these compounds

Composés alkylaminoalkylamines et -éthers ainsi que des procédés et composés intermédiaires pour leur préparation et médicaments contenant ces composés

(84) Benannte Vertragsstaaten:
**AT BE CH DE DK ES FR GB GR IT LI LU NL SE**

(30) Priorität: **19.12.1990 DE 4040633**
**18.09.1991 DE 4130947**

(43) Veröffentlichungstag der Anmeldung:
**24.06.1992 Patentblatt 1992/26**

(73) Patentinhaber: **Kali-Chemie Pharma GmbH**
**D-30173 Hannover (DE)**

(72) Erfinder:
- **Krähling, Hermann**
  **W-3163 Sehnde 1 (DE)**
- **David, Samuel**
  **W-3000 Hannover 1 (DE)**
- **Hell, Insa, Dr.**
  **W-3000 Hannover 1 (DE)**
- **Preuschoff, Ulf**
  **W-3014 Laatzen (DE)**
- **Ban, Ivan**
  **W-3000 Hannover 72 (DE)**
- **Christen, Marie-Odile**
  **F-75016 Paris (FR)**

(74) Vertreter: **Lauer, Dieter, Dr.**
**Solvay Pharma Deutschland GmbH**
**Hans-Böckler-Allee 20**
**D-30173 Hannover (DE)**

(56) Entgegenhaltungen:
- **CHEMICAL ABSTRACTS, vol. 81, no. 21, 25. November 1974, Columbus, Ohio, US; abstract no. 130790R, R. BARONNET ET AL.: 'Synthesis and comparative pharmacology ...' Seite 8; & Eur. J. Med. Chem. - Chim. Ther. 1974, 9(2), 182-7**
- **CHEMICAL ABSTRACTS, vol. 90, no. 11, 12. März 1979, Columbus, Ohio, US; abstract no. 80704D, O. FOUSSARD-BLANPIN ET AL.: 'Comparative pharmacodynamic study ...' Seite 21; & Ann. Pharm. Fr. 1978, 36(5-6) 273-8**
- **CHEMICAL ABSTRACTS, vol. 99, no. 25, 19. Dezember 1983, Columbus, Ohio, US; abstract no. 212722F, T.S. RAIKOVA ET AL.: 'Synthesis and structure of fenchol amino esters. II.' Seite 663 ; & Khim. Prir. Soedin. 1983, (3), 305-8**
- **CHEMICAL ABSTRACTS, vol. 70, no. 7, 17. Februar 1969, Columbus, Ohio, US; abstract no. 29070D, G. MINARDI ET AL.: 'N-Bornylethylenediamines and N-(omega-guanidinoalkyl)bornylamines' Seite 339; & Farmaco, Ed. Sci. 1968, 23(11), 1040-58**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 491 243 B1

Printed by Jouve, 75001 PARIS (FR)

## Beschreibung

Die vorliegende Erfindung betrifft neue Dialkylaminderivate, welche einen durch eine einen mono- oder bicyclischen Kohlenwasserstoffrest enthaltende Alkoxy- oder Alkylthiogruppe substituierten Alkylrest und einen eine Amin- oder Ätherfunktion tragenden Alkylrest enthalten, sowie diese Verbindungen enthaltende pharmazeutische Zubereitungen und Verfahren und Zwischenprodukte zur Herstellung dieser Verbindungen.

In einem Artikel von Baronnet et al, in Eur. J. Med. Chem.-Chimica Therapeutica 9 (1974), 182-187 sind quarternäre Ammoniumderivate von 2-{2-[2-(Dialkylamino)-äthoxy]-äthyl}-6,6-dimethylnorpinanen mit papaverinartigen, spasmolytischen Eigenschaften beschrieben.

Der vorliegenden Erfindung liegt die Aufgabe zugrunde, neue Dialkylamino-Verbindungen mit wertvollen pharmakologischen Eigenschaften herzustellen.

Es wurde nun gefunden, daß die neuen Alkylaminoalkylamin- und -äther-Verbindungen, welche am Alkylrest durch Alkyloxy- oder Alkylthiogruppen substituiert sind, welche mono- oder bicyclische, von Terpenen abgeleitete Kohlenwasserstoffreste enthalten, wertvolle pharmakologische Eigenschaften besitzen und insbesondere eine günstige pharmakologische Wirkung im gastrointestinalen Trakt haben. Siezeichnen sich insbesondere durch gastrointestinal wirksame spasmolytische Eigenschaften bei guter Verträglichkeit und geringer Toxizität aus. Daneben besitzen sie auch gastroprotektive und ulkushemmende Eigenschaften.

Die vorliegende Erfindung betrifft daher neue Dialkylamino-verbindungen der allgemeinen Formel I
(siehe Formel I)
worin

n für 2-5 steht,
m für 2-6 steht,
$R^1$ Wasserstoff oder Alkyl mit 1 - 4 Kohlenstoffatomen bedeutet,
$R^2$ für eine $OR^4$-Gruppe steht, worin $R^4$ Alkyl mit 1 - 4 Kohlenstoffatomen oder eine gegebenenfalls im Phenylring durch 1 - 3 Substituenten aus der Gruppe Alkyl mit 1 - 4 Kohlenstoffatomen, Alkoxy mit 1 - 4 Kohlenstoffatomen, Halogen und Alkylendioxy mit 1 - 2 Kohlenstoffatomen substituierte Phenyl- oder Phenyl-$C_1$-$C_3$-alkylgruppe bedeutet, oder
$R^2$ für eine

```
 R5
 /
N -Gruppe steht,
 \
  R6
```

worin

$R^5$ Wasserstoff, niederes Alkyl mit 1 - 4 Kohlenstoffatomen oder eine gegebenenfalls im Phenylring durch 1 - 3 Substituenten aus der Gruppe Alkyl mit 1 - 4 Kohlenstoffatomen, Alkoxy mit 1 - 4 Kohlenstoffatomen, Halogen, Trifluormethyl und Alkylendioxy mit 1 - 2 Kohlenstoffatomen substituierte Phenyl- oder Phenyl-$C_1$-$C_3$-alkylgruppe bedeutet, und
$R^6$ Wasserstoff, Alkyl mit 1 - 4 Kohlenstoffatomen oder eine gegebenenfalls im Phenylring durch 1 - 3 Substituenten aus der Gruppe Alkyl mit 1 - 4 Kohlenstoffatomen, Alkoxy mit 1 - 4 Kohlenstoffatomen, Halogen, Trifluormethyl und Alkylendioxy mit 1 - 2 Kohlenstoffatomen substituierte Phenyl- oder Phenyl-$C_1$-$C_3$-alkylgruppe bedeutet, oder
$R^5$ und $R^6$ gemeinsam mit dem Stickstoffatom, an welches sie gebunden sind, einen gesättigten 5- oder 6-gliedrigen Heterocyclus darstellen, welcher gegebenenfalls ein zweites Heteroatom aus der Gruppe Sauerstoff und N-$R^7$, worin $R^7$ Alkyl mit 1 - 4 Kohlenstoffatomen oder Benzyl bedeutet, enthalten kann,
$R^3$ für einen gesättigten mono- oder bicyclischen Terpenkohlenwasserstoffrest mit 10 Kohlenstoffatomen oder für einen bicyclischen Kohlenwasserstoffrest mit 11 Kohlenstoffatomen mit der Formel b
(s. Formel b) steht, und

Z für Sauerstoff oder, falls $R^3$ einen Rest der Formel b bedeutet, auch für Schwefel steht,
und deren physiologisch verträgliche Säureadditionssalze.

In den Verbindungen der Formel I kann $R^1$ Wasserstoff oder vorzugsweise niederes Alkyl bedeuten. Eine niedere Alkylgruppe $R^1$ kann geradkettig oder verzweigt sein und 1 bis 4 Kohlenstoffatome enthalten und stellt insbesondere

die Methylgruppe dar.

Der Rest $R^2$ der Verbindungen der Formel I kann einen Oxyrest $OR^4$ oder einen Aminorest $NR^5R^6$ darstellen. Sofern die Substituenten $R^4$, $R^5$ und/oder $R^6$ niedere Alkylgruppen darstellen, können diese geradkettig oder verzweigt sein und 1 bis 4 Kohlenstoffatome enthalten. Sofern der Rest $R^2$ eine Phenylgruppe enthält, kann diese direkt oder über eine niedere Alkylenkette, welche 1 bis 3, insbesondere 1 oder 2, Kohlenstoffatome enthalten kann, mit dem Sauerstoff- oder Stickstoffatom des Restes $R^2$ verbunden sein. Die Phenylgruppe kann unsubstituiert sein oder aber auch durch 1 - 3 Substituenten aus der Gruppe niederes Alkyl, niederes Alkoxy, niederes Alkylendioxy, Halogen und Trifluormethyl substituiert sein. Niedere Alkyl- oder Alkoxysubstituenten der Phenylgruppe können geradkettig oder verzweigt sein und 1 bis 4, insbesondere 1 oder 2 Kohlenstoffatome enthalten und stellen vorzugsweise Methyl oder Methoxy dar. Niederes Alkylendioxygruppen enthalten 1 - 2 Kohlenstoffatome und sind an 2 benachbarte Kohlenstoffatome der Phenylgruppe gebunden. Für eine Mehrfachsubstitution der Phenylgruppe kommen insbesondere Methoxy und Methylendioxy in Frage. Als Halogensubstituenten der Phenylgruppe kommen insbesondere Fluor, Chlor oder Brom in Frage. Als günstig erweisen sich beispielsweise Verbindungen, worin $R^2$ eine Aminogruppe $NR^5R^6$ darstellt, worin die Substituenten $R^5$ und $R^6$ voneinander unabhängig sein können oder auch $R^5$ und $R^6$ gemeinsam mit dem Stickstoffatom, an welches sie gebunden sind, einen Heterocyclus bilden. Sofern ein solcher Heterocyclus ein zweites Heteroglied enthält, stellt dieses insbesondere Sauerstoff dar. Sofern das Heteroglied eine Iminogruppe $NR^7$ darstellt, bedeutet $R^7$ vorzugsweise eine niedere Alkylgruppe, beispielsweise eine Alkylgruppe mit 1 bis 4 Kohlenstoffatomen, insbesondere Methyl. Sofern die Substituenten $R^5$ und $R^6$ voneinander unabhängige Reste bedeuten, erweist es sich als günstig, wenn mindestens einer dieser Substituenten eine gegebenenfalls im Phenylring substituierte Phenylalkylgruppe mit 1 - 2 Kohlenstoffatomen in der Alkylenkette darstellt. Der zweite Substituent kann zweckmäßig eine niedere Alkylgruppe oder ebenfalls eine gegebenenfalls im Phenylring substituierte Phenyl-$C_1$-$C_2$-alkylgruppe darstellen.

Die Alkylenkette $-(CH_2)_m-$ kann 2 bis 6, vorzugsweise 2 oder 3 Glieder enthalten. Die Alkylenkette $-(CH_2)_n-$ kann 2 bis 5, insbesondere 2 oder 3 Glieder enthalten. Z steht vorzugsweise für Sauerstoff.

Die Kohlenwasserstoff-Reste $R^3$ der Verbindungen der Formel I sind von Terpenen abgeleitete Reste mit 10 oder 11 Kohlenstoffatomen, welche 17 - 19 Wasserstoffatome enthalten können. In den Resten sind jeweils mehrere Chiralitätszentren enthalten, welche jedes R- oder S-konfiguriert sein können, sodaß die Substanzen in mehreren diastereoisomeren Formen auftreten können. Erfindungsgemäß umfassen die Verbindungen der Formel I die einzelnen stereoisomeren Formen der Verbindungen der Formel I und deren Gemische. Bevorzugt enthalten die Verbindungen der Formel I solche Kohlenwasserstoff-Reste $R^3$, welche sich von natürlich vorkommenden Terpenderivaten ableiten, beziehungsweise sich aus diesen herstellen lassen. In der Natur kommen Terpenderivate, z.B. gesättigte Terpenalkohole, als Pflanzeninhaltsstoffe in mehr oder weniger sterisch reiner Form oder als Stereoisomerengemische wechselnder Zusammensetzung vor. Dementsprechend liegen auch käuflich erhältliche Terpenderivate, welche im allgemeinen aus Naturprodukten hergestellt werden, in mehr oder weniger sterisch reiner Form oder als Stereoisomerengemische vor. Im Rahmen der vorliegenden Erfindung kommen insbesondere solche Verbindungen der Formel I in Betracht, in welchen der Kohlenwasserstoff-Rest $R^3$ von natürlichen und/oder käuflich erhältlichen Terpenderivaten abstammt.

Bei der erfindungsgemäßen Herstellung von Verbindungen der Formel I bleibt die Konfiguration des Kohlenwasserstoff-Restes $R^3$ der Ausgangsverbindungen $R^3$-Z-H erhalten, so daß je nach eingesetztem Ausgangsprodukt als Endprodukte der Formel I Stereoisomerengemische oder mehr oder weniger stereoisomerenreine Substanzen der Formel I erhalten werden.

Als Kohlenwasserstoff-Reste $R^3$ eignen sich insbesondere mono- oder bicyclische Kohlenwasserstoffreste mit 10 oder 11 Kohlenstoffatomen aus der Gruppe 1-Methyl-4-isopropylcyclohex-3-yl (= Menthyl) der Formel a, 2-(6,6-Dimethylbicyclo[3.1.1]hept-2-yl) -äthyl (=Dihydronopyl) der Formel b, 6, 6-Dimethylbicyclo [3.1.1] hept -2-ylmethyl (= Myrtanyl) der Formel c, 1,3,3-Trimethylbicyclo[2.2.1]hept-2-yl (= Fenchyl) der Formel d und 1,7,7-Trimethylbicyclo[2.2.1] hept-2-yl (= Bornyl) der Formel e

(siehe Formeln a, b, c, d und e)

Unter den vorgenannten Resten $R^3$ ist der 1-Methyl-4-isopropylcyclohex-3-yl-Rest der Formel a (= Menthylrest) besonders geeignet. Dieser von Menthol abstammende Rest enthält in den Positionen 1, 3 und 4 des Cyclohexylgerüstes Chiralitätszentren, welche jeweils R- oder S-konfiguriert sein können. In der Natur kommen am häufigsten die beiden enantiomeren Formen 1R,3R,4S-1-Methyl-4-isopropylcyclohexan-3-ol (= L-Menthol) und 1S,3S,4R-1-Methyl-4-isopropylcyclohexan-3-ol (=D-Menthol) vor. Auch im Rahmen der vorliegenden Erfindung sind unter den menthylsubstituierten Verbindungen der Formel I diese beiden Menthylformen, insbesondere die L-Menthylform oder Stereoisomerengemische, in denen die L-Menthylform überwiegt, bevorzugt.

Ferner ist als Kohlenwasserstoffrest $R^3$ der 2-(6,6-Dimethylbicyclo[3.1.1]hept-2-yl)-äthyl-Rest der Formel b (= Dihydronopylrest) gut geeignet. Dieser Rest enthält in den Positionen 1, 2 und 5 des Bicycloheptan-Ringgerüstes Chiralitätszentren, welche jeweils R- oder S-konfiguriert sein können. Im Rahmen der vorliegenden Erfindung liegt in den Dihydronopyl-Verbindungen der Formel I bevorzugt eine 2-(6,6-Dimethylbicyclo[3.1.1]hept-2-yl)-äthyl-Gruppierung vor, welche von einem Terpenalkohol stammt, welcher sich von dem natürlichen (-)-β-Pinen (= (1S,5S)-(-)-6,6-Dimethyl-2-methylenbicyclo[3,1,1]heptan) ableitet, in welchem die Chiralitätszentren in 1- und 5-Position S-Konfiguration besitzen. Dementsprechend sind auch in den 2-(6,6-Dimethylbicyclo[3.1.1]hept-2-yl)-äthyl-Derivaten der Formel I die Chiralitätszentren in 1- und 5-Position des Terpenringgerüstes S-konfiguriert, während das Chiralitätszentrum in 2-Position S- oder R-Konfiguration besitzen kann, sodaß die entsprechenden Substanzen der Formel I in zwei diastereomeren Formen auftreten können. Von diesen beiden Dihydronopyl-Formen werden insbesondere die cis-Form, in welcher das Chiralitätszentrum in Position 2 des Bicycloheptan-Ringgerüstes S-Konfiguration besitzt, oder Gemische, in denen diese Form in überwiegendem Maße enthalten ist, eingesetzt.

Als Kohlenwasserstoffreste $R^3$ eignen sich auch der 1,3,3-Trimethylbicyclo[2.2,1]hept-2-yl-Rest der Formel d (= Fenchylrest) und der 1,7,7-Trimethylbicyclo[2.2.1]-hept-2-yl-Rest der Formel e (= Bornylrest).

In dem Fenchylrest $R^3$ sind in den Positionen 1, 2 und 4 des Bicycloheptan-Ringgerüstes Chiralitätszentren, welche jeweils R- oder S-konfiguriert sein können, enthalten. Im Rahmen der vorliegenden Erfindung sind Fenchyl-Reste bevorzugt, welche sich von natürlichem (+)-Fenchol ableiten, in welchem eine 1S,4R-Konfiguration vorliegt, während das Chiralitätszentrum in 2-Position vorzugsweise R-Konfiguration aber auch S-Konfiguration besitzen kann.

Der Bornyl-Rest $R^3$ enthält in den Positionen 1,2 und 4 des Bicycloheptan-Ringgerüstes Chiralitätszentren, welche jeweils R- oder S-konfiguriert sein können. In natürlichen Bornyl-Derivaten, kommt hauptsächlich der Endobornyl-Rest vor. Es überwiegt die (-)-Form (= 1S,2R,4S-Form) gegenüber der (+)-Form (= 1R,2S,4R-Form). Auch im Rahmen der vorliegenden Erfindung werden Bornyl-Derivate bevorzugt, deren Bornyl-Rest von natürlichem Borneol abstammt und in der 1S,2R,4S-Form vorliegt, oder Gemische, in denen diese Form in überwiegendem Maße enthalten ist.

Sofern $R^3$ einen 6,6-Dimethylbicyclo[3.1.1]hept-2-ylmethylrest der Formel c (= Myrtanylrest) darstellt, liegt bevorzugt die sich von dem natürlichen (-)-β-Pinen ableitende Konfiguration vor, in welcher die Chiralitätszentren in 1-und 5-Position S-konfiguriert sind.

Die neuen Dialkylamino-Verbindungen der Formel I zeichnen sich durch interessante pharmakologische Eigenschaften aus.

Insbesondere zeigen die Verbindungen eine regulierende Wirkung auf die Motilität des Magen/Darm-Traktes und besitzen die Fähigkeit, Motilitätsstörungen, insbesondere des Colons, entgegenzuwirken. So werden im Experiment mit isolierten Organen aus dem Magen/Darm-Trakt induzierte Spasmen unter dem Einfluß dieser Verbindungen lysiert und in vivo im Tierexperiment Colonamplituden gedämpft.

A. Bestimmung der spasmolytischen Wirkung in vitro.

In einer physiologischen Salzlösung isolierte Organstreifen aus dem Magen/Darm-Trakt wie Magenfundus, Magenantrum, Duodenum, Ileum, proximales Colon und distales Colon reagieren auf Zugabe eines Spasmogens, z.B. Kaliumchloridlösung, mit Kontraktion. Durch Zugabe der erfindungsgemäßen Verbindungen wird diese durch die Spasmogenzugabe induzierte Kontraktion der Organstreifen gemindert. Das Ausmaß der Rückbildung der Kontraktion ist ein Indiz für die spasmolytische Wirksamkeit der Verbindungen.

Versuchsbeschreibung für die Bestimmung der spasmolytischen Wirkung gegen durch Kaliumchlorid induzierte Spasmen am isolierten Ileum des Meerschweinchens.

Weibliche Meerschweinchen des Stammes Wiga mit einem Körpergewicht von ca. 300 g werden getötet. Ca. 1 cm lange Organstreifen aus dem Ileum werden in ein Organbad aus Tyrode-Lösung*) von 37 °C gegeben und in einer zur isotonischen Messung von Längenänderungen des Organstreifens üblichen Apparatur befestigt. Nach einer Äquilibrierungsphase von einer Stunde werden der Badflüssigkeit zur Überprüfung der Sensitivität der Ileumstreifen 50 m Mol/l Kaliumchlorid zugegeben. Anschließend wird wieder mit Tyrodelösung gespült. Sodann werden zur erneuten Induzierung einer krampfbedingten Kontraktion des Ileumstreifens der Badflüssigkeit 50 mMol/l Kaliumchlorid zugegeben. Sobald die krampfbedingte Verkürzung des Ileumstreifens einen konstanten Maximalwert erreicht hat, wird der Badflüssigkeit die Testsubstanz zugesetzt, und es wird die Abnahme der Verkürzung registriert und als % der konstanten Maximalverkürzung berechnet.

Die folgende Tabelle A gibt nach der vorstehend beschriebenen Testmethode erhaltene Ergebnisse wieder. Die für die Verbindungen der Formel I angegebenen Beispielsnummern beziehen sich auf die nachstehenden Herstellungsbeispiele.

In der Tabelle A wird als $EC_{50}$ diejenige Konzentration in Mol/l der Testsubstanz in der Badflüssigkeit angegeben, mit welcher eine ca. 50 %-ige Verminderung der maximalen durch Kaliumchlorid induzierten krampfhaften Verkürzung des Ileumstreifens erzielt wird.

Die für die Verbindungen der Formel I angegebenen Beispielsnummern beziehen sich auf die nachstehenden Herstellungsbeispiele.

*) Tyrodelösung = wäßrige Lösung enthaltend pro Liter 136,9 mMol NaCl, 2,68 mMol KCl, 2,31 mMol $CaCl_2$, 1,0 mMol $MgCl_2$, 11,9 mMol $NaHCO_3$, 1,45 mMol $NaH_2PO_4$ und 5,55 mMol Glucose.

Tabelle A

| Beispiel Nr. | Spasmolytische Wirkung in vitro gegenüber kalium-induzierter Kontraktion am isolierten Meerschweinchen-Ileum $EC_{50}$ in Mol/l |
|---|---|
| 1 | $12 \times 10^{-6}$ |
| 2 | $2,0 \times 10^{-6}$ |
| 7 | $16 \times 10^{-6}$ |
| 10 | $30 \times 10^{-6}$ |
| 16 | $4,9 \times 10^{-6}$ |
| 19 | $0,54 \times 10^{-6}$ |
| 20 | $7,9 \times 10^{-6}$ |
| 21 | $3,6 \times 10^{-6}$ |
| 22 | $8,7 \times 10^{-6}$ |
| 23 | $1,5 \times 10^{-6}$ |
| 24 | $14 \times 10^{-6}$ |
| 32 | $20 \times 10^{-6}$ |
| 34 | $47 \times 10^{-6}$ |
| 35 | $1,9 \times 10^{-6}$ |
| 36 | $43 \times 10^{-6}$ |
| 37 | $26 \times 10^{-6}$ |
| 38 | $19 \times 10^{-6}$ |
| 44 | $2,4 \times 10^{-6}$ |
| 46 | $4,8 \times 10^{-6}$ |
| 48 | $0,68 \times 10^{-6}$ |
| 50 | $1,3 \times 10^{-6}$ |

B. Bestimmung der Wirkung der Testsubstanzen auf die spontane Motilität im Magen/Darm-Trakt in vivo.

Die Effekte der Testsubstanzen auf die Amplituden und Frequenzen der Magen- und Darmkontraktionen werden nach der folgenden Versuchsmethode gemessen.

Versuchsbeschreibung für die Bestimmung der Wirkung der Testsubstanzen auf die spontane Motilität des Colons in narkotisierten Ratten.

Nüchterne Ratten des Stammes SIV 50 mit einem Körpergewicht von 180 - 250 g werden mit einer Ketamin/Xylazin-Mischung anästhesiert. Die Tiere werden tracheotomiert und laparotomiert. Nach Anlegen einer Pylorusligatur wird eine Magensonde in den Magen eingeführt und am anderen Ende über einen Drei-Wege-Hahn mit einem geeichten Druckaufnehmer (Statham-Element P 23 ID) verbunden. Eine entsprechende Sonde wird rektal 8 - 9 cm tief in das Colon eingeführt und ebenfalls in gleicher Weise mit einem geeichten Druckaufnehmer des gleichen Types verbunden. Anschließend wird der Magen der Tiere über die Sonde mit 2-3 ml Wasser gefüllt. Die Druckschwankungen im Colon werden vor und nach Applikation der Testsubstanzen mit Hilfe eines Watanabe Multicorders (MC 641) aufgezeichnet. Die Differenzen zwischen den mittleren Amplituden vor und nach der Behandlung werden bestimmt, und die durch die Testsubstanzen bewirkte Änderung der mittleren Amplituden wird in Prozent bezogen auf die vor der Behandlung erhaltenen Werte angegeben.

C. Bestimmung der minimalen toxischen Dosis.

Männlichen Mäusen von 20-25 g Gewicht werden per os Maximaldosen von 300 mg/kg der Testsubstanz verabreicht. Die Tiere werden 3 Stunden lang sorgfältig auf Toxizitätssymptome beobachtet. Über einen Zeitraum von 24 Stunden nach der Applikation werden zusätzlich alle Symptome und Todesfälle registriert. Begleitsymptome werden ebenfalls beobachtet und registriert. Wenn Tod oder starke toxische Symptome beobachtet werden, werden weiteren Mäusen zunehmend geringere Dosen verabreicht, bis keine toxischen Symptome mehr auftreten. Die niedrigste Dosis, welche Tod oder starke toxische Symptome hervorruft, wird als minimale toxische Dosis angegeben.

Die folgende Tabelle B gibt nach den vorstehend beschriebenen Testmethoden B und C erhaltene Ergebnisse wieder.

Tabelle B

| Beispiel Nr. | Wirkung auf die spontane Motilität im Colon der Ratte bei einer Dosis von 100 µMol/kg i.p. % Amplitudenverminderung | minimale toxische Dosis in der Maus mg/kg p.o. |
|---|---|---|
| 1 | 55 | >300 |
| 2 | 55 | >300 |
| 19 | 74 | - |
| 20 | 69 | >300 |
| 21 | 62 | 300 |
| 22 | 85 | 100 |
| 48 | 85 | - |

Neben ihren spasmolytischen Wirkungen im gastrointestinalen Trakt haben die Verbindungen auch eine Schutzwirkung auf die gastrointestinale Schleimhaut ausübende, insbesondere ulcushemmende, Eigenschaften.

Aufgrund ihrer Wirkungen im gastrointestinalen Trakt sind die Verbindungen der Formel I in der Gastroenterologie als Arzneimittel für größere Säugetiere, insbesondere Menschen, zur Prophylaxe und Behandlung von Krampfzuständen im Magen/ Darm-Trakt geeignet. So sind die Substanzen beispielsweise nützlich zur Behandlung von funktionellen Motilitätsstörungen des Magen/Darm-Traktes mit Symptomen wie Übelkeit, Bauchbeschwerden, Krampfzuständen des Darmes oder auch anderer intestinaler Organe und Reizdarmsyndrom.

Die zu verwendenden Dosen können individuell verschieden sein und variieren naturgemäß je nach Art des zu behandelnden Zustandes, der verwendeten Substanz und der Applikationsform. Zum Beispiel werden parenterale Formulierungen im allgemeinen weniger Wirkstoffe enthalten als orale Präparate. Im allgemeinen eignen sich jedoch für Applikationen an größeren Säugetieren, insbesondere Menschen, Arzneiformen mit einem Wirkstoffgehalt von 10 bis 200 mg pro Einzeldosis.

Als Heilmittel können die Verbindungen der Formel I mit üblichen pharmazeutischen Hilfsstoffen in galenischen Zubereitungen wie z.B. Tabletten, Kapseln, Suppositorien oder Lösungen enthalten sein. Diese galenischen Zubereitungen können nach an sich bekannten Methoden hergestellt werden unter Verwendung üblicher fester Trägerstoffe wie z.B. Milchzucker, Stärke oder Talkum oder flüssiger Verdünnungsmittel wie z.B. Wasser, fetten Ölen oder flüssigen Paraffinen und unter Verwendung von pharmazeutisch üblichen Hilfsstoffen, beispielsweise Tablettensprengmitteln, Lösungsvermittlern oder Konservierungsmitteln.

Erfindungsgemäß werden die neuen Verbindungen der Formel I und deren Säureadditionssalze erhalten, indem man in an sich bekannter Weise

a) zur Herstellung von Verbindungen der allgemeinen Formel Ia

(s. Formel Ia) worin n, m, $R^2$, $R^3$ und Z obige Bedeutung besitzen und $R^{1'}$ $C_1$-$C_4$-Alkyl bedeutet, Verbindungen der allgemeinen Formel II

(s. Formel II) worin n, m, $R^{1'}$, $R^3$ und Z obige Bedeutung besitzen und X für eine abspaltbare Fluchtgruppe Y steht oder zur Herstellung solcher Verbindungen, worin $R^2$ eine $OR^4$-Gruppe darstellt, auch für Hydroxy stehen kann, umsetzt mit Verbindungen der allgemeinen Formel III

(s. Formel III) worin $R^2$ obige Bedeutung besitzt, oder falls X Hydroxy ist, auch mit einer Verbindung der allgemeinen Formel III'

(s. Formel III') worin $R^4$ obige Bedeutung besitzt und Y für eine abspaltbare Fluchtgruppe steht, oder

b) zur Herstellung von Verbindungen der allgemeinen Formel I, Verbindungen der allgemeinen Formel IV

(s. Formel IV) worin n, m, $R^1$, $R^3$ und Z obige Bedeutung besitzen und D für den Rest $CH_2$-$N_3$, für eine $R^2$-$CH_2$-Gruppe, worin $R^2$ obige Bedeutung besitzt, oder für eine

$R^5$
/
N -CO-Gruppe,
\
$R^6$

worin $R^5$ und $R^6$ obige Bedeutung besitzen, steht, reduziert, oder

c) zur Herstellung von Verbindungen der allgemeinen Formel Ib

(s. Formel Ib) worin n, m, $R^{1'}$, $R^3$ und Z obige Bedeutung besitzen und $R^{2'}$ die für $R^2$ angegebene Bedeutung mit Ausnahme von eine NH-Funktion enthaltenden Resten besitzt, Verbindungen der allgemeinen Formel V

(s. Formel V) worin n, $R^3$ und Z obige Bedeutung besitzen und Q für einen

$R^{1'}$
/
N -Rest,
\
H

worin $R^{1'}$ obige Bedeutung besitzt, oder für eine abspaltbare Fluchtgruppe Y steht, umsetzt mit Verbindungen der allgemeinen Formel VI

(s. Formel VI)

worin m und $R^{2'}$ obige Bedeutung besitzen und Q' für eine abspaltbare Fluchtgruppe Y steht, falls Q den Rest

$R^{1'}$
/
-N bedeutet,
\
H

oder Q' für den Rest

$R^{1'}$
/
-N steht,
\
H

falls Q eine abspaltbare Fluchtgruppe Y bedeutet, oder

d) zur Herstellung von Verbindungen der allgemeinen Formel Ic

(s. Formel Ic) worin $R^{1'}$, $R^{2'}$, $R^3$ und Z obige Bedeutung besitzen und n' und m' die für n und m angegebene Bedeutung besitzen, wobei jedoch, falls $R^{2'}$ für einen $NR^5R^6$-Rest steht, n' und m' zusammen nicht 4 sein können, Verbindungen der allgemeinen Formel VII

(s. Formel VII) worin $R^3$ und Z obige Bedeutung besitzen, umsetzt mit Verbindungen der allgemeinen Formel VIII

(s. Formel VIII) worin n', m', $R^{1'}$, $R^{2'}$ und Y obige Bedeutung besitzen, oder

e) zur Herstellung von Verbindungen der allgemeinen Formel Id

(s. Formel Id) worin n, m, $R^{1'}$, $R^3$ und Z obige Bedeutung besitzen, in Verbindungen der allgemeinen Formel IX

(s. Formel IX) worin n, m, $R^{1'}$, $R^3$ und Z obige Bedeutung besitzen und A einen Azid- oder Phthalimidrest bedeutet, den Rest A in die $NH_2$-Gruppe überführt, oder

f) zur Herstellung von Verbindungen der allgemeinen Formel Ie

(s. Formel Ie) worin n, m und $R^3$ obige Bedeutung besitzen und $R^{2''}$ die für $R^2$ angegebene Bedeutung mit Ausnahme von eine gegebenenfalls substituierte Benzylgruppe enthaltenden Resten besitzt, aus Verbindungen der allgemeinen Formel X

(s. Formel X)

worin n, m, $R^{2''}$ und $R^3$ obige Bedeutung besitzen und B für eine hydrogenolytisch abspaltbare Gruppe steht, die Gruppe B hydrogenolytisch abspaltet, und gewünschtenfalls in erhaltene Verbindungen der allgemeinen Formel I, worin $R^1$ Wasserstoff bedeutet, einen $C_1$-$C_4$-Alkylrest $R^{1'}$ oder in erhaltene Verbindungen der Formel I, worin $R^2$ eine freie NH-Funktion enthält, einen gegebenenfalls durch eine gegebenenfalls durch 1 - 3 Substituenten aus der Gruppe $C_1$-$C_4$ - Alkyl, $C_1$ -$C_4$ - Alkoxy, Halogen, Trifluormethyl und $C_1$-$C_2$-Alkylendioxy substituierte Phenyl-

gruppe substituierten $C_1$-$C_4$ - Alkylrest $R^{5'}$ einführt, und gewünschtenfalls freie Verbindungen der Formel I in ihre Säureadditionssalze überführt oder die Säureadditionssalze in die freien Verbindungen der Formel I überführt.

Die Umsetzung von Verbindungen der Formel II mit Verbindungen der Formel III gemäß Verfahrensvariante a) kann auf an sich bekannte Weise unter zur Alkylierung von Aminen oder Alkoholen üblichen Bedingungen erfolgen. Als Fluchtgruppe Y in den Verbindungen der Formel II oder den Verbindungen der Formel III' kommen bevorzugt Halogene wie Chlor, Brom oder Jod oder auch organische Sulfonsäurereste in Frage, beispielsweise Reste von Niederalkansulfonsäuren wie z.B. Methansulfonsäure oder von aromatischen Sulfonsäuren wie Benzolsulfonsäure oder durch niederes Alkyl oder Halogen substituierten Benzolsulfonsäuren, z.B. Toluolsulfonsäuren oder Brombenzolsulfonsäuren. Die Umsetzung wird zweckmäßig in Lösung unter basischen Bedingungen durchgeführt. Als Lösungsmittel können inerte organische Lösungsmittel oder auch ein Überschuß einer Verbindung der Formel III dienen. Als inerte organische Lösungsmittel eignen sich niedere Alkohole, Dimethylformamid, Acetonitril oder auch Äther, insbesondere cyclische Äther wie Tetrahydrofuran, oder aromatische Kohlenwasserstoffe wie Benzol oder Toluol oder Gemische aus den vorgenannten Lösungsmitteln. Sofern die Verbindung der Formel III einen niederen Alkohol darstellt, werden bevorzugt aprotische Lösungsmittel eingesetzt oder es kann auch ein Überschuß dieses Alkoholes, gegebenenfalls im Gemisch mit Wasser, als Lösungsmittel dienen. Zweckmäßigerweise wird die Reaktion in Gegenwart einer mindestens equivalenten Menge einer säurebindenden Base durchgeführt. Beispiele von bei der Aminoalkylierung geeigneten Basen sind Alkalimetallcarbonate oder auch -hydroxide oder tertiäre organische Basen, insbesondere tertiäre Niederalkylamine wie Triäthylamin oder N,N-Dimethylaminopyridin oder gegebenenfalls auch ein Überschuß eines Amins der Formel III. Sofern in den Verbindungen der Formel II m für 2 steht, bildet sich unter den Reaktionsbedingungen durch intramolekularen Ringschluß intermediär ein Aziridinring, welcher schon in Gegenwart relativ milder Basen und bei verhältnismäßig geringen Temperaturen mit den Verbindungen der Formel III reagiert. Sofern als Verbindungen der Formel III primäre Amine oder Ammoniak verwendet werden, ist es zweckmäßig, einen vielfachen Überschuß an diesen Verbindungen der Formel III einzusetzen, um Nebenreaktionen zu vermeiden. Zur Ätherbildung ist es zweckmäßig, starke Basen wie Alkalimetalle, Alkalimetallamide, -hydride, -hydroxide oder -alkoxide einzusetzen und den Alkohol der Formel II oder III zunächst durch Umsetzen mit einer der vorgenannten starken Basen in das entsprechende Alkoholat zu überführen und dieses dann weiter umzusetzen. Die Reaktionstemperatur kann je nach Art der verwendeten Reagenzien variieren und zwischen 0 °C und Siedetemperatur des Lösungsmittels, insbesondere zwischen ca. 20 °C und Siedetemperatur des Lösungsmittels, gewählt werden. Die Reaktionsdauer kann je nach Art der gewählten Reaktionsbedingungen zwischen zwei und zwölf Stunden betragen.

Die Reduktion von Amiden der Formel IV gemäß Verfahrensvariante b) kann auf an sich bekannte Weise mit zur Reduktion von Amiden geeigneten Reduktionsmitteln erfolgen. Als Reduktionsmittel eignen sich insbesondere komplexe Metallhydride wie Lithiumaluminiumhydrid, Diboran, Natriumborhydrid in Eisessig oder Diisobutylaluminiumhydrid. Die Reduktion erfolgt in einem unter den Reaktionsbedingungen inerten Lösungsmittel, z.B. einem vorzugsweise cyclischen Äther wie Tetrahydrofuran. Die Reaktionstemperatur kann zwischen Raumtemperatur und Siedetemperatur des Lösungsmittels liegen. Die Reaktionsdauer kann je nach Art des verwendeten Reduktionsmittels und der gewählten Reaktionsbedingungen zwischen 3 und 10 Stunden betragen.

Die Umsetzung von Verbindungen der Formel V mit Verbindungen der Formel VI gemäß Verfahrensvariante c) kann nach an sich zur Aminoalkylierung üblichen Methoden erfolgen. So kann die Umsetzung beispielsweise unter den für die Umsetzung von Verbindungen der Formel II mit Verbindungen der Formel III angegebenen Bedingungen durchgeführt werden.

Die Umsetzung von Verbindungen der Formel VII mit Verbindungen der Formel VIII gemäß Verfahrensvariante d) kann auf an sich bekannte Weise nach zur Äther- oder Thioätherbildung üblichen Methoden durchgeführt werden. So werden die Verbindungen der Formel VII mit den Verbindungen der Formel VIII zweckmäßigerweise in einem unter den Reaktionsbedingungen inerten organischen Lösungsmittel und in Gegenwart einer starken Base, welche fähig ist mit den Alkoholen bzw. Thioalkoholen der Formel VII zu den entsprechenden Alkoholaten bzw. Thioalkoholaten zu reagieren, umgesetzt. Als starke Basen eignen sich beispielsweise Alkalimetalle, Alkalimetallhydride, Alkalimetallamide oder Alkalimetallhydroxide. Als inerte organische Lösungsmittel eignen sich beispielsweise Äther, insbesondere cyclische Äther wie Tetrahydrofuran oder Dioxan, oder aromatische Kohlenwasserstoffe wie Benzol, Toluol oder Xylol. Gewünschtenfalls kann die Umsetzung von Verbindungen der Formel VII mit Verbindungen der Formel VIII auch ohne Zusatz eines Lösungsmittels durchgeführt werden. Die Reaktionstemperatur kann zwischen Raumtemperatur und Siedetemperatur des Reaktionsgemisches liegen und die Reaktionsdauer kann je nach Reaktionsbedingungen zwischen 2 und 10 Stunden betragen.

Die Gewinnung von Aminverbindungen der Formel Id gemäß Verfahrensvariante e) aus entsprechenden Aziden oder Phthalimiden der Formel IX kann auf an sich bekannte Weise nach zur Freisetzung von Aminen aus entsprechenden Aziden oder Phthalimiden üblichen Methoden erfolgen. So können beispielsweise Phthalimide der Formel IX auf an sich bekannte Weise durch Behandeln mit Hydrazin zu den Verbindungen der Formel Id gespalten werden. Azide der Formel IX können auf an sich bekannte Weise durch Behandeln mit einem komplexen Metallhydrid, beispielsweise Lithiumaluminiumhydrid oder, sofern Z für Sauerstoff steht, auch durch katalytische Hydrierung, beispielsweise unter Verwendung eines Palladium/Kohle-Katalysators, in die Aminverbindungen Id überführt werden.

Die Gewinnung von Verbindungen der Formel Ie aus Verbindungen der Formel X gemäß Verfahrensvariante f) kann auf an sich bekannte Weise durch katalytische Hydrogenolyse erfolgen. Als hydrogenolytisch abspaltbare Gruppen B kommen insbesondere der Benzylrest oder ein im Phenylring substituierter Benzylrest in Frage. Die Hydrogenolyse kann in Gegenwart von zur hydrogenolytischen Debenzylierung geeigneten Katalysatoren bei einem Wasserstoffdruck von 1 bis 10, insbesonere 1 - 6, bar und Temperaturen von 0 bis 60 °C in einem unter den Reaktionsbedingungen inerten Lösungsmittel durchgeführt werden. Als Katalysatoren eignen sich beispielsweise Palladium auf Kohle, Palladiumhydroxid auf Kohle oder Raney Nickel. Als Lösungsmittel eignen sich beispielsweise niedere Alkohole wie Äthanol, Essigsäureäthylester, Essigsäure oder aromatische Kohlenwasserstoffe wie Toluol oder deren Gemische, gegebenenfalls auch im Gemisch mit Wasser. Zweckmäßig wird die Hydrogenolyse in angesäuertem Medium, beispielsweise in einem einen Zusatz von Salzsäure enthaltenden Reaktionsmedium, durchgeführt.

Erhaltene Verbindungen der Formel I, worin $R^1$ Wasserstoff bedeutet oder worin $R^1$ niederes Alkyl bedeutet und worin $R^2$ eine freie NH-funktion enthält, können gewünschtenfalls nachträglich in an sich bekannter Weise zu entsprechenden N-substituierten Verbindungen alkyliert werden. Als Alkylierungsmittel kommen Verbindungen $R^{1'}$-Y oder $R^{5'}$-Y, worin $R^{1'}$, $R^{5'}$ und Y obige Bedeutung besitzen, in Frage, insbesondere $R^{1'}$- und $R^{5'}$-halogenide, insbesondere -jodide, und -sulfate oder -sulfonsäureester in Frage. Zweckmäßigerweise wird die Alkylierung in einem unter den Reaktionsbedingungen inerten organischen Lösungsmittel in Gegenwart einer Base wie beispielsweise einem Alkalimetallcarbonat oder einem tertiären organischen Amin, insbesondere einem tertiären Niederalkylamin, durchgeführt. Je nach der verwendeten Base eignen sich als Lösungsmittel Dimethylformamid, Acetonitril, cyclische Äther wie Tetrahydrofuran oder Dioxan, aromatische Kohlenwasserstoffe wie Toluol oder auch niedere Alkohole. Die Umsetzung kann bei Temperaturen zwischen Raumtemperatur und Siedetemperatur des Lösungsmittels erfolgen. Die nachträgliche Alkylierung kann auch als reduktive Alkylierung auf an sich bekannte Weise durch Umsetzen mit einem entsprechenden Aldehyd, insbesondere Formaldehyd, unter reduzierenden Bedingungen erfolgen. Z.B. können die Verbindungen mit dem Aldehyd in Gegenwart eines Reduktionsmittels, beispielsweise Ameisensäure, umgesetzt werden. Sofern in den Verbindungen der Formel I Z für Sauerstoff steht und die Verbindungen keine hydrogenolytisch abspaltbaren Reste enthalten, kann die reduktive Alkylierung auch durch Umsetzen der Verbindung mit dem Aldehyd und katalytische Hydrierung des Reaktionsgemisches erfolgen. Als Hydrierungskatalysator eignet sich zum Beispiel Palladium auf Kohle oder Raney Nickel. Bei der Einführung eines Alkyl-Restes $R^{1'}$ wird eine allfällige freie NH-funktion in dem Rest $R^2$ ebenfalls mitalkyliert.

Die Verbindungen der Formel I können auf an sich bekannte Weise aus dem Reaktionsgemisch isoliert und gereinigt werden. Säureadditionssalze können in üblicher Weise in die freien Basen überführt werden und diese gewünschtenfalls in bekannter Weise in pharmakologisch verträgliche Säureadditionssalze überführt werden.

Aus Stereoisomerengemischen der Verbindungen der Formel I können die einzelnen stereoisomeren Formen gewünschtenfalls durch übliche Trennverfahren, z.B. fraktionierte Kristallisation geeigneter Salze oder chromatographische Verfahren, angereichert und isoliert werden.

Als pharmakologisch annehmbare Säureadditionssalze der Verbindungen der Formel I eignen sich beispielsweise deren Salze mit anorganischen Säuren, z.B. Halogenwasserstoffsäuren, insbesondere Chlorwasserstoffsäure, Schwefelsäure oder Phosphorsäuren, oder mit organischen Säuren, beispielsweise niederen aliphatischen Mono- oder Dicarbonsäuren wie Maleinsäure, Fumarsäure, Oxalsäure, Milchsäure, Weinsäure oder Essigsäure, oder Sulfonsäuren, beispielsweise Niederalkylsulfonsäuren wie Methansulfonsäure oder gegebenenfalls im Benzolring durch Halogen oder niederes Alkyl substituierte Benzolsulfonsäuren wie p-Toluolsulfonsäure, oder Cyclohexylaminsulfonsäure.

Die Ausgangsverbindungen der Formel II können auf an sich bekannte Weise erhalten werden.

Verbindungen der allgemeinen Formel IIa

(siehe Formel IIa) worin $R^{1'}$, $R^3$, Z, n und m obige Bedeutung besitzen, können erhalten werden, indem man Amide der allgemeinen Formel XIa

(siehe Formel XIa) worin $R^{1'}$, $R^3$, Z, n und m obige Bedeutung besitzen, auf an sich bekannte Weise reduziert. Die Reduktion kann beispielsweise unter den für die Reduktion von Verbindungen der Formel IV vorstehend angegebenen Bedingungen erfolgen. Aus den Verbindungen der Formel IIa können Verbindungen der allgemeinen Formel IIb

(siehe Formel IIb) worin $R^{1'}$, $R^3$, Z, n, m und Y obige Bedeutung besitzen, gewonnen werden, indem man die Hydroxygruppe in den Verbindungen der Formel IIa in an sich bekannter Weise in eine Fluchtgruppe Y überführt. So können die Verbindungen der Formel IIa beispielsweise zur Einführung eines Halogenrestes Y mit Thionylchlorid oder mit Phosphorhalogeniden auf an sich bekannte Weise umgesetzt werden. Sulfonsäurereste Y können auf an sich bekannte Weise eingeführt werden, indem man Verbindungen der Formel IIa mit einem entsprechenden Sulfonsäurehalogenid acyliert.

Verbindungen der Formel II können auch erhalten werden, indem man Verbindungen der allgemeinen Formel Va

(siehe Formel Va) worin $R^{1'}$, $R^3$, Z und n obige Bedeutung besitzen, mit Verbindungen der allgemeinen Formel XII

(siehe Formel XII) worin Y, m und X obige Bedeutung besitzen, umsetzt. Die Umsetzung kann nach an sich zur

Aminoalkylierung üblichen Methoden, beispielsweise bei den vorstehend für die Umsetzung von Verbindungen der Formel II mit Verbindungen der Formel III angegebenen Bedingungen erfolgen. Zur Vermeidung von Nebenreaktionen ist es zweckmäßig, einen Überschuß an Verbindungen der Formel XII einzusetzen. Sofern in den Verbindungen in der Formel XII der Rest X eine Fluchtgruppe Y darstellt, ist es vorteilhaft, wenn die beiden in der Verbindung der Formel XII enthaltenen Fluchtgruppen unterschiedliche Reaktivität aufweisen, um eine gleichzeitige Reaktion beider Fluchtgruppen mit Verbindungen der Formel Va zu vermeiden.

Verbindungen der Formel IIa können auch erhalten werden, indem man ein Amin der allgemeinen Formel XIIIa

(siehe Formel XIIIa) worin $R^{1'}$ und m obige Bedeutungen besitzen, mit Verbindungen der allgemeinen Formel Vb

(siehe Formel Vb) worin $R^3$, Z, n und Y obige Bedeutung besitzen, umsetzt. Die Umsetzung kann nach an sich zur Aminoalkylierung üblichen Methoden, z.B. analog der Umsetzung von Verbindungen der Formel V mit Verbindungen der Formel VI erfolgen.

Amide der Formel XIa können erhalten werden, indem man eine Säure der allgemeinen Formel XIV

(siehe Formel XIV) worin $R^3$, Z und n obige Bedeutung besitzen, mit Aminen der Formel XIIIa auf an sich bekannte Weise umsetzt. So können reaktionsfähige Derivate der Säuren der Formel XIV, insbesondere deren Säurehalogenide, vorzugsweise -chloride, Ester und gemischte Anhydride, nach an sich zur Bildung von Amid-Gruppierungen durch Aminoacylierung üblichen Methoden mit den Aminen der Formel XIIIa umgesetzt werden. Die Aminoacylierung kann in einem unter den Reaktionsbedingungen inerten Lösungsmittel bei Temperaturen zwischen Raumtemperatur und Siedetemperatur des Lösungsmittels erfolgen. Als Lösungsmittel eignen sich halogenierte Kohlenwasserstoffe wie Dichlormethan oder Chloroform, aromatische Kohlenwasserstoffe wie Benzol, Toluol, Xylol oder Chlorbenzol, cyclische Äther wie Tetrahydrofuran oder Dioxan, Dimethylformamid oder Gemische dieser Lösungsmittel. Die Aminoacylierung kann gegebenenfalls, insbesondere wenn ein Säurehalogenid oder gemischtes Säureanhydrid verwendet wird, in Gegenwart eines säurebindenden Reagenzes durchgeführt werden. Als säurebindende Mittel eignen sich insbesondere organische Basen, beispielsweise tertiäre Niederalkylamine und Pyridine. Falls Säurehalogenide eingesetzt werden, kann die Reaktion auch in wäßrigem Medium und in Gegenwart einer anorganischen Base in an sich bekannter Weise nach der Methode von Schotten-Baumann durchgeführt werden. Falls die Säure selbst eingesetzt wird, so wird die Umsetzung zweckmäßigerweise in Gegenwart eines aus der Peptidchemie als geeignet bekannten Kopplungsreagenzes durchgeführt. Als Beispiel von Kopplungsreagenzien, welche die Amidbildung mit der freien Säure dadurch fördern, daß sie mit der Säure in vitro unter Bildung eines reaktionsfähigen Säurederivates reagieren, seien insbesondere genannt Alkyl-, vorzugsweise Cycloalkylcarbodiimide wie Dicyclohexylcarbodiimid, oder N-Niederalkyl-2-halogenpyridiniumsalze, insbesondere Halogenide oder Tosylate, vorzugsweise N-Methyl-2-chlorpyridiniumjodid (siehe z.B. Mukayama in angew. Chemie 91, 789 bis 912). Die Umsetzung in Gegenwart eines Kopplungsreagenzes kann beispielsweise bei Temperaturen von -30 bis +100 °C unter Benutzung von Lösungsmitteln wie halogenierten Kohlenwasserstoffen und/oder aromatischen Lösungsmitteln in Gegenwart eines säurebindenden Amins durchgeführt werden.

Säuren der Formel XIV können erhalten werden, indem man Verbindungen der Formel VII mit Halogencarbonsäuren der allgemeinen Formel XV

(siehe Formel XV) worin n obige Bedeutung besitzt und T Halogen bedeutet, umsetzt. Die Umsetzung von Verbindungen der Formel VII mit den Säuren der Formel XV kann auf an sich bekannte Weise unter zur Äther- oder Thioätherbildung üblichen Reaktionsbedingungen erfolgen. So können Salze der Säuren der Formel XV mit Alkalimetallsalzen der Verbindungen der Formel VII unter basischen Bedingungen umgesetzt werden. Die Reaktion kann beispielsweise bei den für die Umsetzung von Verbindungen der Formel VII mit Verbindungen der Formel VIII angegebenen Bedingungen durchgeführt werden.

Die Verbindungen der Formel IV können ausgehend von Säuren der Formel XIV erhalten werden. So können Säuren der Formel XIV mit Verbindungen der allgemeinen Formel VI'

(siehe Formel VI') worin $R^1$, $R^{2'}$ und m obige Bedeutung besitzen, zu solchen Verbindungen der Formel IV umgesetzt werden, worin D eine $R^{2'}$-$CH_2$-Gruppe darstellt, oder die Säuren der Formel XIV können mit Verbindungen der allgemeinen Formel XVI

(siehe Formel XVI) worin $R^1$, $R^5$, $R^6$ und m obige Bedeutung besitzen, umgesetzt werden zu solchen Verbindungen der Formel IV, worin D eine $NR^5R^6$-CO-Gruppe darstellt. Die Umsetzungen können nach zur Aminoacylierung üblichen Methoden, beispielsweise bei den vorstehend für die Umsetzung von Säuren der Formel XIV mit Aminen der Formel XIIIa angegebenen Reaktionsbedingungen durchgeführt werden. Ausgehend von Amiden der allgemeinen Formel XI

(siehe Formel XI) worin $R^1$, $R^3$, Z, n und m obige Bedeutung besitzen, können solche Verbindungen der Formel IV erhalten werden, worin der Rest D eine $N_3$-$CH_2$-Gruppe oder eine $NH_2$-$CH_2$-Gruppe darstellt. Hierzu wird die Hydroxygruppe der Verbindungen der Formel XI zunächst auf an sich bekannte Weise in eine funktionelle Gruppe Y umgewandelt. Auf an sich bekannte Weise wird das Reaktionsprodukt anschließend mit einem Alkalimetallazid umgesetzt, oder es wird zunächst mit einem Alkalimetallphthalimid umgesetzt und das Phthalimid anschließend zur $NH_2$-Gruppe gespalten.

Amide der Formel XI können durch Umsetzung der Säuren XIV mit Aminen der allgemeinen Formel XIII

(siehe Formel XIII) worin $R^1$ und m obige Bedeutung besitzen, unter zur Aminoacylierung üblichen Bedingungen erhalten werden.

Verbindungen der Formel XVI können erhalten werden, indem man ein reaktionsfähiges Säurederivat einer Halogencarbonsäure der allgemeinen Formel XVII

(siehe Formel XVII) worin T und m obige Bedeutung besitzen, mit einem Amin der allgemeinen Formel IIIa

(siehe Formel IIIa) worin $R^5$ und $R^6$ obige Bedeutung besitzen, nach an sich zur Bildung einer Amidgruppierung üblichen Methoden umsetzt zu einem Amid der allgemeinen Formel XVIII

(siehe Formel XVIII) worin $R^5$, $R^6$, m und T obige Bedeutung besitzen, und dieses Amid anschließend weiter mit einem Amin der allgemeinen Formel XIX

(siehe Formel XIX) worin $R^1$ obige Bedeutung besitzt, umsetzt. Sofern T für Chlor steht, wird dieses zweckmäßig zunächst auf an sich bekannte Weise in Jod überführt. Um Nebenreaktionen zu vermeiden ist es zweckmäßig einen vielfachen Überschuß an Amin einzusetzen.

Verbindungen der Formel Va können durch Reduktion von Amiden der allgemeinen Formel XX

(siehe Formel XX) worin $R^{1'}$, $R^3$, Z und n obige Bedeutung besitzen, erhalten werden. Die Reduktion kann auf an sich bekannte Weise, beispielsweise unter den zur Reduktion von Verbindungen der Formel IV angegebenen Bedingungen, durchgeführt werden.

Amide der Formel XX können erhalten werden, indem man reaktive Säurederivate von Säuren der Formel XIV mit Aminen der allgemeinen Formel XIXa

(siehe Formel XIXa) worin $R^{1'}$ obige Bedeutung besitzt, unter zur Amidbildung üblichen Bedingungen umsetzt.

Verbindungen den allgemeinen Formel Va'

(siehe Formel Va') worin $R^{1'}$, $R^3$ und n obige Bedeutung besitzen, können auch durch Hydrogenolyse von Verbindungen der allgemeinen Formel XXI

(siehe Formel XXI) worin $R^{1'}$, $R^3$, n und B obige Bedeutung besitzen, insbesondere hydrogenolytische Debenzylierung von solchen Verbindungen der Formel XXI, worin B Benzyl bedeutet, erhalten werden. Die Hydrogenolyse kann unter den für die Hydrogenolyse der Verbindungen der Formel X angegebenen Bedingungen erfolgen.

Verbindungen der Formel Vb können erhalten werden, indem man ein Alkalimetallsalz einer Verbindung der Formel VII auf an sich bekannte Weise mit einer Verbindung der allgemeinen Formel XXII

(siehe Formel XXII) worin T, n und X obige Bedeutung besitzen, umsetzt, und, sofern X in den Verbindungen der Formel XXII eine Hydroxygruppe darstellt, diese anschließend in an sich bekannter Weise in eine Fluchtgruppe Y umwandelt. Sofern der Rest X in den Verbindungen der Formel XXII eine Fluchtgruppe Y darstellt, ist es zur Vermeidung von Nebenreaktionen zweckmäßig, daß diese Fluchtgruppe weniger reaktionsfähig als der Halogensubstituent T ist. Sofern Verbindungen der Formel Vb, in denen Z Sauerstoff bedeutet und an eine $-CH_2$-gruppe im Rest $R^3$ gebunden ist, hergestellt werden sollen, kann anstelle eines Halogenalkohols der Formel XXII auch das entprechende Diol eingesetzt werden.

Verbindungen der Formel Vb können auch erhalten werden indem man Säuren der Formel XIV zu dem entsprechenden Alkohol reduziert und in diesem anschließend die Hydroxygruppe in eine Fluchtgruppe Y überführt. Verbindungen der Formel VIa

(siehe Formel VIa) worin $R^{2'}$, m und Y obige Bedeutung besitzen, sind bekannt oder können nach an sich bekannten Methoden hergestellt werden. So können Aminverbindungen der Formel VIa beispielsweise erhalten werden, indem Säuren der Formel XVIII mit Aminen der allgemeinen Formel IIIb

(siehe Formel IIIb) worin $R^{2'}$ obige Bedeutung besitzt, umgesetzt werden, und die erhaltenen Amide anschließend reduziert werden. Aminverbindungen der Formel VIa können auch erhalten werden, indem man Verbindungen der Formel XII, worin X Hydroxy bedeutet, mit Aminen der Formel IIIb umsetzt und in dem erhaltenen Reaktionsprodukt die Hydroxygruppe in eine Fluchtgruppe Y überführt. Verbindungen der Formel VIa, worin $R^{2'}$ für eine $OR^4$-Gruppe steht, können beispielsweise erhalten werden, indem man Halogencarbonsäuren der Formel XVII mit einem Alkohol der allgemeinen Formel XXV

(siehe Formel XXV) worin $R^4$ obige Bedeutung besitzt, umsetzt und das ein Derivat der Säure der allgemeinen Formel XXVI

(siehe Formel XXVI) worin $R^4$ und m obige Bedeutung besitzen, darstellende Reaktionsprodukt zu dem entsprechenden Alkohol reduziert und in diesem die Hydroxygruppe in eine Fluchtgruppe Y überführt.

Verbindungen der Formel VIb

(siehe Formel VIb) worin $R^{1'}$, $R^{2'}$ und n obige Bedeutung besitzen, können erhalten werden, indem man Verbindungen der Formel VIa mit einem Amin der Formel XIXa auf an sich bekannte Weise umsetzt. Verbindungen der Formel VIb, worin der Rest $R^{2'}$ keine gegebenenfalls substituierte Benzylgruppe enthält, können auch durch Hydrogenolyse von Verbindungen der allgemeinen Formel XXIII

(siehe Formel XXIII) worin $R^{1'}$, m und B obige Bedeutung besitzen und $R^{2'''}$ die für $R^{2'}$ angegebene Bedeutung mit Ausnahme von gegebenenfalls substituierte Benzylgruppen enthaltenden Resten besitzt, erhalten werden, insbesondere durch hydrogenolytische Debenzylierung solcher Verbindungen der Formel XXIII, worin B Benzyl bedeutet.

Verbindungen der Formel XXIII können ihrerseits durch Umsetzung von Aminen der allgemeinen Formel IIIc

(siehe Formel IIIc) worin $R^{2'''}$ obige Bedeutung besitzt, mit Verbindungen der allgemeinen Formel XIII'

(siehe Formel XIII') worin $R^{1'}$, m, X und B obige Bedeutung besitzen, erhalten werden.

Verbindungen der allgemeinen Formel VIIa

(siehe Formel VIIa) worin $R^3$ obige Bedeutung besitzt, sind bekannt. Verbindungen der allgemeinen Formel VIIb

(siehe Formel VIIb) worin $R^3$ obige Bedeutung besitzt, können ausgehend von Verbindungen der Formel VIIa erhalten werden. Hierzu wird die Hydroxygruppe der Verbindungen der Formel VIIa zunächst in Halogen überführt und das Reaktionsprodukt anschließend mit Kaliumthioacetat zu Verbindungen der allgemeinen Formel XXIV

(siehe Formel XXIV) worin $R^3$ obige Bedeutung besitzt, umgesetzt. Aus diesen wird dann durch Behandeln mit einem Alkalimetallalkoxid das Alkalimetallsalz der Verbindung VIIb erhalten, welches direkt für die weiteren Umsetzungen eingesetzt werden kann.

Verbindungen der Formel VIII können erhalten werden, indem man Verbindungen der Formel VIb mit Halogenalkoholen der allgemeinen Formel XXIIa

(siehe Formel XXIIa) worin T und n obige Bedeutung besitzen, umsetzt und in dem erhaltenen Reaktionsprodukt die Hydroxygruppe in eine Fluchtgruppe Y überführt.

Verbindungen der Formel IX können erhalten werden, indem man Verbindungen der Formel IIb auf an sich bekannte Weise mit einem Alkalimetallazid oder einem Alkalimetallphthalimid umsetzt.

Verbindungen der Formel X können erhalten werden, indem man Verbindungen der allgemeinen Formel II'

(siehe Formel II') worin $R^3$, n, m, Y und B obige Bedeutung besitzen, mit Aminen der Formel IIId

(siehe Formel IIId) worin $R^{2''}$ obige Bedeutung besitzt, umsetzt. Verbindungen der Formel II' können ihrerseits erhalten werden, indem man Amide der Formel XI'

(siehe Formel XI') worin $R^3$, n, m und B obige Bedeutung besitzen, reduziert und in dem Reaktionsprodukt anschließend die Hydroxygruppe in eine Fluchtgruppe Y überführt. Verbindungen der Formel XI' können ihrerseits erhalten werden durch Umsetzung von Säuren der allgemeinen Formel XIVa

(siehe Formel XIVa) worin $R^3$ und n obige Bedeutung besitzen, mit Aminen der allgemeinen Formel XIII''

(siehe Formel XIII'') worin m und B obige Bedeutung besitzen.

Verbindungen der Formel X können auch durch Umsetzung von Verbindungen der allgemeinen Formel VI''

(siehe Formel VI'') worin $R^2$, Y und m obige Bedeutung besitzen, mit Verbindungen der allgemeinen Formel XXI'

(siehe Formel XXI') worin $R^3$, n und B obige Bedeutung besitzen, erhalten werden.

Verbindungen der Formel VI'' sind bekannt oder können nach an sich bekannten Methoden hergestellt werden, beispielsweise durch Umsetzung von Aminen der Formel III mit Säuren der Formel XVII und anschließende Reduktion des Reaktionsproduktes, oder durch Umsetzung von Aminen der Formel III mit Verbindungen der Formel XII, worin X Hydroxy bedeutet, und anschließende Umwandlung der Hydroxygruppe in eine Fluchtgruppe Y.

Verbindungen der Formel XXI können erhalten werden, indem man Säuren der Formel XIVa mit einem Amin der allgemeinen Formel XIX'

(s. Formel XIX') worin $R^{1'}$ und B obige Bedeutung besitzen, umsetzt und die als Reaktionsprodukt erhaltenen Amide reduziert. Verbindungen der Formel XXI können auch erhalten werden, indem man Verbindungen der allgemeinen Formel Vb'

(s. Formel Vb') worin $R^3$, n und Y obige Bedeutung besitzen, mit Verbindungen der Formel XIX' umsetzt. Verbindungen der Formel XXI können auch erhalten werden, indem man ein Alkalimetallsalz einer Verbindung der Formel VIIa mit einer Verbindung der allgemeinen Formel XXVII

(siehe Formel XXVII) worin $R^{1'}$, n, Y und B obige Bedeutung besitzen, umsetzt. Verbindungen der Formel XXVII können ihrerseits durch Umsetzung eines Halogenalkohols der Formel XXIIa mit einem Amin der Formel XIX' und anschließende Überführung der Hydroxygruppe in eine Fluchtgruppe Y erhalten werden. Verbindungen der Formel XXI' können erhalten werden, indem man Verbindungen der Formel Vb' zunächst mit einem Alkalimetallazid zu dem entsprechenden Azid umsetzt und dieses zu dem entsprechenden Amin der allgemeinen Formel V'

(s. Formel V') worin $R^3$ und n obige Bedeutung besitzen, reduziert, und dieses Amin anschließend mit einem Benzaldehyd umsetzt und die als Reaktionsprodukt erhaltene Schiff'sche Base reduziert. Amine der Formel V', worin n für 3 steht, können auch erhalten werden, indem man Verbindungen der Formel VIIa mit Acrylnitril umsetzt und das Reaktionsprodukt reduziert.

Die nachstehenden Beispiele sollen die Erfindung näher erläutern, ohne jedoch ihren Umfang zu beschränken.

Die in den Beispielen angegebenen stereoisomeren Formen von einen 2-(6,6-Dimethylbicyclo[3.1.1]hept-2-yl)-äthylrest enthaltenden Verbindungen können bis zu ca. 5 % an anderen stereoisomeren Formen dieser Verbindungen enthalten.

Beispiel 1:

N-[2-((1R,3R,4S)-1-Methyl-4-isopropylcyclohex-3-yloxy)-äthyl]-N-[2-(morpholin-1-yl)-äthyl]-N-methylamin

A) 2,1 g L-Menthyloxyessigsäure (= ((1R,3R,4S)-1-Methyl-4-isopropylcyclohex-3-yloxy)-essigsäure) wurden in 50 ml Dichlormethan gelöst. Zu der Lösung wurden 3,1 g 2-Chlor-1-methylpyridiniumjodid, 0,8 g N-(2-Hydroxyäthyl)-N-methylamin und 2,6 g N,N-Diisopropyl-N-äthylamin zugegeben und das Reaktionsgemisch am Rückfluß gekocht. Nach Beendigung der Reaktion wurden zur Aufarbeitung 20 ml verdünnte Salzsäure hinzugegeben und das Gemisch dreimal mit Dichlormethan extrahiert. Die vereinigten Dichlormethanextrakte wurden über Natriumsulfat getrocknet, filtriert und unter vermindertem Druck eingedampft. Es wurden 2,6 g rohes N-(2-Hydroxyäthyl)-N-(methyl)-((1R,3R,4S)-1-methyl-4-isopropylcyclohex-3-yloxy)-acetamid erhalten, welches ohne weitere Reinigung in der nächsten Reaktionsstufe eingesetzt wurde. Die über Kieselgel chromatographisch unter Verwendung von n-Hexan/Äthanol als Elutionsmittel gereinigte Substanz hatte einen Schmelzpunkt von 65 - 66 °C.

B) 1,45 g des vorstehend erhaltenen Produktes wurden in 10 ml Tetrahydrofuran gelöst. Zu der Lösung wurden unter Stickstoffatmosphäre bei einer Temperatur von 0 °C 0,95 g Natriumborhydrid gegeben und dann wurden 1,5 g Eisessig tropfenweise zugesetzt. Anschließend wurde die Lösung 4 Stunden unter Rückfluß gekocht. Zur Aufarbeitung wurde die Lösung sodann unter vermindertem Druck eingedampft, der verbleibende Rückstand mit 20 ml Wasser versetzt und dreimal mit je 20 ml Dichlormethan extrahiert. Die vereinigten organischen Phasen wurden über Natriumsulfat getrocknet, filtriert und unter vermindertem Druck eingedampft. Es wurden 0,7 g N-[2-((1R,3R,4S)-1-Methyl-4-isopropylcyclohex-3-yloxy)-äthyl]-N-(2-hydroxyäthyl)-N-methylamin erhalten.

C) 0,7 g des vorstehend erhaltenen Produktes wurden in 25 ml Dichlormethan gelöst. Zu der Lösung wurden bei 0 °C 0,43 g Pyridin und 0,3 g Methansulfonsäurechlorid gegeben und das Reaktionsgemisch wurde 6 Stunden bei Raumtemperatur gerührt. Anschließend wurde die Lösung unter vermindertem Druck eingedampft und zu dem verbleibenden N-[2-((1R,3R,4S)-1-Methyl-4-isopropylcyclohex-3-yloxy)-äthyl]-N-(2-sulfonyloxyäthyl)-N-methylamin wurden sofort 2 ml Morpholin zugegeben. Das Reaktionsgemisch wurde dann 2 Stunden auf 40 °C erwärmt und anschließend unter vermindertem Druck eingedampft. Der verbleibende Rückstand wurde mit 10 ml Wasser versetzt und 3mal mit je 10 ml Essigsäureäthylester extrahiert. Die vereinigten organischen Phasen wurden über Natriumsulfat getrocknet, filtriert und unter vermindertem Druck eingeengt. Die verbleibende rohe Titelverbindung wurde anschließend durch Chromatographie an Kieselgel unter Verwendung von n-Hexan/Äther/Äthanol als Elutionsmittel gereinigt. Es wurden 0,77 g N-[2-((1R,3R,4S)-1-Methyl-4-isopropylcyclohex-3-yloxy)-äthyl]-N-[2-(morpholin-1-yl)-äthyl]-N-methylamin erhalten.

D) Die N-[2-((1R,3R,4S)-1-Methyl-4-isopropylcyclohex-3-yloxy)-äthyl]-N-(2-morpholin-1-yläthyl)-N-methylamin-Base wurde in 2 ml Äther gelöst und die Lösung mit gesättigter methanolischer Chlorwasserstofflösung versetzt. Der ausgefallene Niederschlag wurde abfiltriert, mit wenig Äther gewaschen und bei 40 °C getrocknet. Es wurden 0,7 g N-[2-((1R,3R,4S)-1-Methyl-4-isopropylcyclohex-3-yloxy)-äthyl]-N-[2-(morpholin-1-yl)-äthyl]-N-methylamin-Dihydrochlorid mit einem Schmelpunkt von 218 - 228 °C erhalten.
$[\alpha]_D^{20}$= -51.1° (c=1; Methanol)

Beispiel 2:

N-[2-((1R,3R,4S)-1-Methyl-4-isopropylcyclohex-3-yloxy)-äthyl]-N-[2-(2,2-dimethyläthoxy)-äthyl]-N-methylamin

A) 19 g N-[2-((1R,3R,4S)-1-Methyl-4-isopropylcyclohex-3-yloxy)-äthyl]-N-(2-hydroxyäthyl)-N-methylamin wurden in 200 ml Dichlormethan gelöst. Zu der Lösung wurden unter Eiskühlung 17,6 g Thionylchlorid getropft. Sodann wurden 2 Tropfen Dimethylformamid als Katalysator zugegeben und die Lösung 3 Stunden unter Rückfluß erhitzt. Zur Aufarbeitung wurde das Reaktionsgemisch unter Zusatz von Toluol eingedampft und der Rückstand noch je 2mal mit frischem Toluol versetzt und wiederum eingedampft. Der verbleibende Rückstand wurde anschließend in Dichlormethan gelöst und der Lösung wurde zur Ausfällung des Reaktionsproduktes Äther zugetropft. Der gebildete Niederschlag wurde abfiltriert und getrocknet. Es wurden 22,5 g N-[2-((1R,3R,4S)-1-Methyl-4-isopropylcyclohex-3-yloxy)-äthyl]-N-(2-chloräthyl)-N-methylamin-Hydrochlorid mit einem Schmelzpunkt von 184 - 194 °C erhalten.

B) 2,66 g des vorstehend erhaltenen Produktes wurden in 20 ml Isobutanol suspendiert. Zu dieser Suspension wurde bei einer Temperatur von 0 °C eine Suspension von 0,7 g festem, fein gemörsertem Natriumhydroxid in 50 ml Isobutanol zugesetzt. Anschließend wurde die Mischung 3 Stunden bei einer Temperatur von 40 °C gerührt. Zur Aufarbeitung wurde das Reaktionsgemisch dann unter vermindertem Druck eingedampft, der Rückstand mit Wasser versetzt und mit Essigsäureäthylester extrahiert. Die organische Phase wurde über Natriumsulfat getrocknet, filtriert und unter vermindertem Druck eingeengt. Das verbleibende Rohprodukt wurde durch Chromatographie an Kieselgel unter Verwendung von n-Hexan/Essigsäureäthylester/Äthanol als Elutionsmittel gereinigt. Es wurden 2,5 g N-[2-((1R,3R,4S)-1-Methyl-4-isopropylcyclohex-3-yloxy)-äthyl]-N-[2-(2,2-dimethyläthoxy)-äthyl]-N-methylamin erhalten.

C) 1,7 g N-[2-((1R,3R,4S)-1-Methyl-4-isopropylcyclohex-3-yloxy)-äthyl]-N-[2-(2,2-dimethyläthoxy)-äthyl]-N-methylamin-Base wurden in 30 ml Aceton gelöst. Zu der Lösung wurde eine Lösung von 0,68 g Oxalsäure in 10 ml Aceton gegeben. Nach Zugabe von 10 ml n-Hexan wurde die Mischung unter vermindertem Druck eingedampft. Der Rückstand wurde in 10 ml 2-Butanon aufgenommen und tropfenweise mit n-Hexan bis zur Trübung versetzt. Es wurden 1,96 g N-[2-((1R,3R,4S)-1-Methyl-4-isopropylcyclohex-3-yloxy)-äthyl]-N-[2-(2,2-dimethyläthoxy)-äthyl]-N-methylamin-oxalat mit dem Schmelzpunkt 113 - 115 °C erhalten.

Beispiel 3:

N-[2-((1R,3R,4S)-1-Methyl-4-isopropylcyclohex-3-yloxy)-äthyl]-N-[2-(isopropylamino)-äthyl]-N-methylamin.

5 g N-[2-((1R,3R,4S)-1-Methyl-4-isopropylcyclohex-3-yloxy)-äthyl]-N-(2-chloräthyl)-N-methylamin-Hydrochlorid wurden in eine Mischung aus 50 ml Isopropanol und 50 ml Wasser gegeben. Sodann wurden 1,9 g Isopropylamin und 7,7 g Kaliumcarbonat zugesetzt und das Reaktionsgemisch 5 Stunden unter Rückfluß gekocht. Zur Aufarbeitung wurde das Reaktionsgemisch sodann mit 100 ml Wasser verdünnt und die Lösung mit Essigsäureäthylester extrahiert. Die organische Phase wurde wie in Beispiel 1 C) beschrieben weiter aufgearbeitet. Es wurden 4,78 g N-[2-((1R,3R,4S)-1-Methyl-4-isopropylcyclohex-3-yloxy)-äthyl]-N-[2-(isopropylamino)-äthyl]-N-methylamin erhalten. Die freie Base wurde anschließend analog der in Beispiel 1 D) beschriebenen Methode in ihr Hydrochlorid überführt. Es wurden 5,1 g N-[2-((1R,3R,4S)-1-Methyl-4-isopropylcyclohex-3-yloxy)-äthyl]-N-[2-(isopropylamino)-äthyl]-N-methylamin-dihydrochlorid mit einem Schmelzpunkt von 156 - 158 °C erhalten.
$[\alpha]_D^{20}$ = -55,2 °(c=1; Methanol)

Beispiel 4:

N-[2-((1R,3R,4S)-1-Methyl-4-isopropylcyclohex-3-yloxy)-äthyl]-N-[3-(morpholin-1-yl)-propyl]-N-methylamin.

A) 4,2 g L-Menthyloxyessigsäure wurden in 50 ml Tetrahydrofuran gelöst. Der Lösung wurden 2,02 g Triäthylamin und anschließend bei einer Temperatur von -10 °C tropfenweise 2,2 g Chlorameisensäureäthylester zugesetzt. Anschließend wurden 1,2 g Methylamin in Form einer 33%igen äthanolischen Lösung hinzugegeben und das Reaktionsgemisch 2 Stunden bei Raumtemperatur gerührt. Zur Aufarbeitung wurden dem Reaktionsgemisch anschließend 100 ml Wasser zugesetzt und die Lösung mit Dichlormethan extrahiert. Der Dichlormethanextrakt wurde über Natriumsulfat getrocknet, filtriert und eingedampft. Es wurden 3,64 g ((1R,3R,4S)-1-Methyl-4-isopropylcyclohex-3-yloxy)-acetyl-N-methylamid erhalten.

B) Zu 4 g Natriumborhydrid in 100 ml Tetrahydrofuran wurde bei einer Temperatur von 0 °C unter Stickstoffatmosphäre eine Lösung von 5,3 g des vorstehend erhaltenen Produktes in 100 ml Tetrahydrofuran zugetropft. Anschließend wurden 6 g Eisessig tropfenweise zugegeben und das Reaktionsgemisch 4 Stunden unter Rückfluß gekocht. Zur Aufarbeitung wurde die Lösung unter vermindertem Druck eingedampft. Der Rückstand wurde in 50 ml Methanol aufgenommen und mit 3 ml konz. HC1 versetzt. Dann wurde die Mischung 1 Stunde unter Rückfluß gekocht und anschließend zur Trockene eingedampft (Zerstörung des Borkomplexes). Der Rückstand wurde in 50 ml Wasser aufgenommen, durch Zugabe von Natriumhydroxid-Lösung auf pH 11 gebracht und mit Dichlormethan extrahiert. Es wurden 2,9 g rohes N-[2-((1R,3R,4S)-1-Methyl-4-isopropylcyclohex-3-yloxy)-äthyl]-N-methylamin erhalten, welches direkt weiterverarbeitet wurde.

C) 6 g des vorstehend erhaltenen Produktes wurden in ein Gemisch aus 50 ml Isopropanol und 25 ml Wasser gegeben. Der Mischung wurden 6 g Kaliumcarbonat und 3,9 g 3-Brom-1-propanol zugegeben und das Reaktionsgemisch 3 Stunden unter Rückfluß gekocht. Zur Aufarbeitung wurde das Lösungsmittel unter vermindertem Druck abdestilliert und der Rückstand wurde mit 50 ml Wasser versetzt und mit tert. Butyl-methyl-äther extrahiert. Die organische Phase wurde über Natriumsulfat getrocknet, filtriert und eingeengt. Das als Rückstand verbleibende Rohprodukt wurde durch Chromatographie über Kieselgel unter Verwendung von Tetrahydrofuran/Äthanol als Elutionsmittel gereinigt. Es wurden 5,30 g N-[2-((1R,3R,4S)-1-Methyl-4-isopropylcyclohex-3-yloxy) -äthyl] -N-(3-hydroxypropyl)-N-methylamin erhalten.

D) 5,3 g des vorstehend erhaltenen Produktes wurden in 50 ml Dichlormethan gelöst. Die Lösung wurde mit 2 ml Thionylchlorid und 2 Tropfen Dimethylformamid versetzt und dann 3 Stunden unter Rückfluß gekocht. Anschließend wurde das Reaktionsgemisch analog Beispiel 2 A) aufgearbeitet. Es wurden 5,12 g N-[2-((1R,3R,4S)-1-Methyl-4-isopropylcyclohex-3-yloxy)-äthyl]-N-(3-chlorpropyl)-N-methylamin erhalten.

E) 5,0 g des vorstehend erhaltenen Produktes wurden in 24 ml Morpholin gegeben und das Reaktionsgemisch wurde 8 Stunden bei 50 °C gerührt. Anschließend wurde das überschüssige Morpholin abdestilliert. Der verbleibende Rückstand wurde in Wasser aufgenommen, das Gemisch durch Zusatz von Zitronensäure auf pH 5 gebracht. Die wässrige Phase wurde mit Dichlormethan extrahiert, sodann durch Zusatz von Kaliumhydrogencarbonat auf pH 8

eingestellt und nochmals mit Dichlormethan extrahiert. Die vereinigten Dichlormethanextrakte wurden über Natriumsulfat getrocknet, filtriert und das Lösungsmittel abdestilliert. Es wurden 5,19 g N-[2-((1R,3R,4S)-1-Methyl-4-isopropylcyclohex-3-yloxy)-äthyl]-N-[3-(morpholin-1-yl)-propyl]-N-methylamin erhalten.

F) 5,19 g N-[2-((1R,3R,4S)-1-Methyl-4-isopropylcyclohex-3-yloxy)-äthyl]-N-[3-(morpholin-1-yl)-propyl]-N-methylamin wurden in 50 ml Dichlormethan gelöst und die Lösung wurde bis zur sauren Reaktion mit ätherischer Salzsäurelösung versetzt. Das ausgefallene Kristallisat wurde abfiltriert, mit Äther gewaschen und bei 60 °C getrocknet. Es wurden 5,03 g N-[2-((1R,3R,4S)-1-Methyl-4-isopropylcyclohex-3-yloxy)-äthyl]-N-[3-(morpholin-1-yl)-propyl]-N-Methylamindihydrochlorid mit einem Schmelzpunkt von 232 - 236°C erhalten.

$[\alpha]_D^{20}$= -48,5° (c=1; $CH_3OH$)

Beispiel 5:

N-[2-((1R,3R,4S)-1-Methyl-4-isopropylcyclohex-3-yloxy)-äthyl]-N-[2-(3,4-dimethoxybenzyloxy)-äthyl]-N-methylamin

A) 4,7 g 3,4-Dimethoxybenzylalkohol wurden in 50 ml Toluol gelöst. Zu der Lösung wurden 1,1 g einer 60-%igen Natriumhydrid-Dispersion zugesetzt und das Reaktionsgemisch 1 Stunde unter Rückfluß gekocht. Dann wurden 4,4 g N-[2-((1R,3R,4S)-1-Methyl-4-isopropylcyclohex-3-yloxy)-äthyl]-N-(2-chloräthyl)-N-methylamin-Hydrochlorid zugegeben und die Reaktionsmischung weitere 4 Stunden gekocht. Zur Aufarbeitung wurde anschließend das Lösungsmittel abdestilliert und der Rückstand mit Wasser versetzt und mit Dichlormethan extrahiert. Die Dichlormethanphase wurde über Natriumsulfat getrocknet, filtriert und das Lösungsmittel abdestilliert, wobei als Rückstand 8,56 g der rohen Titelverbindung erhalten wurden. Diese wurde durch Chromatographie über Kieselgel unter Verwendung von n-Hexan/Tetrahydrofuran als Elutionsmittel und anschließende nochmalige Chromatographie über Aluminiumoxid, ebenfalls unter Verwendung von n-Hexan/Tetrahydrofuran als Elutionsmittel, gereinigt. Es wurden 4,27 g reines N-[2-((1R,3R,4S)-1-Methyl-4-isopropylcyclohex-3-yloxy)-äthyl]-N-[2-(3,4-dimethoxybenzyloxy)-äthyl]-N-methylamin erhalten.

B) 4,27 g der N-[2-((1R,3R,4S)-1-Methyl-4-isopropylcyclohex-3-yloxy)-äthyl]-N-[2-(3,4-dimethoxybenzyloxy)-äthyl]-N-methylamin-Base wurden in 50 ml Tetrahydrofuran gelöst. Zu der Lösung wurde eine Lösung von 1,22 g Fumarsäure in 15 ml Isopropanol zugesetzt. Anschließend wurde die Lösung unter vermindertem Druck auf ein Volumen von ca. 20 ml eingeengt und es wurde ein Gemisch aus Diäthyläther/n-Hexan bis zur Trübung zugesetzt und dann auf 0 °C abgekühlt. Die ausgefallenen Kristalle wurden abfiltriert und bei 50 °C getrocknet. Es wurden 4,28 g N-[2-((1R,3R,4S)-1-Methyl-4-isopropylcyclohex-3-yloxy)-äthyl]-N-[2-(3,4-dimethoxybenzyloxy)-äthyl]-N-methylamin-Fumarat mit einem Schmelzpunkt von 72 -73 °C erhalten.

$[\alpha]_D^{20}$= -37,0° (c=1; Methanol)

Beispiel 6:

N-[2-((1R,3R,4S)-1-Methyl-4-isopropylcyclohex-3-yloxy)-äthyl]-N-(2-äthoxyäthyl)-N-methylamin.

A) 2,57 g N-[2-((1R,3R,4S)-1-Methyl-4-isopropylcyclohex-3-yloxy)-äthyl]-N-(2-hydroxyäthyl)-N-methylamin wurden in 100 ml Tetrahydrofuran gelöst. Zu der Lösung wurden bei Raumtemperatur 0,48 g einer 50%-igen Natriumhydrid-Dispersion zugefügt und das Reaktionsgemisch 3 Stunden unter Rückfluß gekocht. Der Reaktionslösung wurde dann in der Siedehitze tropfenweise eine Lösung von 1,1 g Äthylbromid in 20 ml Tetrahydrofuran zugegeben und die Reaktionslösung wurde weitere 6 Stunden am Rückfluß gekocht. Zur Aufarbeitung wurden der Reaktionslösung 2 ml Wasser zugesetzt und das Gemisch unter vermindertem Druck eingeengt. Der verbleibende Rückstand wurde über Kieselgel unter Verwendung von n-Hexan/Dichlormethan/Methanol als Elutionsmittel chromatographisch gereinigt. Es wurden 0,55 g öliges N-[2-((1R,3R,4S)-1-Methyl-4-isopropylcyclohex-3-yloxy)-äthyl]-N-(2-äthoxyäthyl)-N-methylamin erhalten.

$[a]_D^{20}$ = - 51,20 (c=1, Methanol)

Beispiel 7:

N-(2-[2-((1S,2S,5S)-6,6-Dimethylbicyclo[3.1.1]hept-2-yl)-äthylthio]-äthyl)-N-[2-(morpholin-1-yl)-äthyl]-N-methylamin.

A) 15,6 g Triphenylphosphin wurden in 300 ml Acetonitril gelöst und zu der Lösung wurden 3,02 ml Brom tropfenweise unter starkem Rühren bei einer Temperatur von 0 °C zugegeben. Dann wurde eine Lösung von 10 g cis-Dihydronopol (= 2-((1S,2S,5S)-6,6-Dimethylbicyclo[3.1.1]hept-2-yl)-äthanol) in 100 ml Acetonitril zugegeben und das Reaktionsgemisch 5 Stunden auf 100 °C (Badtemperatur) erhitzt. Zur Aufarbeitung wurde das Lösungsmittel unter vermindertem Druck abdestilliert und der Rückstand über Kieselgel unter Verwendung von Dichlormethan als Elutionsmittel chromatographisch gereinigt. Es wurden 12,0 g 2-((1S,2S,5S)-6,6-Dimethylbicyclo[3.1,1]hept-2-yl)-äthylbromid

erhalten.

B) 4 g des vorstehend erhaltenen Produktes wurden in 20 ml Dimethylformamid gelöst, zu der Lösung wurden 2,0 g Kaliumthioacetat zugegeben und das Reaktionsgemisch 14 Stunden bei Raumtemperatur gerührt. Anschließend wurde das ausgefallene Kaliumbromid abfiltriert und das Lösungsmittel wurde unter vermindertem Druck abdestilliert. Der verbleibende Rückstand wurde chromatographisch über Kieselgel unter Verwendung von Toluol/Cyclohexan als Elutionsmittel gereinigt. Es wurden 3,8 g 2-((1S,2S,5S)-6,6-Dimethylbicyclo[3.1.1]hept-2-yl)-äthylthioacetat erhalten.

C) Zu einer Lösung von 7,5 g des vorstehend erhaltenen Produktes in 30 ml Methanol wurden 2,2 g Natriummethylat gegeben und die Lösung wurde bei Raumtemperatur 2 Stunden gerührt. Anschließend wurde das Reaktionsgemisch unter vermindertem Druck eingedampft. Der das Natriumsalz des 2-((1S,2S,5S)-6,6-Dimethylbicyclo[3.1.1] hept-2-yl)-thioäthanols enthaltende Rückstand wurde in Dimethylformamid gelöst, zu der Lösung wurden 4,75 g 2-Bromethanol und 9,2 g Kaliumcarbonat gegeben, und die Reaktionsmischung dann 4 Stunden unter Rückfluß gekocht. Zur Aufarbeitung wurde das Lösungsmittel unter vermindertem Druck abdestilliert, der Rückstand in Wasser aufgenommen und mit Dichlormethan extrahiert. Der Dichlormethanextrakt wurde über Natriumsulfat getrocknet, filtriert und eingedampft. Der verbleibende Rückstand wurde chromatographisch über Kieselgel unter Verwendung von n-Hexan/Essigsäureäthylester (7:1) gereinigt. Es wurden 2,8 g 2-[2-((1S,2S,5S)-6,6-Dimethylbicyclo[3.1.1]hept-2-yl)-äthylthio]-äthanol erhalten.

D) 2,8 g des vorstehend erhaltenen Produktes wurden in 30 ml Dichlormethan gelöst. Zu der Lösung wurden nacheinander bei einer Temperatur von 0 °C 1,54 g Pyridin, 2,22 g Methansulfonylchlorid und 2 ml Triäthylamin zugegeben. Das Reaktionsgemisch wurde 4 Stunden bei Raumtemperatur gerührt. Zur Aufarbeitung wurde einmal mit 20 ml Wasser ausgeschüttelt und die das gebildete 2-[2-((1S,2S,5S)-6,6-Dimethylbicyclo[3.1.1]hept-2-yl)-äthylthio]-äthyl-methansulfonat enthaltende organische Phase über Natriumsulfat getrocknet und filtriert.

E) Zu der vorstehend erhaltenen Lösung von 2-[2-((1S,2S,5S)-6,6-Dimethylbicyclo[3.1,1]hept-2-yl)-äthylthio]-äthyl-methansulfonat in Dichlormethan wurden 10 ml N-(2-Hydroxyäthyl)-N-methylamin zugesetzt. Anschließend wurde das Dichlormethan unter vermindertem Druck abdestilliert und das verbleibende Reaktionsgemisch 4 Stunden bei 60 °C gerührt. Zur Aufarbeitung wurden dem Reaktionsgemisch sodann 30 ml Wasser zugesetzt, und es wurde mit Essigsäureäthylester extrahiert. Die organische Phase wurde abgetrennt und eingeengt. Der verbleibende Rückstand wurde chromatographisch an Kieselgel unter Verwendung von n-Hexan/Dichlormethan/Methanol als Elutionsmittel gereinigt. Es wurden 2,6 g N-(2-[2-((1S,2S,5S)-6,6-Dimethylbicyclo[3.1.1]hept-2-yl)-äthylthio]-äthyl)-N-(2-hydroxyäthyl)-N-methylamin erhalten.

F) 2,6 g des vorstehend erhaltenen Produktes wurden in 100 ml Dichlormethan gelöst und der Lösung wurden 1,52 g Pyridin und anschließend bei einer Temperatur von 0 °C 1,6 g Methansulfonsäurechlorid zugesetzt. Die Lösung wurde dann 15 Stunden bei Raumtemperatur gerührt. Anschließend wurde das Reaktionsgemisch unter vermindertem Druck zur Trockene eingeengt. Das als Rückstand verbliebene rohe N-(2-[2-((1S,2S,5S)-6,6-Dimethylbicyclo[3.1.1] hept-2-yl)-äthylthio]-äthyl)-N-(2-sulfonyloxyäthyl)-N-methylamin wurde ohne weitere Reinigung weiter verarbeitet.

G) Zu dem vorstehend erhaltenen Produkt wurden 10 ml Morpholin gegeben, und das Reaktionsgemisch wurde 6 Stunden bei 60 °C gerührt. Zur Aufarbeitung wurde die Lösung unter vermindertem Druck eingeengt, der Rückstand in Wasser aufgenommen und mit Essigsäureäthylester extrahiert. Der Extrakt wurde chromatographisch an Kieselgel unter Verwendung von Essigsäureäthylester/Dichlormethen als Elutionsmittel gereinigt. Es wurden 2,8 g N-(2-[2-((1S, 2S,5S)-6,6-Dimethylbicyclo[3.1.1]hept-2-yl)-äthylthio]-äthyl)-N-[2-(morpholin-1-yl)-äthyl]-N-methylamin erhalten.

H) 2,8 g der freien Base wurden anschließend in 20 ml Methanol gelöst. Die Lösung wurde mit methanolischer Salzsäurelösung bis zu einer sauren Reaktion versetzt. Dann wurde die Lösung unter vermindertem Druck eingedampft und der Rückstand aus Methanol/Diäthyläther umkristallisiert. Es wurden 2,7 g N-(2-[2-((1S,2S,5S)-6,6-Dimethylbicyclo-[3.1.1]hept-2-yl)-äthylthio]-äthyl)-N-[2-(morpholin-1-yl)-äthyl]-N-methylamin-dihydrochlorid mit einem Schmelzpunkt von 189-209 °C erhalten.

$[\alpha]_D^{20}$= -15,1 (c=1; Methanol)

Beispiel 8:

N-[2-((1R,3R,4S)-1-Methyl-4-isopropylcyclohex-3-yloxy)-äthyl]-N-(2-diäthylaminoäthyl)-N-äthylamin.

A) 5,0 g ((1R,3R,4S)-1-Methyl-4-isopropylcyclohex-3-yloxy)essigsäure wurden in 100 ml Tetrahydrofuran gelöst. Zu der Lösung wurden 3,4 g (= 4,2 ml) N,N,N'-Triäthyl-äthylendiamin und 5,3 g Dicyclohexylcarbodiimid zugegeben und das Reaktionsgemisch 24 Stunden bei Raumtemperatur gerührt. Zur Aufarbeitung wurde unter vermindertem Druck eingeengt. Der verbleibende Rückstand wurde chromatographisch über Kieselgel unter Verwendung von Dichlormethan/ Methanol als Elutionsmittel gereinigt. Es wurden 4,8 g N-(2-Diäthyl-aminoäthyl)-N-(äthyl)-((1R,3R,4S)-1-methyl-4-isopropyl-cyclohex-3-yloxy)-essigsäureamid erhalten.

B) 1,4 g des vorstehend erhaltenen Produktes wurden in 20 ml Tetrahydrofuran gelöst, und die Lösung wurde unter Stickstoffatmosphäre tropfenweise zu einer Suspension von 0,3 g Lithiumaluminiumhydrid in 20 ml Tetrahydrofuran gegeben und das Reaktionsgemisch wurde anschließend 6 Stunden unter Rückfluß gekocht. Zur Aufarbeitung wurde das Reaktionsgemisch auf 0 °C abgekühlt und tropfenweise mit 1 ml Wasser und mit 4 ml einer wäßrigen 1N Natriumhydroxidlösung versetzt. Der gebildete Niederschlag wurde abfiltriert und das Filtrat unter vermindertem Druck eingeengt. Der Rückstand wurde mit 20 ml Wasser versetzt und mit Dichlormethan extrahiert. Nach Einengen des Dichlormethanextraktes wurden 0,82 g N-[2-((1R,3R,4S)-1-Methyl-4-isopropylcyclohex-3yloxy)-äthyl]-N-(2-diäthylaminoäthyl)-N-äthylamin erhalten.

C) 0,82 g der vorstehend gewonnenen Base wurden in 10 ml Aceton gelöst und mit einer Lösung von 0,62 g Oxalsäuredihydrat in 5 ml Aceton versetzt. Das Gemisch wurde 24 Stunden im Kühlschrank gekühlt und der gebildete Niederschlag wurde abfiltriert. Es wurden 0,45 g N-[2((1R,3R,4S)-1-Methyl-4-isopropylcyclohex-3-yloxy)-äthyl]-N-(2-diäthylaminoäthyl)-N-äthylamin-dioxalat mit einem Schmelzpunkt von 103 - 108 °C erhalten.
$[\alpha]_D^{20}$= -39,1° (c=1; Methanol)

Beispiel 9:

N-[2-((1R,3R,4S)-1-Methyl-4-isopropylcyclohex-3-yloxy)-äthyl]-N-[2-(morpholin-1-yl)-äthyl]-N-methylamin.

A) 33 g N-Benzyl-N-methyl-2-aminoäthanol wurden in 300 ml Dichlormethan gelöst. Zu der Lösung wurden bei 0 °C 48 g Thionylchlorid und 2 Tropfen Dimethylformamid zugegeben. Anschließend wurde das Reaktionsgemisch 6 Stunden unter Rückfluß gekocht. Zur Aufarbeitung wurde das Reaktionsgemisch zur Trockne eingedampft und der Rückstand noch dreimal in je 300 ml Toluol aufgenommen und wieder zur Trockene eingedampft. Der verbleibende Rückstand wurde in Dichlormethan gelöst und durch Zugabe von Diäthyläther zu der Lösung wurde das N-Benzyl-N-(2-chloräthyl)-N-methylamin-hydrochlorid auskristallisiert. Das Kristallisat (Fp.= 142 - 145 °C) wurde abfiltriert und in Eiswasser gelöst. Die Lösung wurde durch Zugabe von wäßriger Natriumhydroxidlösung bis zu pH 9 alkalisiert und sodann mit Toluol extrahiert. Die organische Phase wurde abgetrennt, über Natriumsulfat getrocknet, filtriert und unter vermindertem Druck eingeengt. Als Rückstand wurden 29 g N-Benzyl-N-(2-chloräthyl)-N-methylamin erhalten.

B) 23 g L-Menthol wurden in 200 ml Toluol gelöst. Zu der Lösung wurde bei einer Temperatur von 0 °C unter Stickstoffatmosphäre eine Suspension von 9 g Natriumhydrid in 200 ml Toluol zugegeben. Anschließend wurde das Reaktionsgemisch 3 Stunden unter Rückfluß gekocht. Dann wurde eine Lösung von 29 g des vorstehend unter A) erhaltenen Produktes in 100 ml Toluol zugesetzt und das Reaktionsgemisch weitere 4 Stunden unter Rückfluß gekocht. Zur Aufarbeitung wurde auf eine Temperatur von 0 °C abgekühlt und tropfenweise 20 ml Methanol hinzugegeben. Sodann wurden 300 ml Eiswasser und 200 ml verdünnter wäßriger Salzsäurelösung zugegeben und mit Essigsäureäthylester extrahiert. Die organische Phase wurde verworfen, und die wäßrige Phase wurde durch Zugabe von wäßriger Natriumhydroxidlösung alkalisch (pH 10) gestellt. Sodann wurde Natriumchlorid zugegeben und wiederum mit Essigsäureäthylester extrahiert. Die Essigsäureäthylesterphase wurde über Natriumsulfat getrocknet, filtriert und unter vermindertem Druck eingeengt. Es wurden 44 g rohes N-[2-((1R,3R,4S)-1-Methyl-4-isopropylcyclohex-3-yloxy)-äthyl]-N-benzyl-N-methylamin erhalten, welches direkt weiter umgesetzt wurde.

C) 44 g des vorstehend erhaltenen Produktes wurden in 200 ml Äthanol gelöst, und der Lösung wurde konzentrierte wäßrige Salzsäurelösung bis zur sauren Reaktion zugesetzt. Zu der Lösung wurden 20 g Hydrierungskatalysator (Palladium/Kohle 10 %-ig) gegeben und das Reaktionsgemisch bei Raumtemperatur und 5 bar Wasserstoffdruck hydriert. Nach beendigter Hydrierung wurde vom Katalysator abfiltriert, und die Lösung wurde unter vermindertem Druck eingeengt. Es wurden 29,2 g rohes N-[2-((1R,3R,4S)-1-Methyl-4-isopropylcyclohex-3-yloxy)-äthyl]-N-methylamin erhalten.

D) 29,2 g des vorstehend erhaltenen Produktes wurden in einem Gemisch aus 300 ml Isopropanol und 200 ml Wasser gelöst. Dem Gemisch wurden dann 36,5 g N-(2-Chloräthyl)-morpholin-hydrochlorid und 50 g Kaliumcarbonat zugesetzt, und das Reaktionsgemisch wurde 6 Stunden unter Rückfluß gekocht. Zur Aufarbeitung wurde die Reaktionslösung unter vermindertem Druck auf die Hälfte eingeengt und dann mit Essigsäureäthylester extrahiert. Der Essigsäureäthylesterextrakt wurde wie in Beispiel 1 C) beschrieben aufgearbeitet. Es wurden 20,8 g N-[2-((1R,3R,4S)-1-Methyl-4-isopropylcyclohex-3-yloxy)-äthyl]-N-[2-(morpholin-1-yl)-äthyl]-N-methylamin-dihydrochlorid mit einem Schmelzpunkt von 218 - 228 °C erhalten.

Beispiel 10:

N-(5-[2-((1S,2S,5S)-6,6-Dimethylbicyclo[3.1.1]hept-2-yl)-äthoxy]-pentyl)-N-[2-(morpholin-1-yl)-äthyl]-N-methylamin.

A) 200 g cis-Dihydronopol wurden in 340 ml Toluol gelöst. Zu der Lösung wurden tropfenweise 200 ml Thionylchlorid zugesetzt. Dann wurden 5 ml Dimethylformamid zugesetzt und das Reaktionsgemisch wurde 4 Stunden unter Rückfluß gekocht. Zur Aufarbeitung wurde das Lösungsmittel unter vermindertem Druck abdestilliert, der Rückstand wurde in 300 ml Toluol aufgenommen, wiederum zur Trockene eingeengt und nochmals in 300 ml Toluol aufgenommen

und zur Trockene eingedampft. Es wurden 206 g rohes 2-((1S,2S,5S)-6,6-Dimethylbicyclo[3.1.1]hept-2-yl) -äthylchlorid erhalten, welche ohne weitere Reinigung in der nächsten Stufe weiterverarbeitet wurden.

B) 12,2 g elementares Natrium wurden portionsweise über 12 Stunden verteilt bei einer Temperatur von 90 °C in 357 ml 1,5-Pentandiol gelöst. Zu dieser Lösung wurden dann tropfenweise 80 g 2-((1S,2S,5S)-6,6-Dimethylbicyclo-[3.1.1]hept-2-yl)-äthylchlorid zugegeben und das Reaktionsgemisch 15 Stunden auf 130 °C erhitzt. Zur Aufarbeitung wurde das Reaktionsgemisch auf Raumtemperatur abgekühlt, dann wurden 300 ml einer 5 %-igen wäßrigen Salzsäurelösung hinzugegeben und mit Äther extrahiert. Nach Einengen des Ätherextraktes wurden 99,6 g rohes 5-[2-((1S, 2S,5S)-6,6-Dimethylbicyclo[3.1.1]hept-2-yl)-äthoxy]-pentanol erhalten.

C) 99,6 g des vorstehend erhaltenen Produktes wurden in 120 ml Toluol gelöst. Zu der Lösung wurden 1 ml Dimethylformamid gegeben und anschließend bei Raumtemperatur tropfenweise 65 ml Thionylchlorid zugesetzt und das Reaktionsgemisch anschließend 3 Stunden am Rückfluß gekocht. Das Reaktionsgemisch wurde wie in Beispiel 10 A) beschrieben aufgearbeitet. Es wurden 102,1 g rohes 5-[2-((1S,2S,5S)-6,6-Dimethylbicyclo[3.1.1]hept-2-yl)-äthoxy]-pentylchlorid erhalten, welches sofort in der nächsten Reaktionsstufe weiterverarbeitet wurde.

D) Zu einer Lösung von 7,2 g des vorstehend erhaltenen Produktes in 80 ml Isopropanol und 20 ml Wasser wurden 10 g Kaliumcarbonat und 3,33 g N-Benzyl-N-methylamin gegeben. Die Lösung wurde 30 Stunden unter Rückfluß gekocht. Das Lösungsmittel wurde unter vermindertem Druck abdestilliert. Zu dem verbliebenen Rückstand wurden 30 ml Wasser gegeben und dann wurde mit Dichlormethan extrahiert. Der Dichlormethanextrakt wurde eingeengt und durch Chromatographie über Kieselgel unter Verwendung von Dichlormethan als Elutionsmittel gereinigt. Es wurden 5,7 g N-(5-[2-((1S,2S,5S)-6,6-Dimethylbicyclo[3.1.1]hept-2-yl)-äthoxy]-pentyl)-N-benzyl-N-methylamin erhalten.

E) 5,7 g des vorstehend erhaltenen Produktes wurden in einem Gemisch aus 80 ml Äthanol und 20 ml Wasser gelöst. Zu der Lösung wurden 20 ml konzentrierte Salzsäure und 10 g Katalysator (Palladium/Kohle 10 %-ig) gegeben und das Reaktionsgemisch bei Raumtemperatur und 3 bar Wasserstoffdruck hydriert. Nach beendeter Hydrierung wurde vom Katalysator abfiltriert und die Lösung unter vermindertem Druck eingeengt. Der Rückstand wurde mit 30 ml Wasser versetzt, das Gemisch mit Salzsäure angesäuert und mit Dichlormethan extrahiert. Nach Eindampfen des Dichlormethanextraktes wurden 2,03 g N-(5-[2-((1S,2S,5S)-6,6-Dimethylbicyclo[3.1.1]hept-2-yl)-äthoxy]-pentyl)-N-methylamin-Hydrochlorid erhalten.

F) 2,03 g des vorstehend erhaltenen Produktes wurden in einem Gemisch aus 80 ml Isopropanol und 20 ml Wasser gelöst. Zu der Lösung wurden nacheinander 8 g Kaliumcarbonat und 1,5 g N-(2-Chloräthyl)-morpholin-hydrochlorid zugegeben, und das Reaktionsgemisch wurde 3 Stunden bei 80 °C gerührt. Zur Aufarbeitung wurde das Lösungsmittel unter vermindertem Druck eingedampft. Der Rückstand wurde mit 20 ml Wasser versetzt und mit Dichlormethan extrahiert. Der nach Einengen des Dichlormethanextraktes verbleibende Rückstand wurde durch Chromatographie über Kieselgel unter Verwendung von Toluol/Äthanol/Ammoniak (80:20:1) gereinigt. Es wurden 1,57 g N-(5-[2-((1S,2S,5S)-6,6-Dimethylbicyclo[3.1.1]hept2-yl)-äthoxy]-pentyl)-N-[2-(morpholin-1-yl)-äthyl]-N-methylamin erhalten.

G) Das vorstehend erhaltene Amin wurde in 30 ml Dichlormethan gelöst und zu der Lösung wurde ätherische Salzsäure bis zur sauren Reaktion zugefügt. Die Lösung wurde im Vakuum eingeengt und der Rückstand aus Isopropanol umkristallisiert.

Es wurden 0,7 g -(5-[2-((1S,2S,5S)-6,6-Dimethylbicyclo-[3.1.1]hept-2-yl)-äthoxy]-pentyl)-N-[2-(morpholin-1-yl)-äthyl]-N-methylamin-Dihydrochlorid mit einem Schmelzpunkt von 228 - 240 °C erhalten.

Beispiel 11:

N-(5-[2-((1S,2S,5S)-6,6-Dimethylbicyclo[3.1.1]hept-2-yl)-äthoxy]-pentyl)-N-[2-(2,2-dimethyläthoxy)-äthyl]-N-methylamin.

15 g 5-[2-((1S,2S,5S)-6,6-Dimethylbicyclo[3.1.1]hept-2yl)-äthoxy]-pentylchlorid wurden in 100 ml Dimethylformamid gelöst. Zu der Lösung wurden 5,13 g N-[2-(2,2-Dimethyläthoxy)-äthyl]-N-methylamin (Herstellung siehe Beispiel 12 B)), 12 g Kaliumcarbonat und 1 g Kaliumjodid zugesetzt. Das Reaktionsgemisch wurde 12 Stunden bei einer Temperatur von 80 °C gerührt. Zur Aufarbeitung wurde das Reaktionsgemisch anschließend unter vermindertem Druck eingeengt. Der Rückstand wurde mit 100 ml Wasser versetzt und dreimal mit je 80 ml Dichlormethan extrahiert. Die vereinigten Dichlormethanextrakte wurden über Natriumsulfat getrocknet, filtriert und eingeengt. Das als Rückstand verbleibende Rohprodukt wurde chromatographisch über Kieselgel unter Verwendung von Dichlormethan/Methanol als Elutionsmittel gereinigt. Es wurden 4,1 g öliges N-(5-[2-((1S,2S,5S)-6,6-Dimethylbicyclo[3.1.1]hept-2-yl)-äthoxy]-pentyl)-N-[2-(2,2-dimethyläthoxy)-äthyl]-N-methylamin erhalten.
$[\alpha]_D^{20}$= -9,1° (c=1; Wasser)

Beispiel 12:

N-(2-[2-((1S,2S,5S)-6,6-Dimethylbicyclo[3.1.1]hept-2-yl)-äthoxy]-äthyl)-N-[2-(2,2-dimethyläthoxy)-äthyl]-N-methylamin.

A) Zu einer Lösung von 3,25 g Natriumhydrid in 100 ml Toluol wurde bei einer Temperatur von 0 °C eine Lösung von 5,0 g Isobutanol in 50 ml Toluol gegeben, und das Reaktionsgemisch wurde 3 Stunden unter Rückfluß gekocht. Dann wurden zu dem Reaktionsgemisch 12,4 g N-Benzyl-N-methyl-2-chloräthylamin hinzugegeben, und die Lösung wurde weitere 8 Stunden unter Rückfluß gekocht. Zur Aufarbeitung wurden der Reaktionslösung vorsichtig 5 ml Methanol und dann 100 ml Wasser zugesetzt und dann durch Zugabe von verdünnter wäßriger Salzsäurelösung auf pH 2 eingestellt. Nach Schütteln wurde die organische Phase abgetrennt und verworfen. Die wäßrige Phase wurde durch Zugabe von wäßriger Natriumhydroxidlösung auf einen pH 13 gebracht und mit Essigsäureäthylester extrahiert. Der Essigsäureäthylesterextrakt wurde über Natriumsulfat getrocknet, filtriert und eingeengt. Der verbleibende Rückstand wurde chromatographisch über Kieselgel unter Verwendung von n-Hexan/Essigsäureäthylester als Elutionsmittel gereinigt. Es wurden 10,7 g N-[2-(2,2-Dimethyläthoxy)-äthyl]-N-benzyl-N-methylamin erhalten.

B) 8,9 g des vorstehend erhaltenen Produktes wurden in 200 ml Äthanol gelöst. Der Lösung wurden 6 g Hydrierungskatalysator (Palladium/Kohle 5 %-ig) und 5,2 ml konzentrierte wäßrige Salzsäurelösung zugesetzt. Dann wurde das Gemisch bei Raumtemperatur unter einem Wasserstoffdruck von 3,5 bar hydriert. Nach beendigter Hydrierung wurde vom Katalysator abfiltriert, das Filtrat unter vermindertem Druck eingeengt, und der verbleibende Rückstand wurde chromatographisch über Kieselgel unter Verwendung von Dichlormethan/Methanol als Elutionsmittel gereinigt. Es wurden 5,13 g N-[2-(2,2-Dimethyläthoxy)-äthyl]-N-methylamin erhalten.

C) 5,13 g des vorstehend erhaltenen Produktes wurden in 30 ml Dimethylformamid gelöst. Zu der Lösung wurden 12 g Kaliumcarbonat und 10,7 g 2-Bromäthanol gegeben und das Reaktionsgemisch anschließend 12 Stunden bei einer Temperatur von 60 °C gerührt. Zur Aufarbeitung wurde das Reaktionsgemisch mit 200 ml Wasser versetzt und mit Essigsäureäthylester extrahiert. Der Essigsäureäthylesterextrakt wurde über Natriumsulfat getrocknet, filtriert und eingeengt. Nach chromatographischer Reinigung des verbleibenden Rückstandes wurden 3,9 g N-[2-(2,2-Dimethyläthoxy)-äthyl]-N-(2-hydroxyäthyl)-N-methylamin erhalten.

D) 3,9 g des vorstehend erhaltenen Produktes wurden in 50 ml Dichlormethan gelöst. Zu der Lösung wurden 5,3 g Thionylchlorid und dann 2 Tropfen Dimethylformamid zugegeben. Das Reaktionsgemisch wurde 5 Stunden unter Rückfluß gekocht. Zur Aufarbeitung wurde zur Trockne eingeengt, der Rückstand zweimal mit je 100 ml Toluol aufgenommen und wiederum zur Trockne eingedampft. Der verbleibende Rückstand wurde in wenig Dichlormethan gelöst, und die Lösung wurde tropfenweise mit n-Hexan bis zur Trübung versetzt. Die Mischung wurde dann 12 Stunden im Kühlschrank aufbewahrt, und die ausgefallen Kristalle wurden abfiltriert und getrocknet. Es wurden 3,77 g N-[2-(2,2-Dimethyläthoxy)-äthyl]-N-(2-chloräthyl)-N-methylamin-hydrochlorid erhalten.

E) 3,3 g des vorstehend erhaltenen Produktes wurden in 20 ml cis-Dihydronopol gelöst. Der Lösung wurden bei einer Temperatur von 0 °C 1,6 g festes fein gemörsertes Natriumhydroxid zugesetzt. Das Reaktionsgemisch wurde dann 7 Stunden bei einer Temperatur von 40 °C gerührt. Zur Aufarbeitung wurde die Reaktionsmischung unter vermindertem Druck eingeengt. Der Rückstand wurde mit 50 ml Wasser versetzt und mit Essigsäureäthylester extrahiert. Der nach Einengen des Extraktes verbleibende Rückstand wurde chromatographisch über Kieselgel unter Verwendung von Dichlormethan/Methanol als Elutionsmittel gereinigt. Es wurden 1,6 g N-(2-[2((1S,2S,5S)-6,6-Dimethylbicyclo [3.1.1]hept-2-yl)-äthoxy]-äthyl)-N-[2-(2,2-dimethyläthoxy)-äthyl]-N-methylamin erhalten.

F) 1,6 g des vorstehend erhaltenen Produktes wurden in 20 ml Dichlormethan gelöst und die Lösung wurde mit methanolischer Salzsäure bis zur sauren Reaktion versetzt. Dann wurde die Lösung unter vermindertem Druck eingedampft. Der Rückstand wurde in 50 ml Toluol gelöst und die Lösung wurde wieder unter vermindertem Druck eingeengt und der Rückstand wurde in 5 ml Dichlormethan gelöst. Zu der Lösung wurde n-Hexan bis zum Auftreten einer Trübung zugegeben. Dann wurde die Mischung im Kühlschrank über Nacht stehengelassen. Die ausgefallenen Kristalle wurden dann abfiltriert und getrocknet. Es wurden 1,2 g N-(2-[2-((1S,2S,5-S)-6,6-Dimethylbicyclo[3.1.1]hept-2-yl)-äthoxy]-äthyl)-N-[2-(2,2-dimethyläthoxy)-äthyl]-N-methylamin-Hydrochlorid mit einem Schmelzpunkt von 86 - 88 °C erhalten.

Beispiel 13:

N-[2-((1R,3R,4S)-1-Methyl-4-isopropylcyclohex-3-yloxy)-äthyl]-N-(2-aminoäthyl)-N-methylamin.

A) 4,5 g N-[2-((1R,3R,4S)-1-Methyl-4-isopropylcyclohex-3-yloxy)-äthyl]-N-(2-chloräthyl)-N-methylamin wurden in ein Gemisch aus 40 ml Wasser und 20 ml Tetrahydrofuran gegeben. Dem Gemisch wurden 2 g Kaliumcarbonat und 1 g Natriumazid zugesetzt. Die Reaktionslösung wurde anschließend 8 Stunden bei Raumtemperatur gerührt. Zur Aufarbeitung wurde die Reaktionslösung auf ca. 30 ml eingeengt. Die wäßrige Phase wurde dann mit Essigsäureäthylester extrahiert. Nach Einengen des Essigsäureäthylesterextraktes wurden 4,5 g rohes N-[2-((1R,3R,4S)-1-Methyl-4-isopropylcyclohex-3-yloxy)-äthyl]-N-(2-azidoäthyl)-N-methylamin erhalten, welches ohne weitere Reinigung in der nächsten Stufe eingesetzt wurde.

B) 4,5 g des vorstehend erhaltenen rohen Produktes wurden in 200 ml Äthanol gelöst. Zu der Lösung wurden 3 g Hydrierungskatalysator (Palladium/Kohle 10 %-ig) gegeben und das Reaktionsgemisch bei Raumtemperatur und einem Wasserstoffdruck von 5 bar hydriert. Zur Aufarbeitung wurde von dem Katalysator abfiltriert, und die filtrierte Lösung wurde unter vermindertem Druck zur Trockne eingeengt. Die verbleibende rohe Titelverbindung wurde in Tetrahydrofuran gelöst und über Magnesiumsilikat gereinigt. Es wurden 3,95 g N-[2-((1R,3R,4S)-1-Methyl-4-isopropylcyclohex-3-yloxy)-äthyl]-N-(2-aminoäthyl)-N-methylamin erhalten.

C) 1,42 g der vorstehend erhaltenen Base wurden in 40 ml Tetrahydrofuran und 5 ml Methanol gelöst. Der Lösung wurden 1,29 g Fumarsäure zugesetzt und das Reaktionsgemisch 8 Stunden bei Raumtemperatur gerührt. Das ausgefallene Kristallisat des Fumarates der Titelverbindung wurde abfiltriert und unter vermindertem Druck getrocknet. Es wurden 1,50 g N-[2-((1R,3R,4S)-1-Methyl-4-isopropylcyclohex-3-yloxy)-äthyl]-N-(2-aminoäthyl)-N-methylamin-difumarat mit einem Schmelzpunkt von 152 - 154 °C erhalten.

Beispiel 14:

N-[3-((1R,3R,4S)-1-Methyl-4-isopropylcyclohex-3-yloxy)-propyl] -N-[2-(morpholin-1-yl)-äthyl]-amin.

A) Zu einer Lösung von 30 g L-Menthol in 300 ml Dichlormethan wurden 1 g einer Natriumhydrid-Suspension (50 %-ig) in Paraffinöl bei einer Temperatur von 0 °C zugegeben. Anschließend wurde eine Lösung von 12,5 ml Acrylnitril in 50 ml Dichlormethan langsam hinzugetropft und das Reaktionsgemisch dann weitere 5 Stunden bei Raumtemperatur gerührt. Zur Aufarbeitung wurde das Reaktionsgemisch vorsichtig mit 10 ml Eisessig versetzt. Dann wurden 200 ml Wasser zugesetzt und mit Dichlormethan extrahiert. Der Dichlormethanextrakt wurde über Natriumsulfat getrocknet, filtriert und eingeengt. Nach chromatographischer Reinigung des Rückstandes über Kieselgel unter Verwendung von n-Hexan/Dichlormethan als Elutionsmittel wurden 25,03 g rohes 2-((1R,3R,4S)-1-Methyl-4-isopropylcyclohex-3-yloxy)-propionitril erhalten, welches ohne weitere Reinigung für die nächste Reaktionsstufe verwendet wurde.

B) 25 g des vorstehend erhaltenen Produktes wurden in 100 ml Methanol gelöst. Zu der Lösung wurden 50 ml konzentrierte wäßrige Ammoniaklösung gegeben. Dann wurden 20 g Raney-Nickel zugesetzt und das Reaktionsgemisch bei Raumtemperatur und einem Wasserstoffdruck von 4 bar hydriert. Nach Beendigung der Wasserstoffaufnahme wurde vom Katalysator abfiltriert und das Filtrat unter vermindertem Druck eingedampft. Der Rückstand wurde mit 200 ml Wasser versetzt und mit tert. Butylmethyläther extrahiert. Die organische Phase wurde eingeengt und der Rückstand an Kieselgel unter Verwendung von Dichlormethan/Methanol/Ammoniak als Elutionsmittel chromatographiert. Es wurden 22,33 g 3-((1R,3R,4S)-1-Methyl-4-isopropylcyclohex-3-yloxy)-propylamin erhalten.

C) 19,98 g des vorstehend erhaltenen Produktes wurden in 200 ml Äthanol gelöst. Zu der Lösung wurden 10,5 ml Benzaldehyd gegeben, und das Reaktionsgemisch wurde 1 Stunde auf 50 °C erwärmt. Dann wurde auf 0 °C abgekühlt, und es wurden portionsweise 3,6 g Natriumborhydrid zugesetzt und das Reaktionsgemisch eine weitere Stunde reagieren gelassen. Zur Aufarbeitung wurden dann vorsichtig 10 ml Wasser zugegeben und das Gemisch durch Zugabe von 2N wäßriger Salzsäurelösung auf pH 10 gebracht. Das Äthanol wurde abdestilliert, und die verbleibende wäßrige Phase wurde mit Diäthyläther extrahiert. Nach Eindampfen des Ätherextraktes wurden 28,3 g rohes N-[3-((1R,3R,4S)-1-Methyl-4-isopropylcyclohex-3-yloxy)-propyl]-N-benzylamin erhalten.

D) 9,65 g des vorstehend erhaltenen Produktes wurden in einem Gemisch aus 50 ml Isopropanol und 30 ml Wasser gelöst. Der Lösung wurden 10,0 g N-(2-Chloräthyl)-morpholin-Hydrochlorid und 9,0 g Kaliumcarbonat zugesetzt und das Reaktionsgemisch unter Rückfluß 10 Stunden gekocht. Zur Aufarbeitung wurde das Isopropanol aus dem Reaktionsgemisch unter vermindertem Druck abdestilliert, und die wäßrige Phase wurde mit Dichlormethan extrahiert. Die Dichlormethanphase wurde über Natriumsulfat getrocknet, filtriert und eingeengt. Nach chromatographischer Reinigung des verbleibenden Rückstandes über Kieselgel unter Verwendung von n-Hexan/Tetrahydrofuran als Elutionsmittel wurden 9,89 g N-[3-((1R,3R,4S)-1-Methyl-4-isopropylcyclohex-3-yloxy)-propyl]-N-[2-(morpholin-1-yl)-äthyl]-N-benzylamin erhalten.

E) 5,5 g des vorstehend erhaltenen Produktes wurden in einem Gemisch aus 100 ml Äthanol und 20 ml Wasser gelöst. Der Lösung wurden 12 ml wäßrige 2N Salzsäurelösung und 10 g Hydrierungskatalysator (Palladium/Kohle 10 %-ig) zugesetzt, und das Reaktionsgemisch wurde bei Raumtemperatur und einem Wasserstoffdruck von 4,5 bar hydriert. Nach Beendigung der Wasserstoffaufnahme wurde vom Katalysator abfiltriert und die filtrierte Lösung im Vakuum eingedampft. Der Rückstand wurde mit verdünnter wäßriger Natriumhydroxidlösung (pH 11) versetzt und mit tert. Butylmethyläther extrahiert. Die organische Phase wurde über Natriumsulfat getrocknet, filtriert und eingeengt. Es wurden 4,03 g N-[3-((1R,3R,4S)-1-Methyl-4-isopropylcyclohex-3-yloxy)-propyl]-N-[2-(morpholin-1-yl)-äthyl]-amin erhalten. Die freie Base wurde analog der in Beispiel 1D beschriebenen Methode in das N-[3((1R,3R,4S)-1-Methyl-4-isopropylcyclohex-3-yloxy)-propyl) -N-[2-(morpholin-1-yl)-äthyl]-amin-dihydrochlorid mit einem Schmelzpunkt von

222-226 °C überführt.

Beispiel 15:

N-[3-((1R,3R,4S)-1-Methyl-4-isopropylcyclohex-3-yloxy)-propyl]-N-[2-(morpholin-1-yl)-äthyl]-N-methylamin.

A) 4 g N-[3-((1R,3R,4S)-1-Methyl-4-isopropylcyclohex-3-yloxy)-propyl]-N-[2-(morpholin-1-yl)-äthyl]-amin wurden in 100 ml Äthanol gelöst. Der Lösung wurden 1,4 ml 37 %-iger Formaldehyd-Lösung und ca. 5 g Hydrierungskatalysator (Palladium/Kohle 10 %-ig) zugegeben, und das Reaktionsgemisch wurde unter einem Wasserstoffdruck von 4,5 bar bei Raumtemperatur hydriert. Nach Beendigung der Wasserstoffaufnahme wurde vom Katalysator abfiltriert und das Filtrat unter vermindertem Druck eingedampft. Der Rückstand wurde in 20 ml Wasser aufgenommen und mit Äther extrahiert. Nach Eindampfen des Ätherextraktes wurden 3,85 g N-[3-((1R,3R,4S)-1-Methyl-4-isopropylcyclohex-3-yloxy)-propyl]-N-[2-(morpholin-1-yl)-äthyl]-N-methylamin erhalten.

B) 3,85 g der vorstehend erhaltenen freien Base wurden in 60 ml Dichlormethan gelöst. Der Lösung wurde bis zur sauren Reaktion eine ätherische Salzsäurelösung zugesetzt. Die ausgefallenen Kristalle wurden filtriert und mit 50 ml eines Gemisches Diäthyläther/n-Hexan (1 : 1) gewaschen und anschließend aus einem Gemisch aus 2-Butanon/Äthanol (bis zu 10%) umkristallisiert. Es wurden 3,90 g N-[3-((1R,3R,4S)-1-Methyl-4-isopropylcyclohex-3-yloxy)-propyl]-N-[2-(morpholin-1-yl)-äthyl]-N-methylamin-dihydrochlorid mit einem Schmelzpunkt von 193-196 °C erhalten.

Beispiel 16:

N-[2-((1R,3R,4S)-1-Methyl-4-isopropylcyclohex-3-yloxy)-äthyl]-N-[2-(2,2-dimethyläthoxy)-äthyl]-amin.

A) Zu einer Lösung von 34,3 g ((1R,3R,4S)-1-Methyl-4-isopropylcyclohex-3-yloxy)-essigsäure in 350 ml Dichlormethan wurden 49,4 g 2-Chlor-1-methyl-pyridiniumjodid gegeben und das Reaktionsgemisch 1 Stunde bei Raumtemperatur gerührt. Dann wurden bei einer Temperatur von 0 °C 24,2 g 2-(Benzylamino)-äthanol und 41,4 g N,N-Diisopropyl-N-äthylamin zugetropft. Die Lösung wurde anschließend 6 Stunden unter Rückfluß gekocht. Zur Aufarbeitung wurde das Reaktionsgemisch anschließend einmal mit wäßriger 1N Salzsäurelösung und einmal mit wäßriger Natriumhydroxidlösung ausgeschüttelt. Anschließend wurde die organische Phase über Natriumsulfat getrocknet, filtriert und unter vermindertem Druck eingeengt. Es wurden 57,6 g rohes N-(2-Hydroxyäthyl)-N-(benzyl)-((1R,3R,4S)-1-methyl-4-isopropylcyclohex-3-yloxy)-acetamid erhalten.

B) Zu in 200 ml Tetrahydrofuran enthaltenen 11,4 g Natriumborhydrid wurde bei Raumtemperatur unter Stickstoffatmosphäre eine Lösung von 20 g des vorstehend erhaltenen Produktes in 200 ml Tetrahydrofuran gegeben. Anschließend wurden 18 ml Essigsäure tropfenweise über einen Zeitraum von 30 Minuten verteilt hinzugegeben und die Mischung 6 Stunden unter Rückfluß gekocht. Zur Aufarbeitung wurde die Lösung unter vermindertem Druck eingeengt und der verbleibende Rückstand vorsichtig mit 200 ml Wasser versetzt. Dann wurde durch Zugabe von wäßriger 2N Natriumhydroxidlösung auf pH 10 eingestellt und mit Dichlormethan extrahiert. Nach Einengen des Dichlormethanextraktes wurden 18,4 g rohes N-[2-((1R,3R,4S)-1-Methyl-4-isopropylcyclohex-3-yloxy)-äthyl]-N-(2-hydroxyäthyl)-N-benzylamin erhalten, welches ohne weitere Reinigung für die nächste Reaktionsstufe eingesetzt wurde.

C) Zu einer Lösung von 8 g Thionylchlorid in 100 ml Dichlormethan wurde bei einer Temperatur von 0 °C eine Lösung von 8 g des vorstehend erhaltenen Produktes in 70 ml Dichlormethan getropft. Dann wurden 2 Tropfen Dimethylformamid als Katalysator zugegeben, und die Lösung wurde 4 Stunden unter Rückfluß gekocht. Zur Aufarbeitung wurde die Lösung eingedampft und der Rückstand dreimal mit je 100 ml Toluol aufgenommen und wiederum zur Trockne eingedampft. Der verbleibende Rückstand wurde in 50 ml Dichlormethan gelöst, und zu der Lösung wurde tropfenweise Diäthyläther bis zum Auftreten einer Trübung gegeben. Das Gemisch wurde 12 Stunden im Kühlschrank stehengelassen. Dann wurden die ausgefallenen Kristalle abfiltriert und unter vermindertem Druck getrocknet. Es wurden 6,7 g N-[2-((1R,3R,4S)-1-Methyl-4-isopropylcyclohex-3-yloxy)-äthyl]-N-(2-chloräthyl)-N-benzylamin-hydrochlorid erhalten.

D) 6,7 g des vorstehend erhaltenen Produktes wurden in 150 ml Isobutanol gelöst. Zu der Lösung wurden bei einer Temperatur von 0 °C 1,6 g festes fein gemörsertes Natriumhydroxid gegeben und die Mischung anschließend 6 Stunden bei 40 °C gerührt. Zur Aufarbeitung wurde die Lösung dann unter vermindertem Druck eingedampft, der verbleibende Rückstand wurde in 100 ml Wasser aufgenommen und mit Essigsäureäthylester extrahiert. Nach Eindampfen des Essigsäureäthylesterextraktes wurden 6,55 g Rohprodukt als Rückstand erhalten. Dieses wurde chromatographisch über Kieselgel unter Verwendung von n-Hexan/Dichlormethan als Elutionsmittel gereinigt. Es wurden 6,03 g N-[2-((1R,3R,4S)1-Methyl-4-isopropylcyclohex-3-yloxy)-äthyl]-N-[2-(2,2-dimethyläthoxy)-äthyl]-N-benzylamin erhalten.

E) 3 g des vorstehend erhaltenen Produktes wurden in einem Gemisch aus 100 ml Äthanol und 30 ml Wasser gelöst. Zu der Lösung wurden nacheinander 1,5 g konzentrierte Salzsäurelösung und 2,5 g Hydrierkatalysator (Palladium/Kohle 5 %-ig) gegeben und die Mischung bei Raumtemperatur und einem Wasserstoffdruck von 4 bar hydriert. Nach ca. 3 Stunden war die Wasserstoffaufnahme beendet. Es wurde vom Katalysator abfiltriert und das Filtrat unter vermindertem Druck eingedampft. Der verbleibende Rückstand wurde in 100 ml Wasser aufgenommen und mit Essigsäureäthylester extrahiert. Die organische Phase wurde über Natriumsulfat getrocknet, filtriert und unter vermindertem Druck eingeengt. Die erhaltene rohe Titelverbindung wurde durch Chromatographie über Kieselgel unter Verwendung von n-Hexan/Dichlormethan als Elutionsmittel gereinigt. Es wurden 1,8 g öliges N-[2-((1R,3R,4S)-1-Methyl-4-isopropylcyclohex-3-yloxy)-äthyl]-N-[2-(2,2-dimethyläthoxy)-äthyl]-amin erhalten.
$[\alpha]_D^{20}$ = - 58,2° (c=1; Methanol).

Beispiel 17:

N-[2-((1R,3R,4S)-1-Methyl-4-isopropylcyclohex-3-yloxy)-äthyl]-N-[2-(4-phenylbutylamino)-äthyl]-N-methylamin.

3,07 g N-[2-((1R,3R,4S)-1-Methyl-4-isopropylcyclohex-3-yloxy)-äthyl]-N-(2-chloräthyl)-N-methylamin-hydrochlorid wurden mit 8,8 g 4-Phenylbutylamin gemischt. Das Reaktionsgemisch wurde 8 Stunden bei einer Temperatur von 45 °C gerührt. Zur Aufarbeitung wurde das Reaktionsgemisch durch Chromatographie über Kieselgel unter Verwendung von tert. Butylmethyläther gereinigt und dabei auch von überschüssigem 4-Phenylbutylamin befreit. Es wurden 3,52 g öliges N-[2-((1R,3R,4S)-1-Methyl-4-isopropylcyclohex-3-yloxy)-äthyl]-N-[2-(4-phenylbutylamino)-äthyl]-N-methylamin erhalten.

3,5 g der vorstehend erhaltenen öligen Titelbase wurden in 20 ml Tetrahydrofuran gelöst. Zu der Lösung wurde eine Lösung von 2,1 g Fumarsäure in 50 ml Tetrahydrofuran zugesetzt. Anschließend wurde dem Reaktionsgemisch bis zum Auftreten einer Trübung Diäthyläther zugesetzt und das Gemisch 12 Stunden im Kühlschrank aufbewahrt. Die ausgefallenen Kristalle wurden abfiltriert und getrocknet. Es wurden 4,4 g Difumarat der Titelverbindung mit einem Schmelzpunkt von 156 - 158 °C erhalten.
$[\alpha]_D^{20}$= - 30,7° (c=1; $CH_3OH$).

Beispiel 18:

N-[2-((1R,3R,4S)-1-Methyl-4-isopropylcyclohex-3-yloxy)-äthyl]-N-[2-(3,4,5-trimethoxybenzylamino)-äthyl]-N-methylamin.

6,5 g N-[2-((1R,3R,4S)-1-Methyl-4-isopropylcyclohex-3-yloxy)-äthyl]-N-(2-chloräthyl)-N-methylamin-hydrochlorid wurden in 15 ml Isopropanol gegeben. Dann wurden 25 ml 3,4,5-Trimethoxybenzylamin zugesetzt und das Reaktionsgemisch wurde 8 Stunden bei einer Temperatur von 40 °C gerührt. Zur Aufarbeitung wurde das Lösungsmittel abdestilliert und der Rückstand wurde in 150 ml tert. Butylmethyläther gelöst. Zur Entfernung von überschüssigem 3,4,5-Trimethoxybenzylamin wurde zu der Lösung verdünnte wäßrige Salzsäure gegeben (pH-Wert der wäßrigen Phase = 7). Die organische Phase wurde abgetrennt, über Natriumsulfat getrocknet, filtriert und unter vermindertem Druck eingeengt. Das als Rückstand verbleibende Rohprodukt wurde durch Chromatographie über Kieselgel gereinigt unter Verwendung von n-Hexan, welchem von 30 bis 100 % ansteigende Mengen Tetrahydrofuran zugesetzt wurden. Es wurden 8,2 g öliges N-[2-((1R,3R,4S)-1-Methyl-4-isopropylcyclohex-3-yloxy)-äthyl]-N-[2-(3,4,5-trimethoxybenzylamino)-äthyl]-N-methylamin erhalten.

1,7 g der vorstehend erhaltenen öligen Titelbase wurden in 10 ml Tetrahydrofuran gelöst. Zu der Lösung wurde eine Lösung von 0,95 g Fumarsäure in 10 ml Tetrahydrofuran gegeben. Das Reaktionsgemisch wurde unter vermindertem Druck eingeengt, dann wurden 20 ml Isopropanol zugesetzt und das Gemisch 5 Stunden bei 50 °C gerührt. Anschließend wurde auf Raumtemperatur abgekühlt und die ausgefallenen Kristalle wurden abfiltriert. Es wurden 1,50 g des Difumarates der Titelverbindung mit einem Schmelzpunkt von 127 bis 134 °C erhalten.

Beispiel 19:

N-[2-((1R,3R,4S)-1-Methyl-4-isopropylcyclohex-3-yloxy)-äthyl]-N-(2-[N'-(3,4,5-trimethoxybenzyl)-N'-methylamino]-äthyl)-N-methylamin.

4,1 g N-[2-((1R,3R,4S)-1-Methyl-4-isopropylcyclohex-3-yloxy)-äthyl]-N-[2-(3,4,5-trimethoxybenzylamino)-äthyl]-N-methylamin wurden in 100 ml Methanol gelöst. Zu der Lösung wurden 2,5 ml 37 %-ige Formaldehydlösung und ca. 3 g Raney Nickel gegeben. Das Reaktionsgemisch wurde dann 8 Stunden bei einem Wasserstoffdruck von 4,5 bar hydriert. Zur Aufarbeitung wurde vom Katalysator abfiltriert und das Filtrat unter vermindertem Druck eingeengt. Der Rückstand wurde in Dichlormethan aufgenommen und zweimal mit Wasser extrahiert. Die wäßrige Phase wurde noch zweimal mit Dichlormethan extrahiert. Die vereinigten organischen Phasen wurden anschließend über Natriumsulfat

getrocknet, filtriert und unter vermindertem Druck eingeengt. Es wurden 3,65 g N-[2-((1R,3R,4S)-1-Methyl-4-isopropylcyclohex-3-yloxy)-äthyl]-N-(2-[N'-(3,4,5-trimethoxybenzyl)-N'-methylamino]-äthyl)-N-methylamin erhalten.

Diese Titelbase wurde in 20 ml Tetrahydrofuran gelöst. Zu der Lösung wurde eine Lösung von 1,8 g Fumarsäure in 20 ml Tetrahydrofuran zugegeben. Der Lösung wurde bis zum Auftreten einer Trübung Diäthyläther zugesetzt und das Reaktionsgemisch wurde 12 Stunden im Kühlschrank stehengelassen. Dann wurden die ausgefallenen Kristalle abfiltriert, mit etwas Diäthyläther gewaschen und bei 35 °C getrocknet. Es wurden 3,45 g des Difumarates der Titelverbindung mit einem Schmelzpunkt von 170 bis 171 °C erhalten.

Analog zu den in den vorstehenden Beispielen beschriebenen Methoden wurden auch die folgenden in Tabelle I angegebenen Verbindungen der Formel I erhalten.

Tabelle I

| Bsp. Nr. | $R^1$ | n | m | Z | $R^2$ | $R^3$ | Salzform, phys. Konst. Fp. in °C $[\alpha]_D^{20}$ in ° (c=1, $CH_3OH$) |
|---|---|---|---|---|---|---|---|
| 20 | $(CH_3)_2CH$- | 2 | 2 | 0 | morph- | dihydronop. | 2.HCl, Fp = 170-180 |
| 21 | $CH_3$ | 2 | 2 | 0 | 3,4-di-$CH_3$O-phen-$(CH_2)_2$-N-<br>$CH_3$ | dihydronop. | 2.Fum, Fp = 155-156 |
| 22 | $CH_3$ | 2 | 2 | 0 | $CH_3$-$CH_2$-O- | dihydronop. | HCl, Fp = 72- 76 |
| 23 | $CH_3$ | 2 | 2 | 0 | 3,4-di-$CH_3$O-phen-$CH_2$-N-<br>$CH(CH_3)_2$ | L-menth. | 2.HCl Fp = 160-165 |
| 24 | $CH_3$ | 2 | 2 | 0 | morph- | D-menth. | 2.HCl, Fp = 200-202 |
| 25 | $CH_3$ | 2 | 2 | 0 | $CH_3$-$CH_2$-O- | D-menth. | Mal, Fp = 37- 39 |
| 26 | $CH_3$ | 2 | 2 | 0 | phen-O- | L-menth. | HCl, Fp = 90- 92 |
| 27 | $CH_3$ | 2 | 2 | 0 | $(CH_3)_2CH$-$CH_2$-O- | D-menth. | HCl, $[\alpha]_D^{20}$ =+56,4 |
| 28 | $CH_3$ | 2 | 2 | 0 | $(C_2H_5)_2N$- | L-menth. | 2.HCl, Fp = 168-169 |
| 29 | $CH_3$ | 2 | 2 | 0 | 4-(phen-$CH_2$)-pip- | L-menth. | 3.HCl, Fp = 215-218 |
| 30 | $CH_3$ | 2 | 2 | 0 | $(CH_3)_2CH$-$CH_2$-O- | L-menth. | Ox, Fp = 113-115 |
| 31 | $CH_3$ | 2 | 2 | 0 | phen-$CH_2$-N-<br>$CH_3$ | L-menth. | 2.HCl, Fp = 170-188 |
| 32 | $CH_3$ | 2 | 2 | 0 | pyrro- | L-menth. | 2.HCl, Fp = 225-230 |
| 33 | $CH_3$ | 2 | 2 | 0 | $(CH_3)_2CH$-O | L-menth. | Ox, Fp = 98-100 |
| 34 | $CH_3$ | 2 | 2 | 0 | 4-$CH_3$-pip- | L-menth. | 3.HCl, Fp = 235-239 |
| 35 | $CH_3$ | 2 | 2 | 0 | phen-$CH_2$-O- | D-menth. | Ox, Fp = 107-108 |

| Bsp. Nr. | $R^1$ | n | m | Z | $R^2$ | $R^3$ | Salzform, phys. Konst. Fp. in °C $[\alpha]_D^{20}$ in ° (c=1, $CH_3OH$) |
|---|---|---|---|---|---|---|---|
| 36 | $CH_3$ | 2 | 6 | 0 | morph- | L-menth. | 2.Ox, Fp = 113-115 |
| 37 | $CH_3$ | 2 | 2 | 0 | morph- | fen. | 2.HCl, Fp = 206-207 |
| 38 | $CH_3$ | 2 | 2 | 0 | morph- | born. | 2.HCl, Fp = 233-236 |
| 39 | $CH_3$ | 2 | 2 | 0 | $(CH_3)_2CH-CH_2-O-$ | L-menth. | D-tart, Fp = 68- 74 |
| 40 | $CH_3$ | 2 | 2 | 0 | $CH_3-(CH_2)_4-O-$ | L-menth. | HCl, $[\alpha]_D^{20}$ =-53,0 |
| 41 | H | 2 | 2 | 0 | morph- | L-menth. | 2.HCl, Fp = 193-199 |
| 42 | $CH_3$ | 3 | 2 | 0 | morph- | L-menth. | 2.HCl, Fp = 193-196 |
| 43 | $CH_3$ | 2 | 2 | 0 | 2-Cl-phen-$(CH_2)_2$-NH- | L-menth. | 2 Mal, FP = 160-161 |
| 44 | $CH_3$ | 2 | 2 | 0 | 3,4-di-Cl-phen-$CH_2$-NH- | L-menth. | 2 Fum, FP = 144-146 |
| 45 | $CH_3$ | 2 | 2 | 0 | 4-$CH_3$-phen-$CH_2$-NH- | L-menth. | 2 Fum, FP = 127-134 |
| 46 | $CH_3$ | 2 | 2 | 0 | 2-Cl-phen-$(CH_2)_2$-N-<br>\|<br>$CH_3$ | L-menth. | 2 Mal, FP = 144-145 |
| 47 | $CH_3$ | 2 | 2 | 0 | 3,4-O-$CH_2$-O-phen-$CH_2$-NH- | L-menth. | 2 Mal, FP = 147-149 |
| 48 | $CH_3$ | 2 | 2 | 0 | phen-$CH_2$-N-<br>\|<br>phen-$CH_2$ | L-menth. | 2 HCl, FP = 145-152 |
| 49 | $CH_3$ | 2 | 2 | 0 | 3-$CH_3$O-phen-$(CH_2)_2$-NH- | dihydronop. | 2 Fum, FP = 128-130 |
| 50 | $CH_3$ | 2 | 2 | 0 | phen-$(CH_2)_4$-N-<br>\|<br>$CH_3$ | L-menth. | 2 Fum, FP = 175-177 |

| Bsp. Nr. | $R^1$ | n | m | Z | $R^2$ | $R^3$ | Salzform, phys. Konst. Fp. in °C $[\alpha]_D^{20}$ in ° (c=1, $CH_3OH$) |
|---|---|---|---|---|---|---|---|
| 51 | $CH_3$ | 2 | 2 | 0 | phen-$(CH_2)_2$-NH- | dihydronop. | 1 Fum, Fp = 151-153 |
| 52 | $CH_3$ | 2 | 2 | 0 | 3-$CF_3$-phen-$CH_2$-NH- | L-menth. | Ba, $[\alpha]_D^{20}$ = - 48,1 |
| 53 | $CH_3$ | 2 | 2 | 0 | 3,4,5-tri-$CH_3O$-phen-$CH_2$-N(-2Cl-phen-$CH_2$)- | L-menth. | 2 HCl $[\alpha]_D^{20}$ = - 30,9 |
| 54 | $CH_3$ | 2 | 2 | 0 | 3,4-O-$CH_2O$-phen-$CH_2$-N(-3,4-di-$CH_3O$-phen-$CH_2$)- | L-menth. | 2 HCl $[\alpha]_D^{20}$ = - 32,1 |
| 55 | $CH_3$ | 2 | 2 | 0 | 3,4-di-$CH_3O$-phen-$CH_2$-N(-3,4-di-$CH_3O$-phen-$CH_2$)- | L-menth. | 2 Ox Fp = 92-94 |
| 56 | $CH_3$ | 2 | 2 | 0 | 2-$CH_3O$-phen-$CH_2$-N(-2-$CH_3O$-phen-$CH_2$)- | L-menth. | 2 Ox Fp = 67-70 |

morph = Morpholin-1-yl, phen = Phenyl, pip = Piperazin-1-yl, pyrro = Pyrrolidin-1-yl,
dihydronop. = cis-Dihydronopol = (1S,2S,5S)-2-(6,6-Dimethylbicyclo[3.1.1]-hept-2-yl)-Äthyl,
L-menth. = L-Menthyl = (1R,3R,4S)-1-Methyl-4-isopropylcyclohex-3-yl,
D-menth. = D-Menthyl = (1S,3S,4R)-1-Methyl-4-isopropylcyclohex-3-yl,
fen. = Fenchyl = (1S,2R,4R)-1,3,3-Trimethylbicyclo[2,1,1]hept-2-yl,
born. = Bornyl = (1S,2R,4S)-1,7,7-Trimethylbicyclo[2,2,1]hept-2-yl,
HCl = Hydrochlorid, Fum = Fumarat, Mal = Maleinat, Ox = Oxalat, D-tart = D-Tartrat, Ba = Base

In der nachfolgenden Tabelle II werden Ausgangsverbindungen der Formel X aufgeführt.

Tabelle II

| Bsp. Nr. | B | n | m | $R^2$ | $R^3$ | Salzform, phys. Konst. Fp. in °C $[\alpha]_D^{20}$ in ° (1 % in $CH_3OH$) |
|---|---|---|---|---|---|---|
| 101 | bz | 3 | 2 | morph- | L-menth. | 2.Mal, Fp = 116-118 |
| 102 | bz | 2 | 2 | $(CH_3)_2CH-CH_2-O-$ | L-menth. | HCl, $[\alpha]_D^{20} = -43,2°$ |
| 103 | bz | 2 | 2 | $(CH_3)_2CH-O-$ | L-menth. | HCl, $[\alpha]_D^{20} = -45,4°$ |
| 104 | bz | 2 | 2 | phen-$CH_2-N(CH_3)-$ | L-menth. | 2.HCl Fp = 152-156 |
| 105 | bz | 3 | 2 | $(CH_3)_2CH-CH_2-O-$ | L-menth. | Mal, Fp = 96- 97 |

morph = Morpholin-1-yl, phen = Phenyl, L-menth. = L-Menthyl, Mal = Maleinat,
HCl = Hydrochlorid

Beispiel I:

Man stellte Tabletten in folgender Zusammensetzung pro Tablette her:

| | |
|---|---|
| N-[2-((1R,3R,4S)-1-Methyl-4-isopropylcyclohex-3-yloxy)-äthyl]-N-[2-(morpholin-1-yl)-äthyl]-N-methylamin-dihydrochlorid | 20 mg |
| Maisstärke | 60 mg |
| Milchzucker | 135 mg |
| Gelatine (als 10%-ige Lösung) | 6 mg |

Der Wirkstoff, die Maisstärke und der Milchzucker wurden mit der 10%-igen Gelatine-Lösung eingedickt. Die Paste wurde zerkleinert und das entstandene Granulat wurde auf ein geeignetes Blech gebracht und bei 45 °C getrocknet. Das getrocknete Granulat wurde durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann zu Tabletten von 240 mg verpreßt.

$$R^3-Z-(CH_2)_n-\overset{R^{1}}{\overset{|}{N}}-(CH_2)_m-R^2 \qquad I$$

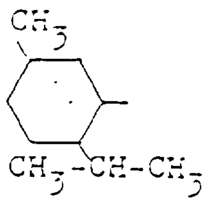

a

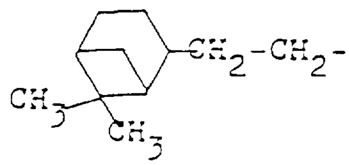

b

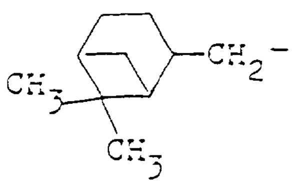

c

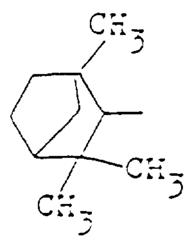

d

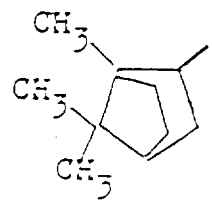

e

$$R^3-Z-(CH_2)_n-\overset{R^{1'}}{\overset{|}{N}}-(CH_2)_m-R^2 \qquad Ia$$

$$R^3-Z-(CH_2)_n-\overset{R^{1'}}{\overset{|}{N}}-(CH_2)_m-R^{2'} \qquad Ib$$

$$R^3-Z-(CH_2)_{n'}-\overset{R^{1'}}{\overset{|}{N}}-(CH_2)_{m'}-R^{2'} \qquad Ic$$

$$R^3-Z-(CH_2)_n-\overset{R^{1'}}{\overset{|}{N}}-(CH_2)_m-NH_2 \qquad Id$$

$$R^3-O-(CH_2)_n-\overset{H}{\overset{|}{N}}-(CH_2)_m-R^{2''} \qquad Ie$$

$$R^3-Z-(CH_2)_n-\overset{R^{1'}}{\overset{|}{N}}-(CH_2)_m-X \qquad II$$

$$\begin{array}{c} R^{1'} \\ | \\ R^3-Z-(CH_2)_n-N-(CH_2)_m-OH \end{array} \qquad IIa$$

$$\begin{array}{c} R^{1'} \\ | \\ R^3-Z-(CH_2)_n-N-(CH_2)_m-Y \end{array} \qquad IIb$$

$$\begin{array}{c} B \\ | \\ R^3-O-(CH_2)_n-N-(CH_2)_m-Y \end{array} \qquad II'$$

$H-R^2$ III

$Y-R^4$ III'

$$\begin{array}{l} \quad R^5 \\ \quad / \\ H-N \\ \quad \backslash \\ \quad R^6 \end{array} \qquad IIIa$$

$H-R^{2'}$ IIIb

$H-R^{2'''}$ IIIc

$H-R^{2''}$ IIId

$$\begin{array}{c} R^1 \\ | \\ R^3-Z-(CH_2)_{n-1}-CO-N-(CH_2)_{m-1}-D \end{array} \qquad IV$$

$R^3-Z-(CH_2)_n-Q$ V

$$\begin{array}{c} R^{1'} \\ | \\ R^3-Z-(CH_2)_n-N-H \end{array} \qquad Va$$

$$\begin{array}{c} R^{1'} \\ | \\ R^3-O-(CH_2)_n-N-H \end{array} \qquad Va'$$

$R^3-Z-(CH_2)_n-Y$ Vb

$R^3-O-(CH_2)_n-Y$ Vb'

$R^3-O-(CH_2)_n-NH_2$ V'

$Q'-(CH_2)_m-R^{2'}$ VI

$Y-(CH_2)_m-R^{2'}$ VIa

$$\begin{array}{l} \;\; R^{1'} \\ \;\; | \\ H-N-(CH_2)_m-R^{2'} \end{array} \qquad VIb$$

$$\begin{array}{l} \;\; R^1 \\ \;\; | \\ H-N-(CH_2)_m-R^{2'} \end{array} \qquad VI'$$

$Y-(CH_2)_m-R^{2}$ VI''

$R^3$-Z-H VII

$R^3$-O-H VIIa

$R^3$-S-H VIIb

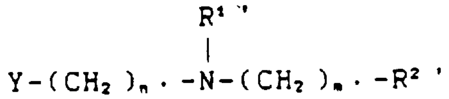 VIII

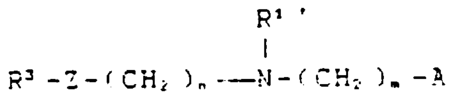 IX

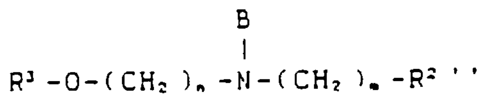 X

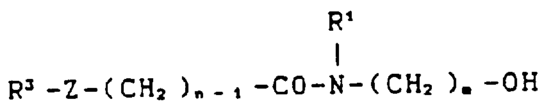 XI

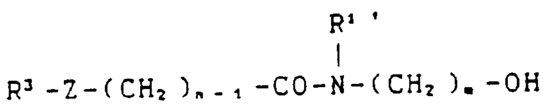 XIa

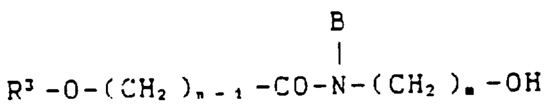 XI'

Y-$(CH_2)_m$-X XII

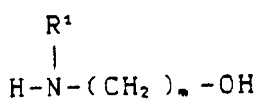 XIII

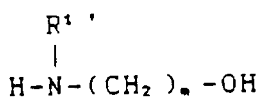 XIIIa

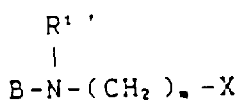 XIII'

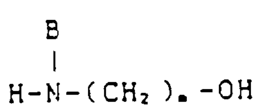 XIII''

$R^3$-Z-$(CH_2)_{n-1}$-COOH XIV

$R^3$-O-$(CH_2)_{n-1}$-COOH XIVa

T-$(CH_2)_{n-1}$-COOH XV

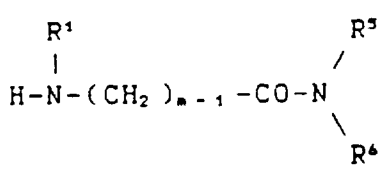 XVI

$$T-(CH_2)_{m-1}-COOH \qquad XVII$$

$$T-(CH_2)_{m-1}-CON\begin{matrix} R^5 \\ R^6 \end{matrix} \qquad XVIII$$

$$\begin{matrix} R^1 \\ | \\ H-N-H \end{matrix} \qquad XIX$$

$$\begin{matrix} R^{1'} \\ | \\ H-N-H \end{matrix} \qquad XIXa$$

$$\begin{matrix} R^{1'} \\ | \\ B-N-H \end{matrix} \qquad XIX'$$

$$R^3-Z-(CH_2)_{n-1}-CO-\begin{matrix} R^{1'} \\ | \\ N \end{matrix}-H \qquad XX$$

$$R^3-O-(CH_2)_n-\begin{matrix} R^{1'} \\ | \\ N \end{matrix}-B \qquad XXI$$

$$R^3-O-(CH_2)_n-\begin{matrix} B \\ | \\ N \end{matrix}-H \qquad XXI'$$

$$T-(CH_2)_n-X \qquad XXII$$

$$T-(CH_2)_n-OH \qquad XXIIa$$

$$B-\begin{matrix} R^{1'} \\ | \\ N \end{matrix}-(CH_2)_m-R^{2'''} \qquad XXIII$$

$$R^3-S-CO-CH_3 \qquad XXIV$$

$$R^4-OH \qquad XXV$$

$$R^4\ O-(CH_2)_{m-1}-COOH \qquad XXVI$$

$$Y-(CH_2)_n-\begin{matrix} R^{1'} \\ | \\ N \end{matrix}-B \qquad XXVII$$

## Patentansprüche

### Patentansprüche für folgende Vertragsstaaten : AT, BE, CH, DE, DK, FR, GB, IT, LU, NL, SE

**1.** Verbindungen der allgemeinen Formel I

$$R^3-Z-(CH_2)_n-\begin{matrix} R^1 \\ | \\ N \end{matrix}-(CH_2)_m-R^2 \qquad I$$

worin

n für 2-5 steht, m für 2-6 steht,

$R^1$ Wasserstoff oder Alkyl mit 1 - 4 Kohlenstoffatomen bedeutet,

$R^2$ für eine $OR^4$-Gruppe steht, worin $R^4$ Alkyl mit 1 - 4 Kohlenstoffatomen oder eine gegebenenfalls im Phenylring durch 1 - 3 Substituenten aus der Gruppe Alkyl mit 1 - 4 Kohlenstoffatomen, Alkoxy mit 1 - 4 Kohlenstoffatomen, Halogen und Alkylendioxy mit 1 - 2 Kohlenstoffatomen substituierte Phenyl- oder Phenyl-$C_1$-$C_3$-alkylgruppe bedeutet, oder

$R^2$ für eine

$R^5$
/
N -Gruppe steht,
\
$R^6$

worin

$R^5$ Wasserstoff, Alkyl mit 1 - 4 Kohlenstoffatomen oder eine gegebenenfalls im Phenylring durch 1 - 3 Substituenten aus der Gruppe Alkyl mit 1 - 4 Kohlenstoffatomen, Alkoxy mit 1 - 4 Kohlenstoffatomen, Halogen, Trifluormethyl und Alkylendioxy mit 1 - 2 Kohlenstoffatomen substituierte Phenyl- oder Phenyl-$C_1$-$C_3$-alkylgruppe bedeutet, und

$R^6$ Wasserstoff, Alkyl mit 1 - 4 Kohlenstoffatomen oder eine gegebenenfalls im Phenylring durch 1 - 3 Substituenten aus der Gruppe Alkyl mit 1 - 4 Kohlenstoffatomen, Alkoxy mit 1 - 4 Kohlenstoffatomen, Halogen, Trifluormethyl und Alkylendioxy mit 1 - 2 Kohlenstoffatomen substituierte Phenyl- oder Phenyl-$C_1$-$C_3$-alkylgruppe bedeutet, oder

$R^5$ und $R^6$ gemeinsam mit dem Stickstoffatom, an welches sie gebunden sind, einen gesättigten 5- oder 6-gliedrigen Heterocyclus darstellen, welcher gegebenenfalls ein zweites Heteroatom aus der Gruppe Sauerstoff und N-$R^7$, worin $R^7$ Alkyl mit 1 - 4 Kohlenstoffatomen oder Benzyl bedeutet, enthalten kann,

$R^3$ für einen gesättigten mono- oder bicyclischen Terpenkohlenwasserstoffrest mit 10 Kohlenstoffatomen oder für einen bicyclischen Kohlenwasserstoffrest mit 11 Kohlenstoffatomen mit der Formel b

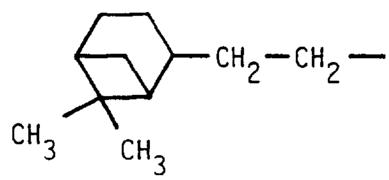

steht,

Z für Sauerstoff oder, falls $R^3$ einen Rest der Formel b bedeutet, auch für Schwefel steht,

und deren physiologisch verträglichen Säureadditionssalze.

**2.** Verbindungen nach Anspruch 1, dadurch gekennzeichnet, daß $R^3$ für einen mono- oder bicyclischen Kohlenwasserstoffrest mit 10 oder 11 Kohlenstoffatomen aus der Gruppe 1-Methyl-4-isopropylcyclohex-3-yl (= Menthyl) der Formel a, 2-(6,6-Dimethylbicyclo[3.1.1]hept-2-yl)-ethyl (= Dihydronopyl) der Formel b, 6,6-Dimethylbicyclo[3.1.1]hept-2-ylmethyl (= Myrtanyl) der Formel c, 1,3,3-Trimethylbicyclo[2.2.1]hept-2-yl (= Fenchyl) der Formel d und 1,7,7-Trimethylbicyclo[2.2.1]hept-2-yl (= Bornyl) der Formel e steht.

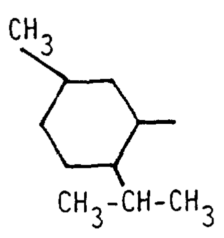

a

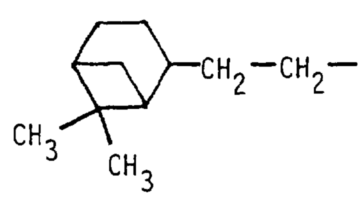

b

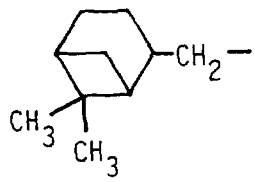

c

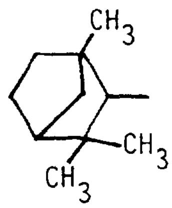

d

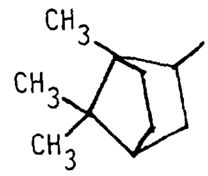

e

**3.** Verbindungen nach Anspruch 2, dadurch gekennzeichnet, daß $R^3$ für einen 1-Methyl-4-isopropylcyclohex-3-ylrest oder einen 2-(6,6-Dimethylbicyclo[3.1.1]hept-2-yl)-ethylrest steht.

**4.** Verbindungen nach Anspruch 3, dadurch gekennzeichnet, daß der Rest $R^3$ einen 1-Methyl-4-isopropylcyclohex-3-yl-rest darstellt, welcher überwiegend in der 1R,3R,4S-Konfiguration oder der 1S,3S,4R-Konfiguration vorliegt.

**5.** Verbindungen nach einem der vorstehenden Ansprüche, dadurch gekennzeichnet, daß $R^2$ für eine

$R^5$
\
N-Gruppe steht.
/
$R^6$

**6.** Verbindungen gemäß Anspruch 5, dadurch gekennnzeichnet, daß $R^5$ Alkyl mit 1 - 4 Kohlenstoffatomen oder eine gegebenenfalls im Phenylring durch 1 - 3 Substituenten aus der Gruppe Alkyl mit 1 - 4 Kohlenstoffatomen, Alkoxy mit 1 - 4 Kohlenstoffatomen, Halogen, Trifluormethyl und $C_1$-$C_2$-Alkylendioxy substituierte Phenyl-$C_1$-$C_2$-alkylgruppe bedeutet und $R^6$ eine gegebenenfalls im Phenylring durch 1 - 3 Substituenten aus der Gruppe Alkyl mit 1 - 4 Kohlenstoffatomen, Alkoxy mit 1 - 4 Kohlenstoffatomen, Halogen, Trifluormethyl und $C_1$-$C_2$-Alkylendioxy substituierte Phenyl-$C_1$-$C_2$-alkylgruppe bedeutet, oder $R^5$ und $R^6$ gemeinsam mit dem Stickstoffatom, an welches sie gebunden sind, einen 5- oder 6-gliedrigen gesättigten Heterocylus darstellen, welcher gegebenenfalls Sauerstoff als zweites Heteroatom enthält.

**7.** Arzneimittel, enthaltend eine pharmakologisch wirksame Menge einer Verbindung gemäß Anspruch 1 und übliche pharmazeutische Hilfs- und/oder Trägerstoffe.

**8.** Verfahren zur Herstellung von Verbindungen der allgemeinen Formel I

$$\begin{array}{c} R^1 \\ | \\ R^3\text{-}Z\text{-}(CH_2)_n\text{-}N\text{-}(CH_2)_m\text{-}R^2 \end{array} \qquad (I)$$

worin n, m, $R^1$, $R^2$, $R^3$ und Z die in Anspruch 1 angegebene Bedeutung besitzen
und deren physiologisch verträglichen Säureadditionssalzen, dadurch gekennzeichnet, daß man

a) zur Herstellung von Verbindungen der allgemeinen Formel Ia

$$\begin{array}{c} R^{1'} \\ | \\ R^3\text{-}Z\text{-}(CH_2)_n\text{-}N\text{-}(CH_2)_m\text{-}R^2 \end{array} \qquad (Ia)$$

worin n, m, $R^2$, $R^3$ und Z obige Bedeutung besitzen und $R^{1'}$ Alkyl mit 1-4 Kohlenstoffatomen bedeutet, Verbindungen der allgemeinen Formel II

$$\begin{array}{l} \qquad\qquad\quad R^{1'} \\ \qquad\qquad\quad | \\ R^3-Z-(CH_2)_n-N-(CH_2)_m-X \end{array} \qquad (II)$$

worin n, m, $R^{1'}$, $R^3$ und Z obige Bedeutung besitzen und X für eine abspaltbare Fluchtgruppe Y steht oder zur Herstellung solcher Verbindungen, worin $R^2$ eine $OR^4$-Gruppe darstellt, auch für Hydroxy stehen kann, umsetzt mit Verbindungen der allgemeinen Formel III

$$H-R^2 \qquad (III)$$

worin $R^2$ obige Bedeutung besitzt, oder falls X Hydroxy ist, auch mit einer Verbindung der allgemeinen Formel III'

$$Y-R^4 \qquad (III')$$

worin $R^4$ obige Bedeutung besitzt und Y für eine abspaltbare Fluchtgruppe steht, oder

b) zur Herstellung von Verbindungen der allgemeinen Formel I, Verbindungen der allgemeinen Formel IV

$$\begin{array}{l} \qquad\qquad\qquad\quad R^{1} \\ \qquad\qquad\qquad\quad | \\ R^3-Z-(CH_2)_{n-1}-CO-N-(CH_2)_{m-1}-D \end{array} \qquad (IV)$$

worin n, m, $R^1$, $R^3$ und Z obige Bedeutung besitzen und D für den Rest $-CH_2-N_3$, für eine $R_2-CH_2$-Gruppe, worin $R_2$ obige Bedeutung besitzt, oder für eine

$$\begin{array}{l} \quad R^5 \\ \ / \\ N-CO\ \text{-Gruppe,} \\ \ \backslash \\ \quad R^6 \end{array}$$

worin $R^5$ und $R^6$ obige Bedeutung besitzen, steht, reduziert, oder

c) zur Herstellung von Verbindungen der allgemeinen Formel Ib

$$\begin{array}{l} \qquad\qquad\quad R^{1'} \\ \qquad\qquad\quad | \\ R^3-Z-(CH_2)_n-N-(CH_2)_m-R^{2'} \end{array} \qquad (Ib)$$

worin n, m, $R^{1'}$, $R^3$ und Z obige Bedeutung besitzen und $R^{2'}$ die für $R^2$ angegebene Bedeutung mit Ausnahme von eine NH-Funktion enthaltenden Resten besitzt, Verbindungen der allgemeinen Formel V

$$R^3-Z-(CH_2)_n-Q \qquad (V)$$

worin n, $R^3$ und Z obige Bedeutung besitzen und Q für einen

$$\begin{array}{l} \quad R^{1'} \\ \ / \\ N\ \text{-Rest,} \\ \ \backslash \\ \quad H \end{array}$$

worin $R^{1'}$ obige Bedeutung besitzt, oder für eine abspaltbare Fluchtgruppe Y steht, umsetzt mit Verbindungen der allgemeinen Formel VI

$$Q'-(CH_2)_m-R^{2'} \qquad (VI)$$

worin m und $R^{2'}$ obige Bedeutung besitzen und Q' für eine abspaltbare Fluchtgruppe Y steht, falls Q den Rest

$$\begin{array}{l} \quad R^{1'} \\ \;\; / \\ -N \quad \text{bedeutet,} \\ \;\; \backslash \\ \quad H \end{array}$$

oder Q' für den Rest

$$\begin{array}{l} \quad R^{1'} \\ \;\; / \\ -N \quad \text{steht,} \\ \;\; \backslash \\ \quad H \end{array}$$

falls Q eine abspaltbare Fluchtgruppe Y bedeutet, oder

d) zur Herstellung von Verbindungen der allgemeinen Formel Ic

$$\begin{array}{c} R^{1'} \\ | \\ R^3\text{-}Z\text{-}(CH_2)_{n'}\text{-}N\text{-}(CH_2)_{m'}\text{-}R^{2'} \end{array} \qquad (Ic)$$

worin $R^{1'}$, $R^{2'}$, $R^3$ und Z obige Bedeutung besitzen und n' und m' die für n und m angegebene Bedeutung besitzen, wobei jedoch, falls $R^{2'}$ für einen $NR^5R^6$-Rest steht, n' und m' zusammen nicht 4 sein können, Verbindungen der allgemeinen Formel VII

$$R^3\text{-}Z\text{-}H \qquad (VII)$$

worin $R^3$ und Z obige Bedeutung besitzen, umsetzt mit Verbindungen der allgemeinen Formel VIII

$$\begin{array}{c} R^{1'} \\ | \\ Y\text{-}(CH_2)_{n'}\text{-}N\text{-}(CH_2)_{m'}\text{-}R^{2'} \end{array} \qquad (VIII)$$

worin n', m', $R^{1'}$, $R^{2'}$ und Y obige Bedeutung besitzen, oder

e) zur Herstellung von Verbindungen der allgemeinen Formel Id

$$\begin{array}{c} R^{1'} \\ | \\ R^3\text{-}Z\text{-}(CH_2)_n\text{-}N\text{-}(CH_2)_m\text{-}NH_2 \end{array} \qquad (Id)$$

worin n, m, $R^{1'}$, $R^3$ und Z obige Bedeutung besitzen, in Verbindungen der allgemeinen Formel IX

$$\begin{array}{c} R^{1'} \\ | \\ R^3\text{-}Z\text{-}(CH_2)_n\text{-}N\text{-}(CH_2)_m\text{-}A \end{array} \qquad (IX)$$

worin n, m, $R^{1'}$, $R^3$ und Z obige Bedeutung besitzen und A einen Azid- oder Phthalimidrest bedeutet, den Rest A in die $NH_2$-Gruppe überführt, oder

f) zur Herstellung von Verbindungen der allgemeinen Formel Ie

$$\begin{array}{c} H \\ | \\ R^3\text{-}O\text{-}(CH_2)_n\text{-}N\text{-}(CH_2)_m\text{-}R^{2''} \end{array} \qquad (Ie)$$

worin n, m und $R^3$ obige Bedeutung besitzen und $R^{2''}$ die für $R^2$ angegebene Bedeutung mit Ausnahme von eine gegebenenfalls substituierte Benzylgruppe enthaltenden Resten besitzt, aus Verbindungen der allgemeinen Formel X

$$R^3-O-(CH_2)_n-\overset{\displaystyle B}{\overset{|}{N}}-(CH_2)_m-R^{2''} \qquad (X)$$

worin n, m, $R^{2''}$ und $R^3$ obige Bedeutung besitzen und B für eine hydrogenolytisch abspaltbare Gruppe steht, die Gruppe B hydrogenolytisch abspaltet, und gewünschtenfalls in erhaltene Verbindungen der allgemeinen Formel I, worin $R^1$ Wasserstoff bedeutet, einen Alkylrest $R^{1'}$ mit 1 - 4 Kohlenstoffatomen oder in erhaltene Verbindungen der Formel I, worin $R^1$ Alkyl mit 1 - 4 Kohlenstoffatomen ist und $R^2$ eine freie NH-Funktion enthält, einen Alkylrest $R^{5'}$ mit 1 - 4 Kohlenstoffatomen oder eine gegebenenfalls durch 1 - 3 Substituenten aus der Gruppe Alkyl mit 1 - 4 Kohlenstoffatomen, Alkoxy mit 1 - 4 Kohlenstoffatomen, Halogen, Trifluormethyl und Alkylendioxy mit 1 - 2 Kohlenstoffatomen substituierte Phenyl-$C_1$-$C_3$-alkylgruppe $R^{5'}$ einführt, und gewünschtenfalls freie Verbindungen der Formel I in ihre Säureadditionssalze überführt oder die Säureadditionssalze in die freien Verbindungen der Formel I überführt.

**Patentansprüche für folgende Vertragsstaaten : ES, GR**

**1.** Verfahren zur Herstellung von Verbindungen der allgemeinen Formel I

$$R^3-Z-(CH_2)_n-\overset{\displaystyle R^1}{\overset{|}{N}}-(CH_2)_m-R^2 \qquad I$$

worin

n für 2-5 steht, m für 2-6 steht,
$R^1$ Wasserstoff oder Alkyl mit 1 - 4 Kohlenstoffatomen bedeutet,
$R^2$ für eine $OR^4$-Gruppe steht, worin $R^4$ Alkyl mit 1 - 4 Kohlenstoffatomen oder eine gegebenenfalls im Phenylring durch 1 - 3 Substituenten aus der Gruppe Alkyl mit 1 - 4 Kohlenstoffatomen, Alkoxy mit 1 - 4 Kohlenstoffatomen, Halogen und Alkylendioxy mit 1 - 2 Kohlenstoffatomen substituierte Phenyl- oder Phenyl-$C_1$-$C_3$-alkylgruppe bedeutet, oder
$R^2$ für eine

$$N\begin{matrix} / R^5 \\ \backslash R^6 \end{matrix} \text{-Gruppe steht,}$$

worin

$R^5$ Wasserstoff, Alkyl mit 1 - 4 Kohlenstoffatomen oder eine gegebenenfalls im Phenylring durch 1 - 3 Substituenten aus der Gruppe Alkyl mit 1 - 4 Kohlenstoffatomen, Alkoxy mit 1 - 4 Kohlenstoffatomen, Halogen, Trifluormethyl und Alkylendioxy mit 1 - 2 Kohlenstoffatomen substituierte Phenyl- oder Phenyl-$C_1$-$C_3$-alkylgruppe bedeutet, und
$R^6$ Wasserstoff, Alkyl mit 1 - 4 Kohlenstoffatomen oder eine gegebenenfalls im Phenylring durch 1 - 3 Substituenten aus der Gruppe Alkyl mit 1 - 4 Kohlenstoffatomen, Alkoxy mit 1 - 4 Kohlenstoffatomen, Halogen, Trifluormethyl und Alkylendioxy mit 1 - 2 Kohlenstoffatomen substituierte Phenyl- oder Phenyl-$C_1$-$C_3$-alkylgruppe bedeutet, oder
$R^5$ und $R^6$ gemeinsam mit dem Stickstoffatom, an welches sie gebunden sind, einen gesättigten 5- oder 6-gliedrigen Heterocyclus darstellen, welcher gegebenenfalls ein zweites Heteroatom aus der Gruppe Sauerstoff und N-$R^7$, worin $R^7$ Alkyl mit 1 - 4 Kohlenstoffatomen oder Benzyl bedeutet, enthalten kann,
$R^3$ für einen gesättigten mono- oder bicyclischen Terpenkohlenwasserstoffrest mit 10 Kohlenstoffatomen oder für einen bicyclischen Kohlenwasserstoffrest mit 11 Kohlenstoffatomen mit der Formel b

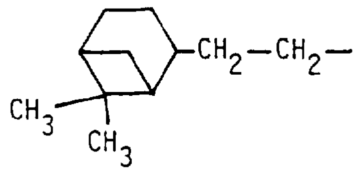

steht,

Z für Sauerstoff oder, falls $R^3$ einen Rest der Formel b bedeutet, auch für Schwefel steht,

und deren physiologisch verträglichen Säureadditionssalzen, dadurch gekennzeichnet, daß man

a) zur Herstellung von Verbindungen der allgemeinen Formel Ia

$$R^3-Z-(CH_2)_n-\underset{}{\overset{R^{1'}\atop|}{N}}-(CH_2)_m-R^2 \qquad (Ia)$$

worin n, m, $R^2$, $R^3$ und Z obige Bedeutung besitzen und $R^{1'}$ Alkyl mit 1 - 4 Kohlenstoffatomen bedeutet, Verbindungen der allgemeinen Formel II

$$R^3-Z-(CH_2)_n-\overset{R^{1'}\atop|}{N}-(CH_2)_m-X \qquad (II)$$

worin n, m, $R^{1'}$, $R^3$ und Z obige Bedeutung besitzen und X für eine abspaltbare Fluchtgruppe Y steht oder zur Herstellung solcher Verbindungen, worin $R^2$ eine $OR^4$-Gruppe darstellt, auch für Hydroxy stehen kann, umsetzt mit Verbindungen der allgemeinen Formel III

$$H-R^2 \qquad (III)$$

worin $R^2$ obige Bedeutung besitzt, oder falls X Hydroxy ist, auch mit einer Verbindung der allgemeinen Formel III'

$$Y-R^4 \qquad (III')$$

worin $R^4$ obige Bedeutung besitzt und Y für eine abspaltbare Fluchtgruppe steht, oder

b) zur Herstellung von Verbindungen der allgemeinen Formel I, Verbindungen der allgemeinen Formel IV

$$R^3-Z-(CH_2)_{n-1}-CO-\overset{R^1\atop|}{N}-(CH_2)_{m-1}-D \qquad (IV)$$

worin n, m, $R^1$, $R^3$ und Z obige Bedeutung besitzen und D für den Rest $-CH_2-N_3$, für eine $R_2-CH_2$-Gruppe, worin $R_2$ obige Bedeutung besitzt, oder für eine

$$\begin{matrix} R^5 \\ / \\ N-CO \\ \backslash \\ R^6 \end{matrix} \text{-Gruppe,}$$

worin $R^5$ und $R^6$ obige Bedeutung besitzen, steht, reduziert, oder

c) zur Herstellung von Verbindungen der allgemeinen Formel Ib

$$R^3-Z-(CH_2)_n-\overset{R^{1'}\atop|}{N}-(CH_2)_m-R^{2'} \qquad (Ib)$$

worin n, m, $R^{1'}$, $R^3$ und Z obige Bedeutung besitzen und $R^{2'}$ die für $R^2$ angegebene Bedeutung mit Ausnahme von eine NH-Funktion enthaltenden Resten besitzt, Verbindungen der allgemeinen Formel V

$$R^3\text{-Z-}(CH_2)_n\text{-Q} \qquad (V)$$

worin n, $R^3$ und Z obige Bedeutung besitzen und Q für einen

```
   R1'
  /
 N -Rest,
  \
   H
```

worin $R^{1'}$ obige Bedeutung besitzt, oder für eine abspaltbare Fluchtgruppe Y steht, umsetzt mit Verbindungen der allgemeinen Formel VI

$$Q'\text{-}(CH_2)_m\text{-}R^{2'} \qquad (VI)$$

worin m und $R^{2'}$ obige Bedeutung besitzen und Q' für eine abspaltbare Fluchtgruppe Y steht, falls Q den Rest

```
    R1'
   /
 -N  bedeutet,
   \
    H
```

oder Q' für den Rest

```
    R1'
   /
 -N  steht,
   \
    H
```

falls Q eine abspaltbare Fluchtgruppe Y bedeutet, oder

d) zur Herstellung von Verbindungen der allgemeinen Formel Ic

```
                    R1'
                    |
R3-Z-(CH2)n'-N-(CH2)m'-R2'        (Ic)
```

worin $R^{1'}$, $R^{2'}$, $R^3$ und Z obige Bedeutung besitzen und n' und m' die für n und m angegebene Bedeutung besitzen, wobei jedoch, falls $R^{2'}$ für einen $NR^5R^6$-Rest steht, n' und m' zusammen nicht 4 sein können, Verbindungen der allgemeinen Formel VII

$$R^3\text{-Z-H} \qquad (VII)$$

worin $R^3$ und Z obige Bedeutung besitzen, umsetzt mit Verbindungen der allgemeinen Formel VIII

```
              R1'
              |
Y-(CH2)n'-N-(CH2)m'-R2'        (VIII)
```

worin n', m', $R^{1'}$, $R^{2'}$ und Y obige Bedeutung besitzen, oder

e) zur Herstellung von Verbindungen der allgemeinen Formel Id

$$R^3\text{-}Z\text{-}(CH_2)_n\text{-}\overset{\overset{R^{1'}}{|}}{N}\text{-}(CH_2)_m\text{-}NH_2 \qquad (Id)$$

worin n, m, $R^{1'}$, $R^3$ und Z obige Bedeutung besitzen, in Verbindungen der allgemeinen Formel IX

$$R^3\text{-}Z\text{-}(CH_2)_n\text{-}\overset{\overset{R^{1'}}{|}}{N}\text{-}(CH_2)_m\text{-}A \qquad (IX)$$

worin n, m, $R^{1'}$, $R^3$ und Z obige Bedeutung besitzen und A einen Azid- oder Phthalimidrest bedeutet, den Rest A in die $NH_2$-Gruppe überführt, oder

f) zur Herstellung von Verbindungen der allgemeinen Formel Ie

$$R^3\text{-}O\text{-}(CH_2)_n\text{-}\overset{\overset{H}{|}}{N}\text{-}(CH_2)_m\text{-}R^{2''} \qquad (Ie)$$

worin n, m und $R^3$ obige Bedeutung besitzen und $R^{2''}$ die für $R^2$ angegebene Bedeutung mit Ausnahme von eine gegebenenfalls substituierte Benzylgruppe enthaltenden Resten besitzt, aus Verbindungen der allgemeinen Formel X

$$R^3\text{-}O\text{-}(CH_2)_n\text{-}\overset{\overset{B}{|}}{N}\text{-}(CH_2)_m\text{-}R^{2''} \qquad (X)$$

worin n, m, $R^{2''}$ und $R^3$ obige Bedeutung besitzen und B für eine hydrogenolytisch abspaltbare Gruppe steht, die Gruppe B hydrogenolytisch abspaltet, und gewünschtenfalls in erhaltene Verbindungen der allgemeinen Formel I, worin $R^1$ Wasserstoff bedeutet, einen Alkylrest $R^{1'}$ mit 1 - 4 Kohlenstoffatomen oder in erhaltene Verbindungen der Formel I, worin $R^1$ Alkyl mit 1 - 4 Kohlenstoffatomen ist und $R^2$ eine freie NH-Funktion enthält, einen Alkylrest $R^{5'}$ mit 1 - 4 Kohlenstoffatomen oder eine gegebenenfalls durch 1 - 3 Substituenten aus der Gruppe Alkyl mit 1 - 4 Kohlenstoffatomen, Alkoxy mit 1 - 4 Kohlenstoffatomen, Halogen, Trifluormethyl und Alkylendioxy mit 1 - 2 Kohlenstoffatomen substituierte Phenyl-$C_1$-$C_3$-alkylgruppe $R^{5'}$ einführt, und gewünschtenfalls freie Verbindungen der Formel I in ihre Säureadditionssalze überführt oder die Säureadditionssalze in die freien Verbindungen der Formel I überführt.

**2.** Verfahren zur Herstellung von Verbindungen nach Anspruch 1, dadurch gekennzeichnet, daß $R^3$ für einen mono- oder bicyclischen Kohlenwasserstoffrest mit 10 oder 11 Kohlenstoffatomen aus der Gruppe 1-Methyl-4-isopropylcyclohex-3-yl (= Menthyl) der Formel a, 2-(6,6-Dimethylbicyclo-[3.1.1]hept-2-yl)-ethyl (= Dihydronopyl) der Formel b, 6,6-Dimethylbicyclo[3.1.1]hept-2ylmethyl (= Myrtanyl) der Formel c, 1,3,3-Trimethylbicyclo[2.2.1]hept-2-yl (= Fenchyl) der Formel d und 1,7,7-Trimethylbicyclo[2.2.1]-hept-2-yl (= Bornyl) der Formel e steht.

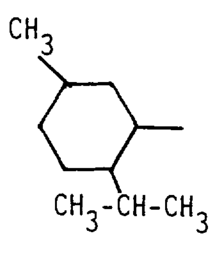

a

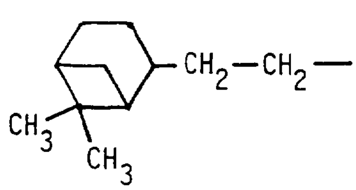

b

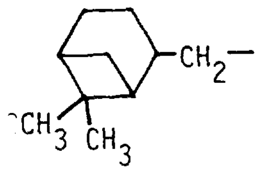 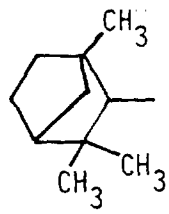 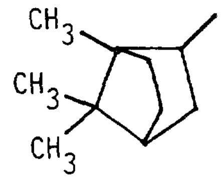

c d e

**3.** Verfahren zur Herstellung von Verbindungen nach Anspruch 2, dadurch gekennzeichnet, daß $R^3$ für einen 1-Methyl-4-isopropylcyclohex-3-yl-rest oder einen 2-(6,6-Dimethylbicyclo[3.1.1]hept-2-yl)-ethylrest steht.

**4.** Verfahren zur Herstellung von Verbindungen nach Anspruch 3, dadurch gekennzeichnet, daß der Rest $R^3$ einen 1-Methyl-4-isopropylcyclohex-3-yl-rest darstellt, welcher überwiegend in der 1R,3R,4S-Konfiguration oder der 1S, 3S,4R-Konfiguration vorliegt.

**5.** Verfahren zur Herstellung von Verbindungen nach einem der vorstehenden Ansprüche, dadurch gekennzeichnet, daß $R^2$ für eine

$R^5$
\
N-Gruppe steht.
/
$R^6$

**6.** Verfahren zur Herstellung von Verbindungen gemäß Anspruch 5, dadurch gekennnzeichnet, daß $R^5$ Alkyl mit 1 - 4 Kohlenstoffatomen oder eine gegebenenfalls im Phenylring durch 1 - 3 Substituenten aus der Gruppe Alkyl mit 1 - 4 Kohlenstoffatomen, Alkoxy mit 1 - 4 Kohlenstoffatomen, Halogen, Trifluormethyl und $C_1$-$C_2$-A-Alkylendioxy substituierte Phenyl-$C_1$-$C_2$-alkylgruppe bedeutet und $R^6$ eine gegebenenfalls im Phenylring durch 1 - 3 Substituenten aus der Gruppe Alkyl mit 1 - 4 Kohlenstoffatomen, Alkoxy mit 1 - 4 Kohlenstoffatomen , Halogen, Trifluormethyl und $C_1$-$C_2$-AlkyDendioxy substituierte Phenyl-$C_1$-$C_2$-alkylgruppe bedeutet, oder $R^5$ und $R^6$ gemeinsam mit dem Stickstoffatom, an welches sie gebunden sind, einen 5- oder 6-gliedrigen gesättigten Heterocylus darstellen, welcher gegebenenfalls Sauerstoff als zweites Heteroatom enthält.

**Claims**

**Claims for the following Contracting States : AT, BE, CH, DE, DK, FR, GB, IT, LI, LU, NL, SE**

**1.** Compounds of the general formula I

$$\begin{array}{c} R^1 \\ | \\ R^3\text{-}Z\text{-}(CH_2)_n\text{-}N\text{-}(CH_2)_m\text{-}R^2 \end{array} \qquad I$$

in which

n represents 2-5, m represents 2-6,
$R^1$ denotes hydrogen or alkyl with 1 - 4 carbon atoms,
$R^2$ represents an $OR^4$ group in which $R^4$ denotes alkyl with 1 - 4 carbon atoms or a phenyl or phenyl-$C_1$-$C_3$-alkyl- group which is optionally substituted in the phenyl ring by 1 - 3 substituents from the group alkyl with 1 - 4 carbon atoms, alkoxy with 1 - 4 carbon atoms, halogen and alkylenedioxy with 1 - 2 carbon atoms, or
$R^2$ represents an

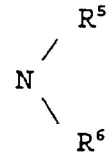

group in which

$R^5$ denotes hydrogen, alkyl with 1 - 4 carbon atoms or a phenyl or phenyl-$C_1$-$C_3$-alkyl group which is optionally substituted in the phenyl ring by 1 - 3 substituents from the group alkyl with 1 - 4 carbon atoms, alkoxy with 1 - 4 carbon atoms, halogen, trifluoromethyl and alkylenedioxy with 1 - 2 carbon atoms, and

$R^6$ denotes hydrogen, alkyl with 1 - 4 carbon atoms or a phenyl or phenyl-$C_1$-$C_3$-alkyl group which is optionally substituted in the phenyl ring by 1 - 3 substituents from the group alkyl with 1 - 4 carbon atoms, alkoxy with 1 - 4 carbon atoms, halogen, trifluoromethyl and alkylenedioxy with 1 - 2 carbon atoms, or

$R^5$ and $R^6$, together with the nitrogen atom to which they are bonded, represent a saturated 5- or 6-membered heterocycle, which can optionally contain a second hetero atom from the group oxygen and N-$R^7$, in which $R^7$ denotes alkyl with 1 - 4 carbon atoms or benzyl,

$R^3$ represents a saturated mono- or bicyclic terpene hydrocarbon radical having 10 carbon atoms or a bicyclic hydrocarbon radical having 11 carbon atoms, of the formula b

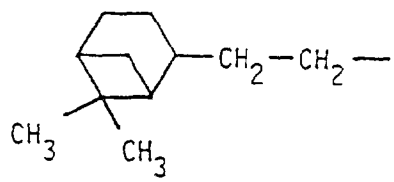

Z represents oxygen or, if $R^3$ denotes a radical of the formula b, also represents sulphur,

and their physiologically tolerable acid addition salts.

**2.** Compounds according to Claim 1, characterised in that $R^3$ represents a mono- or bicyclic hydrocarbon radical having 10 or 11 carbon atoms from the group 1-methyl-4-isopropylcyclohex-3-yl (= menthyl) of the formula a, 2-(6,6-dimethylbicyclo[3.1.1]hept-2-yl)ethyl (= dihydronopyl) of the formula b, 6,6-dimethylbicyclo-[3.1.1]hept-2-ylmethyl (= myrtanyl) of the formula c, 1,3,3-trimethylbicyclo[2.2.1]hept-2-yl (= fenchyl) of the formula d and 1,7,7-trimethylbicyclo[2.2.1]hept-2-yl (= bornyl) of the formula e

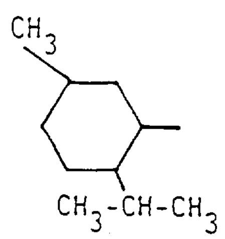

a

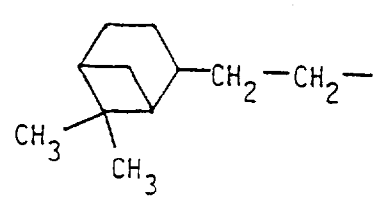

b

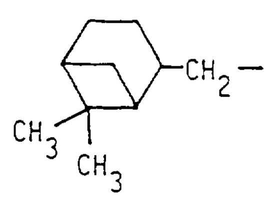

c

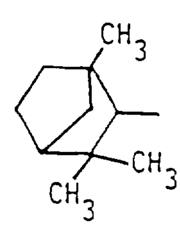

d

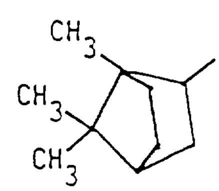

e

**3.** Compounds according to Claim 2, characterised in that $R^3$ represents a 1-methyl-4-isopropylcyclohex-3-yl radical or a 2-(6,6-dimethylbicyclo[3.1.1]hept-2-yl) ethyl radical.

**4.** Compounds according to Claim 3, characterised in that the radical $R^3$ represents a 1-methyl-4-isopropylcyclohex-3-yl radical which is present predominantly in the 1R,3R,4S configuration or the 1S,3S,4R configuration.

**5.** Compounds according to one of the above claims, characterised in that $R^2$ represents an

$$\begin{matrix} R^5 & \\ & \backslash \\ & N \text{ group.} \\ & / \\ R^6 & \end{matrix}$$

**6.** Compounds according to Claim 5, characterised in that $R^5$ denotes alkyl with 1 - 4 carbon atoms or a phenyl-$C_1$-$C_2$-alkyl group optionally substituted in the phenyl ring by 1 - 3 substituents from the group alkyl with 1 - 4 carbon atoms, alkoxy with 1 - 4 carbon atoms, halogen, trifluoromethyl and $C_1$-$C_2$-alkylenedioxy and $R^6$ denotes a phenyl-$C_1$-$C_2$-alkyl group which is optionally substituted in the phenyl ring by 1 - 3 substituents from the group alkyl with 1 - 4 carbon atoms, alkoxy with 1 - 4 carbon atoms, halogen, trifluoromethyl and $C_1$-$C_2$-alkylenedioxy, or $R^5$ and $R^6$, together with the nitrogen atom to which they are bonded, represent a 5- or 6-membered saturated heterocycle which optionally contains oxygen as a second hetero atom.

**7.** Medicaments, containing a pharmacologically active amount of a compound according to Claim 1, and customary pharmaceutical auxiliaries and/or excipients.

**8.** Process for the preparation of compounds of the general formula I

$$R^3-Z-(CH_2)_n-\underset{\displaystyle R^1}{\overset{|}{N}}\!\!-(CH_2)_m-R^2 \qquad (I)$$

in which n, m, $R^1$, $R^2$, $R^3$ and Z have the meanings given in Claim 1,
and their physiologically tolerable acid addition salts, characterised in that

a) for the preparation of compounds of the general formula Ia

$$R^3-Z-(CH_2)_n-\overset{\displaystyle R^{1'}}{\overset{|}{N}}-(CH_2)_m-R^2 \qquad (Ia)$$

in which n, m, $R^2$, $R^3$ and Z have the above meaning and $R^{1'}$ denotes alkyl with 1 - 4 carbon atoms, compounds of the general formula II

$$R^3-Z-(CH_2)_n-\overset{\displaystyle R^{1'}}{\overset{|}{N}}-(CH_2)_m-X \qquad (II)$$

in which n, m, $R^{1'}$, $R^3$ and Z have the above meaning and X represents a removable leaving group Y or, for the preparation of those compounds in which $R^2$ represents an $OR^4$ group, can also represent hydroxyl, are reacted with compounds of the general formula III

$$H\text{-}R^2 \qquad (III)$$

in which $R^2$ has the above meaning, or if X is hydroxyl, also with a compound of the general formula III'

$$Y\text{-}R^4 \qquad (III')$$

in which $R^4$ has the above meaning and Y represents a removable leaving group, or

b) for the preparation of compounds of the general formula I, compounds of the general formula IV

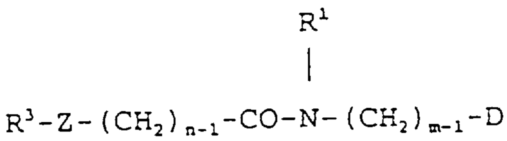 (IV)

in which n, m, $R^1$, $R^3$ and Z have the above meaning and D represents the radical $-CH_2-N_3$, an $R_2-CH_2$ group in which $R_2$ has the above meaning, or an

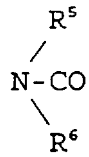 group,

in which $R^5$ and $R^6$ have the above meaning, are reduced, or

c) for the preparation of compounds of the general formula Ib

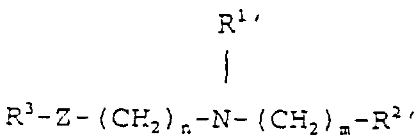 (Ib)

in which n, m, $R^{1'}$, $R^3$ and Z have the above meaning and $R^{2'}$ has the meaning given for $R^2$ with the exception of radicals containing an NH function, compounds of the general formula V

$$R^3-Z-(CH_2)_n-Q \qquad (V)$$

in which n, $R^3$ and Z have the above meaning and Q represents an

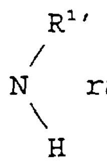 radical,

in which $R^{1'}$ has the above meaning, or represents a removable leaving group Y, are reacted with compounds of the general formula VI

$$Q'-(CH_2)_m-R^{2'} \qquad (VI)$$

in which m and $R^{2'}$ have the above meaning and Q' represents a removable leaving group Y if Q denotes the radical

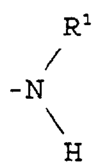 or

Q' represents the radical

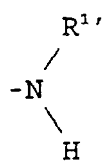

if Q denotes a removable leaving group Y, or

d) for the preparation of compounds of the general formula Ic

$$R^3-Z-(CH_2)_{n'}-N(R^{1'})-(CH_2)_{m'}-R^{2'} \qquad (Ic)$$

in which $R^{1'}$, $R^{2'}$, $R^3$ and Z have the above meaning and n' and m' have the meaning given for n and m, where, however, if $R^{2'}$ represents an $NR^5R^6$ radical, n' and m' together cannot be 4, compounds of the general formula VII

$$R^3\text{-Z-H} \qquad (VII)$$

in which $R^3$ and Z have the above meaning, are reacted with compounds of the general formula VIII

$$Y-(CH_2)_{n'}-N(R^{1'})-(CH_2)_{m'}-R^{2'} \qquad (VIII)$$

in which n', m', $R^{1'}$, $R^{2'}$ and Y have the above meaning, or

e) for the preparation of compounds of the general formula Id

$$R^3-Z-(CH_2)_n-N(R^{1'})-(CH_2)_m-NH_2 \qquad (Id)$$

in which n, m, $R^{1'}$, $R^3$ and Z have the above meaning, in compounds of the general formula IX

$$R^3-Z-(CH_2)_n-N(R^{1'})-(CH_2)_m-A \qquad (IX)$$

in which n, m, $R^{1'}$, $R^3$ and Z have the above meaning and A denotes an azide or phthalimide radical, the radical A is converted into the $NH_2$ group, or

f) for the preparation of compounds of the general formula Ie

$$R^3-O-(CH_2)_n-N(H)-(CH_2)_m-R^{2''} \qquad (Ie)$$

in which n, m and $R^3$ have the above meaning and $R^{2''}$ has the meaning given for $R^2$ with the exception of radicals containing an optionally substituted benzyl group, the group B is removed by hydrogenolysis from compounds of the general formula X

$$R^3-O-(CH_2)_n-N(B)-(CH_2)_m-R^{2''} \qquad (X)$$

in which n, m, $R^{2''}$ and $R^3$ have the above meaning and B represents a group which is removable by hydrogenolysis, and, if desired, in resulting compounds of the general formula I, in which $R^1$ denotes hydrogen, an alkyl radical $R^{1'}$ with 1 - 4 carbon atoms or, in resulting compounds of the formula I in which $R^1$ is alkyl with 1 - 4 carbon atoms and $R^2$ contains a free NH function, an alkyl radical $R^{5'}$ with 1 - 4 carbon atoms or a phenyl-$C_1$-$C_3$-alkyl group $R^{5'}$ which is optionally substituted by 1 - 3 substituents from the group alkyl with 1 - 4 carbon atoms, alkoxy with 1 - 4 carbon atoms, halogen, trifluoromethyl and alkylenedioxy with 1 - 2 carbon atoms, is introduced and, if desired, free compounds of the formula I are converted into their acid addition salts or the acid addition salts are converted into the free compounds of the formula I.

**Claims for the following Contracting States : ES, GR**

1. Process for the preparation of compounds of the general formula I

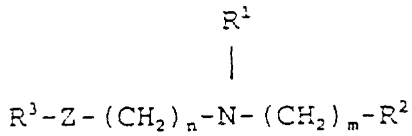

I

in which

n represents 2-5, m represents 2-6,

$R^1$ denotes hydrogen or alkyl with 1 - 4 carbon atoms,

$R^2$ represents an $OR^4$ group in which $R^4$ denotes alkyl with 1 - 4 carbon atoms or a phenyl or phenyl-$C_1$-$C_3$-alkyl group which is optionally substituted in the phenyl ring by 1 - 3 substituents from the group alkyl with 1 - 4 carbon atoms, alkoxy with 1 - 4 carbon atoms, halogen and alkylenedioxy with 1 - 2 carbon atoms, or

$R^2$ represents an

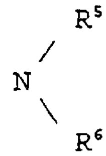

group in which

$R^5$ denotes hydrogen, alkyl with 1 - 4 carbon atoms or a phenyl or phenyl-$C_1$-$C_3$-alkyl group which is optionally substituted in the phenyl ring by 1 - 3 substituents from the group alkyl with 1 - 4 carbon atoms, alkoxy with 1 - 4 carbon atoms, halogen, trifluoromethyl and alkylenedioxy with 1 - 2 carbon atoms, and

$R^6$ denotes hydrogen, alkyl with 1 - 4 carbon atoms or a phenyl or phenyl-$C_1$-$C_3$-alkyl group which is optionally substituted in the phenyl ring by 1 - 3 substituents from the group alkyl with 1 - 4 carbon atoms, alkoxy with 1 - 4 carbon atoms, halogen, trifluoromethyl and alkylenedioxy with 1 - 2 carbon atoms, or

$R^5$ and $R^6$, together with the nitrogen atom to which they are bonded, represent a saturated 5- or 6-membered heterocycle, which can optionally contain a second hetero atom from the group oxygen and N-$R^7$, in which $R^7$ denotes alkyl with 1 - 4 carbon atoms or benzyl,

$R^3$ represents a saturated mono- or bicyclic terpene hydrocarbon radical having 10 carbon atoms or a bicyclic hydrocarbon radical having 11 carbon atoms, of the formula b

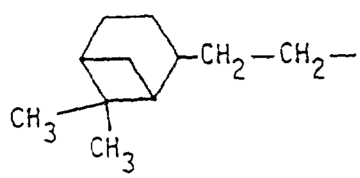

Z represents oxygen or, if $R^3$ denotes a radical of the formula b, also represents sulphur,

and their physiologically tolerable acid addition salts, characterised in that

a) for the preparation of compounds of the general formula Ia

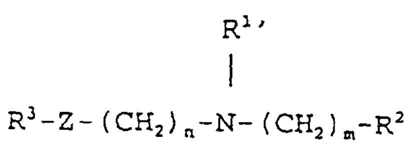

(Ia)

in which n, m, $R^2$, $R^3$ and Z have the above meaning and $R^{1'}$ denotes alkyl with 1 - 4 carbon atoms, compounds of the general formula II

$$R^{1'}$$
$$|$$
$$R^3-Z-(CH_2)_n-N-(CH_2)_m-X \qquad (II)$$

in which n, m, $R^{1'}$, $R^3$ and Z have the above meaning and X represents a removable leaving group Y or, for the preparation of those compounds in which $R^2$ represents an $OR^4$ group, can also represent hydroxyl, are reacted with compounds of the general formula III

$$H-R^2 \qquad (III)$$

in which $R^2$ has the above meaning, or if X is hydroxyl, also with a compound of the general formula III'

$$Y-R^4 \qquad (III')$$

in which $R^4$ has the above meaning and Y represents a removable leaving group, or

b) for the preparation of compounds of the general formula I, compounds of the general formula IV

$$R^1$$
$$|$$
$$R^3-Z-(CH_2)_{n-1}-CO-N-(CH_2)_{m-1}-D \qquad (IV)$$

in which n, m, $R^1$, $R^3$ and Z have the above meaning and D represents the radical $-CH_2-N_3$, an $R_2-CH_2$ group in which $R_2$ has the above meaning, or an

$$R^5$$
$$/$$
$$N-CO$$
$$\backslash$$
$$R^6$$

group, in which $R^5$ and $R^6$ have the above meaning, are reduced, or

c) for the preparation of compounds of the general formula Ib

$$R^{1'}$$
$$|$$
$$R^3-Z-(CH_2)_n-N-(CH_2)_m-R^{2'} \qquad (Ib)$$

in which n, m, $R^{1'}$, $R^3$ and Z have the above meaning and $R^{2'}$ has the meaning given for $R^2$ with the exception of radicals containing an NH function, compounds of the general formula V

$$R^3-Z-(CH_2)_n-Q \qquad (V)$$

in which n, $R^3$ and Z have the above meaning and Q represents an

$$R^{1'}$$
$$/$$
$$N \quad \text{radical,}$$
$$\backslash$$
$$H$$

in which $R^{1'}$ has the above meaning, or represents a removable leaving group Y, are reacted with compounds of the general formula VI

$$Q'-(CH_2)_m-R^{2'} \qquad (VI)$$

in which m and $R^{2'}$ have the above meaning and Q' represents a removable leaving group Y if Q denotes the radical

$$R^{1'}$$
$$/$$
$$-N \quad \text{or}$$
$$\backslash$$
$$H$$

Q' represents the radical

$$-N(R^{1'})H$$

if Q denotes a removable leaving group Y, or

d) for the preparation of compounds of the general formula Ic

$R^{1'}$
|
$R^3-Z-(CH_2)_{n'}-N-(CH_2)_{m'}-R^{2'}$ (Ic)

in which $R^{1'}$, $R^{2'}$, $R^3$ and Z have the above meaning and n' and m' have the meaning given for n and m, where, however, if $R^{2'}$ represents an $NR^5R^6$ radical, n' and m' together cannot be 4, compounds of the general formula VII

$$R^3\text{-}Z\text{-}H \qquad \text{(VII)}$$

in which $R^3$ and Z have the above meaning, are reacted with compounds of the general formula VIII

$R^{1'}$
|
$Y-(CH_2)_{n'}-N-(CH_2)_{m'}-R^{2'}$ (VIII)

in which n', m', $R^{1'}$, $R^{2'}$ and Y have the above meaning, or

e) for the preparation of compounds of the general formula Id

$R^{1'}$
|
$R^3-Z-(CH_2)_n-N-(CH_2)_m-NH_2$ (Id)

in which n, m, $R^{1'}$, $R^3$ and Z have the above meaning, in compounds of the general formula IX

$R^{1'}$
|
$R^3-Z-(CH_2)_n-N-(CH_2)_m-A$ (IX)

in which n, m, $R^{1'}$, $R^3$ and Z have the above meaning and A denotes an azide or phthalimide radical, the radical A is converted into the $NH_2$ group, or

f) for the preparation of compounds of the general formula Ie

H
|
$R^3-O-(CH_2)_n-N-(CH_2)_m-R^{2''}$ (Ie)

in which n, m and $R^3$ have the above meaning and $R^{2''}$ has the meaning given for $R^2$ with the exception of radicals containing an optionally substituted benzyl group, the group B is removed by hydrogenolysis from compounds of the general formula X

B
|
$R^3-O-(CH_2)_n-N-(CH_2)_m-R^{2''}$ (X)

in which n, m, $R^{2''}$ and $R^3$ have the above meaning and B represents a group which is removable by hydrogenolysis, and, if desired, in resulting compounds of the general formula I, in which $R^1$ denotes hydrogen, an alkyl radical $R^{1'}$ with 1 - 4 carbon atoms or, in resulting compounds of the formula I in which $R^1$ is alkyl with 1 - 4 carbon atoms and $R^2$ contains a free NH function, an alkyl radical $R^{5'}$ with 1 - 4 carbon atoms or a phenyl-$C_1$-$C_3$-alkyl group $R^{5'}$ which is optionally substituted by 1 - 3 substituents from the group alkyl with 1 - 4 carbon atoms, alkoxy with 1 - 4 carbon atoms, halogen, trifluoromethyl and alkylenedioxy with 1 - 2 carbon atoms, is introduced and, if desired, free compounds of the formula I are converted into their acid addition salts or the acid addition salts are converted into the free compounds of the formula I.

**2.** Process for the preparation of compounds according to Claim 1, characterised in that $R^3$ represents a mono- or bicyclic hydrocarbon radical having 10 or 11 carbon atoms from the group 1-methyl-4-isopropylcyclohex-3-yl (= menthyl) of the formula a, 2-(6,6-dimethylbicyclo[3.1.1]hept-2-yl)ethyl (= dihydronopyl) of the formula b, 6,6-dimethylbicyclo-[3.1.1]hept-2-ylmethyl (= myrtanyl) of the formula c, 1,3,3-trimethylbicyclo[2.2.1]hept-2-yl (= fenchyl) of the formula d and 1,7,7-trimethylbicyclo[2.2.1]hept-2-yl (= bornyl) of the formula e

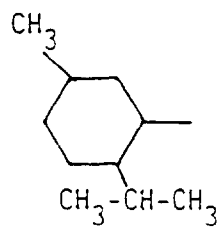

a

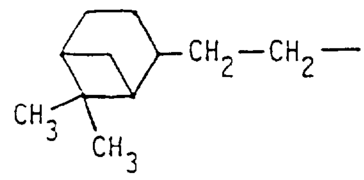

b

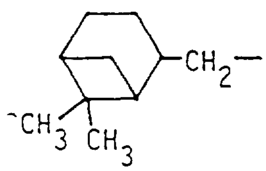

c

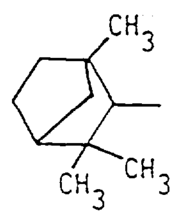

d

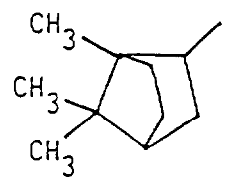

e

**3.** Process for the preparation of compounds according to Claim 2, characterised in that $R^3$ represents a 1-methyl-4-isopropylcyclohex-3-yl radical or a 2-(6,6-dimethylbicyclo[3.1.1]hept-2-yl)ethyl radical.

**4.** Process for the preparation of compounds according to Claim 3, characterised in that the radical $R^3$ represents a 1-methyl-4-isopropylcyclohex-3-yl radical which is present predominantly in the 1R,3R,4S configuration or the 1S, 3S,4R configuration.

**5.** Process for the preparation of compounds according to one of the above claims, characterised in that $R^2$ represents an

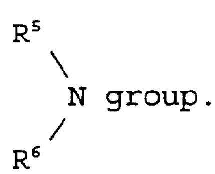

**6.** Process for the preparation of compounds according to Claim 5, characterised in that $R^5$ denotes alkyl with 1 - 4 carbon atoms or a phenyl-$C_1$-$C_2$-alkyl group optionally substituted in the phenyl ring by 1 - 3 substituents from the group alkyl with 1 - 4 carbon atoms, alkoxy with 1 - 4 carbon atoms, halogen, trifluoromethyl and $C_1$-$C_2$-alkylenedioxy and $R^6$ denotes a phenyl-$C_1$-$C_2$-alkyl group which is optionally substituted in the phenyl ring by 1 - 3 substituents from the group alkyl with 1 - 4 carbon atoms, alkoxy with 1 - 4 carbon atoms, halogen, trifluoromethyl and $C_1$-$C_2$-alkylenedioxy, or $R^5$ and $R^6$, together with the nitrogen atom to which they are bonded, represent a 5- or 6-membered saturated heterocycle which optionally contains oxygen as a second hetero atom.

## Revendications

### Revendications pour les Etats contractants suivants : AT, BE, CH, DE, DK, FR, GB, IT, LI, LU, NL, SE

**1.** Composés de la formule générale I

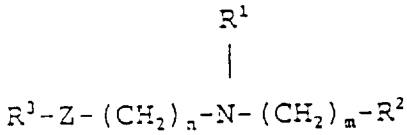

I

dans laquelle

| | |
|---|---|
| n | représente 2 à 5, m 2 à 6, |
| $R^1$ | de l'hydrogène ou un alkyle avec 1 à 4 atomes de carbone, |
| $R^2$ | un groupe $OR^4$, dans lequel $R^4$ signifie un alkyle avec 1 à 4 atomes de carbone ou un groupe phényle ou phényl-$C_1$-$C_3$-alkyle substitué, le cas échéant, dans le cycle phénylique, par 1 à 3 substituants du groupe comportant les groupes alkyles avec 1 à 4 atomes de carbone, les groupes alkoxy avec 1 à 4 atomes de carbone, les halogènes et les groupes alkylènedioxy avec 1 à 2 atomes de carbone, ou |
| $R^2$ | représente un groupe |

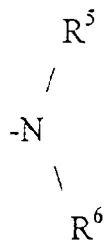

dans lequel

| | |
|---|---|
| $R^5$ | signifie l'hydrogène, un alkyle avec 1 à 4 atomes de carbone ou un groupe phényle ou phényl-$C_1$-$C_3$-alkyle substitué le cas échéant dans le cycle phénylique, par 1 à 3 substituants du groupe comportant les groupes alkyles avec 1 à 4 atomes de carbone, les groupes alkoxy avec 1 à 4 atomes de carbone, les halogènes, le trifluorométhyle et les groupes alkylènedioxy avec 1 à 2 atomes de carbone et |
| $R^6$ | l'hydrogène, un alkyle avec 1 à 4 atomes de carbone ou un groupe phényle ou phényl-$C_1$-$C_3$-alkyle substitué le cas échéant dans le cycle phénylique par 1 à 3 substituants du groupe comportant les groupes alkyles avec 1 à 4 atomes de carbone, les groupes alkoxy avec 1 à 4 atomes de carbone, les halogènes, le trifluorométhyle et les groupes alkylènedioxy avec 1 à 2 atomes de carbone ou |
| $R^5$ et $R^6$ | représentent ensemble avec l'atome d'azote, auquel ils sont liés, un hétérocycle saturé à 5 ou 6 pièces qui peut, le cas échéant, contenir un second hétéroatome du groupe de l'oxygène et de N-$R^7$, dans lequel $R^7$ signifie un alkyle avec 1 à 4 atomes de carbone ou le benzyle, |
| $R^3$ | représente un reste d'hydrocarbure terpénique monocyclique ou bicyclique saturé avec 10 atomes de carbone ou un reste d'hydrocarbure bicyclique avec 11 atomes de carbone de la formule b |

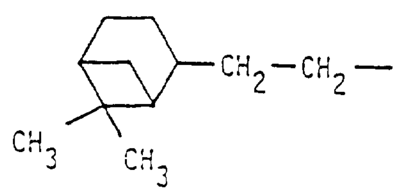

| | |
|---|---|
| Z | l'oxygène ou, au cas où $R^3$ signifie un reste de la formule b, également le soufre, |

et leurs sels d'addition d'acide compatibles physiologiquement.

**2.** Composés selon la revendication 1, caractérisés en ce que $R^3$ représente un reste d'hydrocarbure monocyclique ou bicyclique avec 10 ou 11 atomes de carbone du groupe 1-méthyl-4-isopropylcyclohex-3-yle (= menthyle) de la formule a, 2-(6,6-diméthylbicydo[3.1.1]hept-2-yl)éthyle (= dihydronopyle) de la formule b, 6,6-diméthylbicyclo [3.1.1]hept-2-yhnéthyle (= myrtanyle) de la formule c, 1,3,3-triméthylbicyclo[2.2.1]hept-2-yle (= fenchyle) de la formule d et 1,7,7-triméthylbicyclo[2.2.1]hept-2-yle (= bornyle) de la formule e.

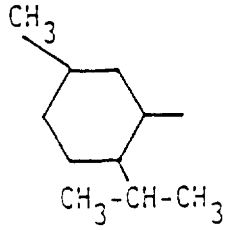

a

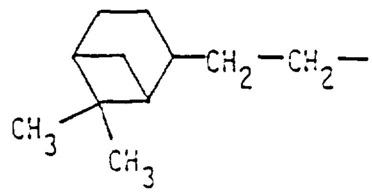

b

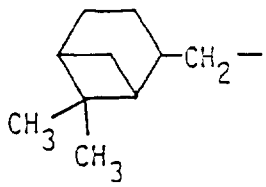

c

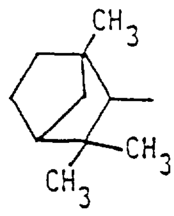

d

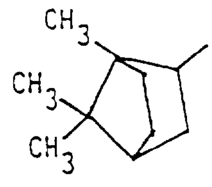

e

**3.** Composés selon la revendication 2, caractérisés en ce que $R^3$ représente un reste 1-méthyl-4-isopropylcyclohex-3-yle ou un reste 2-(6,6-diméthylbicyclo[3.1.1]hept]2-yl)éthyle.

**4.** Composés selon la revendication 3, caractérisés en ce que le reste $R^3$ représente un reste 1-méthyl-4-isopropyl-cyclohex-3-yle qui existe de manière prépondérante dans la configuration 1R, 3R, 4S ou la configuration 1S, 3S, 4R.

**5.** Composés selon l'une des revendications précédentes, caractérisés en ce que $R^2$ représente un groupe

$R^5$
/
-N
\
$R^6$

**6.** Composés selon la revendication 5, caractérisés en ce que $R^5$ représente un alkyle avec 1 à 4 atomes de carbone ou un groupe phényl-$C_1$-$C_2$-alkyle substitué le cas échéant dans le cycle phénylique par 1 à 3 substituants du groupe comportant les groupes alkyles avec 1 à 4 atomes de carbone, les groupes alkoxy avec 1 à 4 atomes de carbone, les halogènes, le trifluorométhyle et le groupe $C_1$-$C_2$-alkylènedioxy et $R^6$ un groupe phényl-$C_1$-$C_2$-alkyle substitué le cas échéant dans le cycle phénylique par 1 à 3 substituants du groupe comportant les groupes alkyles avec 1 à 4 atomes de carbone, les groupes alkoxy avec 1 à 4 atomes de carbone, les halogènes, le trifluorométhyle et le groupe $C_1$-$C_2$-alkylénedioxy, ou $R^5$ et $R^6$ ensemble avec l'atome d'azote, auquel ils sont liés, représentent un hétérocycle saturé à 5 ou 6 pièces qui contient, le cas échéant, de l'oxygène comme second hétéroatome.

**7.** Médicament contenant une quantité pharmacologiquement efficace d'un composé selon la revendication 1 et des substances auxiliaires et/ou porteuses pharmaceutiques usuelles.

**8.** Procédé pour la préparation de composés de la formule générale I

$R^1$
|
$R^3-Z-(CH_2)_n-N-(CH_2)_m-R^2$ (I)

dans laquelle n, m, $R^1$, $R^2$, $R^3$ et Z possèdent la signification indiquée dans la revendication 1 et leurs sels d'addition d'acide physiologiquement compatibles, caractérisé en ce que,

a) pour la préparation de composés de la formule générale Ia

$$\begin{array}{c} R^{1'} \\ | \\ R^3-Z-(CH_2)_n-N-(CH_2)_m-R^2 \end{array} \qquad (Ia)$$

dans laquelle n, m, $R^2$, $R^3$ et Z possèdent la signification ci-dessus et $R^{1'}$ signifie un alkyle avec 1-4 atomes de carbone, on fait réagir des composés de la formule générale II

$$\begin{array}{c} R^{1'} \\ | \\ R^3-Z-(CH_2)_n-N-(CH_2)_m-X \end{array} \qquad (II)$$

dans laquelle n, m, $R^{1'}$, $R^3$ et Z possèdent la signification ci-dessus et X représente un groupe partant éliminable Y ou, pour la préparation de composés dans lesquels $R^2$ représente un groupe $OR^4$, peut aussi représenter un groupe hydroxyle, avec des composés de la formule générale III

$$H-R^2 \qquad (III)$$

dans laquelle $R^2$ possède la signification ci-dessus, ou au cas où X est un groupe hydroxyle, également avec un composé de la formule générale III',

$$Y-R^4 \qquad (III')$$

dans laquelle $R^4$ possède la signification ci-dessus et Y représente un groupe partant éliminable, ou

b) pour la préparation de composés de la formule générale I, on réduit des composés de la formule générale IV

$$\begin{array}{c} R^{1} \\ | \\ R^3-Z-(CH_2)_{n-1}-CO-N-(CH_2)_{m-1}-D \end{array} \qquad (IV)$$

dans laquelle n, m, $R^1$, $R^3$ et Z possèdent la signification ci-dessus et D représente le reste $-CH_2-N_3$, un groupe $R_2-CH_2-$, dans lequel $R_2$ possède la signification ci-dessus, ou représente un groupe

$$\begin{array}{l} \quad R^5 \\ \ / \\ N-CO- \\ \ \backslash \\ \quad R^6 \end{array}$$

dans lequel $R^5$ et $R^6$ possèdent la signification ci-dessus, ou

c) pour la préparation de composés de la formule générale Ib

$$\begin{array}{c} R^{1'} \\ | \\ R^3-Z-(CH_2)_n-N-(CH_2)_m-R^{2'} \end{array} \qquad (Ib)$$

dans laquelle n, m, $R^{1'}$, $R^3$ et Z possèdent la signification ci-dessus et $R^{2'}$ possède la signification indiquée pour $R^2$ à l'exception de restes contenant une fonction NH, on fait réagir des composés de la formule générale V

$$R^{3-}Z-(CH_2)_n-Q \qquad (V)$$

dans laquelle n, $R^3$ et Z possèdent la signification ci-dessus et Q représente un reste

$$\begin{array}{l} \quad R^{1'} \\ \ / \\ N- \\ \ \backslash \\ \quad H \end{array}$$

dans lequel $R^{1'}$ possède la signification ci-dessus ou représente un groupe partant éliminable Y, avec des composés de la formule générale VI

$$Q'\text{-}(CH_2)_m\text{-}R^{2'} \qquad (VI)$$

dans laquelle m et $R^{2'}$ possèdent la signification ci-dessus et Q' représente un groupe partant éliminable Y, au cas où Q signifie le reste

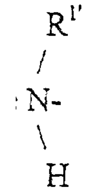

ou Q' représente le reste

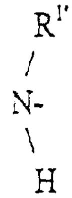

au cas où Q signifie un groupe partant éliminable Y, ou

d) pour la préparation de composés de la formule générale Ic

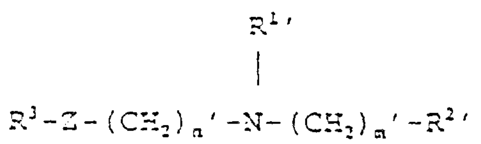

(Ic)

dans laquelle $R^{1'}$, $R^{2'}$, $R^3$ et Z possèdent la signification ci-dessus et n' et m' la signification indiquée pour n et m, cependant que, au cas où $R^{2'}$ représente un reste $NR^5R^6$, n' et m' ensemble ne peuvent pas être 4, on fait réagir des composés de la formule générale VII

$$R^3\text{-}Z\text{-}H \qquad (VII)$$

dans laquelle $R^3$ et Z possèdent la signification ci-dessus, avec des composés de la formule générale VIII

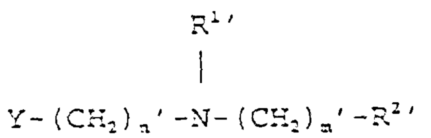

(VIII)

dans laquelle n', m', $R^{1'}$, $R^{2'}$ et Y possèdent la signification ci-dessus, ou

e) pour la préparation de composés de la formule générale Id

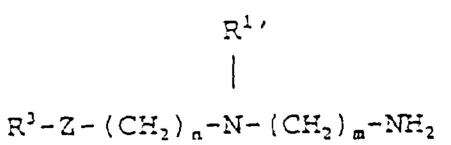

(Id)

dans laquelle n, m, $R^{1'}$, $R^3$ et Z possèdent la signification ci-dessus, on convertit, dans des composés de la formule générale IX

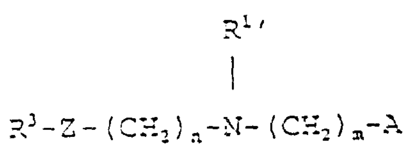

(IX)

dans laquelle n, m, $R^{1'}$, $R^3$ et Z possèdent la signification ci-dessus et A signifie un reste d'azide ou de phtalimide, le reste A en groupe $NH_2$, ou f) pour la préparation de composés de la formule générale Ie

$$\begin{array}{c} H \\ | \\ R^3\text{-}O\text{-}(CH_2)_n\text{-}N\text{-}(CH_2)_m\text{-}R^{2''} \end{array} \qquad (Ie)$$

dans laquelle n, m et $R^3$ possèdent la signification ci-dessus et $R^{2''}$ possède la signification indiquée pour $R^2$ à l'exception de restes contenant un groupe benzylique le cas échéant substitué, on élimine de composés de la formule générale X

$$\begin{array}{c} B \\ | \\ R^3\text{-}O\text{-}(CH_2)_n\text{-}N\text{-}(CH_2)_m\text{-}R^{2''} \end{array} \qquad (X)$$

dans laquelle n, m, $R^{2''}$ et $R^3$ possèdent la signification ci-dessus et B représente un groupe éliminable par hydrogénolyse, le groupe B par hydrogénolyse, et, au cas où on le souhaite, on introduit dans des composés obtenus de la formule générale I, dans laquelle $R^1$ signifie de l'hydrogène, un reste alkyle $R^{1'}$ avec 1 à 4 atomes de carbone ou, dans des composés obtenus de la formule I, dans laquelle $R^1$ est un alkyle avec 1 à 4 atomes de carbone et $R^2$ contient une fonction NH libre, un reste alkyle $R^{5'}$ avec 1 à 4 atomes de carbone ou un groupe phényl-$C_1$-$C_3$-alkyle $R^{5'}$ le cas échéant substitué par 1 à 3 substituants du groupe comportant les groupes alkyles avec 1 à 4 atomes de carbone, les groupes alkoxy avec 1 à 4 atomes de carbone, les halogènes, le trifluorométhyle et les groupes alkylènedioxy avec 1 à 2 atomes de carbone et, au cas où on le souhaite, on transforme des composés libres de la formule I dans leurs sels d'addition d'acide ou les sels d'addition d'acide dans les composés libres de la formule I.

**Revendications pour les Etats contractants suivants : ES, GR**

**1.** Procédé pour la préparation de composés de la formule générale I

$$\begin{array}{c} R^1 \\ | \\ R^3\text{-}Z\text{-}(CH_2)_n\text{-}N\text{-}(CH_2)_m\text{-}R^2 \end{array} \qquad I$$

dans laquelle

n représente 2 à 5, m 2 à 6,

$R^1$ de l'hydrogène ou un alkyle avec 1 à 4 atomes de carbone,

$R^2$ un groupe $OR^4$, dans lequel $R^4$ signifie un alkyle avec 1 à 4 atomes de carbone ou un groupe phényle ou phényl-$C_1$-$C_3$-alkyle substitué, le cas échéant, dans le cycle phénylique, par 1 à 3 substituants du groupe comportant les groupes alkyles avec 1 à 4 atomes de carbone, les groupe alkoxy avec 1 à 4 atomes de carbone, les halogènes et les groupes alkylènedioxy avec 1 à 2 atomes de carbone, ou

$R^2$ représente un groupe

$$\begin{array}{l} \quad R^5 \\ \ / \\ -N \\ \ \backslash \\ \quad R^6 \end{array}$$

dans lequel

$R^5$ signifie l'hydrogène, un alkyle avec 1 à 4 atomes de carbone ou un groupe phényle ou phényl-$C_1$-$C_3$-alkyle substitué le cas échéant dans le cycle phénylique, par 1 à 3 substituants du groupe comportant les groupes alkyles avec 1 à 4 atomes de carbone, les groupes alkoxy avec 1 à 4 atomes de carbone, les halogènes, le trifluorométhyle et les groupes alkylènedioxy avec 1 à 2 atomes de carbone et

$R^6$ l'hydrogène, un alkyle avec 1 à 4 atomes de carbone ou un groupe phényle ou phényl-$C_1$-$C_3$-alkyle

substitué le cas échéant dans le cycle phénylique par 1 à 3 substituants du groupe comportant les groupes alkyles avec 1 à 4 atomes de carbone, les groupes alkoxy avec 1 à 4 atomes de carbone, les halogènes, le trifluorométhyle et les groupes alkylènedioxy avec 1 à 2 atomes de carbone ou

$R^5$ et $R^6$ représentent ensemble avec l'atome d'azote, auquel ils sont liés, un hétérocycle saturé à 5 ou 6 pièces qui peut, le cas échéant, contenir un second hétéroatome du groupe de l'oxygène et de N-$R^7$, dans lequel $R^7$ signifie un alkyle avec 1 à 4 atomes de carbone ou le benzyle,

$R^3$ représente un reste d'hydrocarbure terpénique monocyclique ou bicyclique saturé avec 10 atomes de carbone ou un reste d'hydrocarbure bicyclique avec 11 atomes de carbone de la formule b

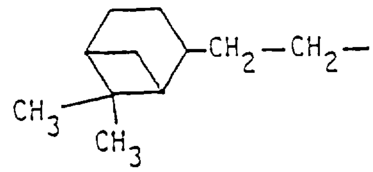

Z l'oxygène ou, au cas où $R^3$ signifie un reste de la formule b, également le soufre,

et leurs sels d'addition d'acide compatibles physiologiquement, caractérisé en ce que,

a) pour la préparation de composés de la formule générale Ia

$$R^3-Z-(CH_2)_n-\underset{\displaystyle R^{1'}}{\overset{}{N}}-(CH_2)_m-R^2 \qquad (Ia)$$

dans laquelle n, m, $R^2$, $R^3$ et Z possèdent la signification ci-dessus et $R^{1'}$ signifie un alkyle avec 1-4 atomes de carbone, on fait réagir des composés de la formule générale II

$$R^3-Z-(CH_2)_n-\underset{\displaystyle R^{1'}}{\overset{}{N}}-(CH_2)_m-X \qquad (II)$$

dans laquelle n, m, $R^{1'}$, $R^3$ et Z possèdent la signification ci-dessus et X représente un groupe partant éliminable Y ou, pour la préparation de composés dans lesquels $R^2$ représente un groupe $OR^4$, peut aussi représenter un groupe hydroxyle, avec des composés de la formule générale III

$$H-R^2 \qquad (III)$$

dans laquelle $R^2$ possède la signification ci-dessus, ou au cas où X est un groupe hydroxyle, également avec un composé de la formule générale III',

$$Y-R^4 \qquad (III')$$

dans laquelle $R^4$ possède la signification ci-dessus et Y représente un groupe partant éliminable, ou

b) pour la préparation de composés de la formule générale I, on réduit des composés de la formule générale IV

$$R^3-Z-(CH_2)_{n-1}-CO-\underset{\displaystyle R^{1}}{\overset{}{N}}-(CH_2)_{m-1}-D \qquad (IV)$$

dans laquelle n, m, $R^1$, $R^3$ et Z possèdent la signification ci-dessus et D représente le reste $-CH_2-N_3$, un groupe $R_2-CH_2-$, dans lequel $R_2$ possède la signification ci-dessus, ou représente un groupe

$R^5$
/
N-CO -
\
$R^6$

dans lequel $R^5$ et $R^6$ possèdent la signification ci-dessus, ou

c) pour la préparation de composés de la formule générale Ib

$$\begin{array}{c} \qquad\qquad\qquad R^{1'} \\ \qquad\qquad\qquad | \\ R^3-Z-(CH_2)_n-N-(CH_2)_m-R^{2'} \end{array} \qquad (Ib)$$

dans laquelle n, m, $R^{1'}$, $R^3$ et Z possèdent la signification ci-dessus et $R^{2'}$ possède la signification indiquée pour $R_2$ à l'exception de restes contenant une fonction NH, on fait réagir des composés de la formule générale V

$$R^3\text{-}Z\text{-}(CH_2)_n\text{-}Q \qquad (v)$$

dans laquelle n, $R^3$ et Z possèdent la signification ci-dessus et Q représente un reste

$$\begin{array}{c} R^{1'} \\ / \\ N- \\ \backslash \\ H \end{array}$$

dans lequel $R^{1'}$ possède la signification ci-dessus ou représente un groupe partant éliminable Y, avec des composés de la formule générale VI

$$Q'\text{-}(CH_2)_m\text{-}R^{2'} \qquad (VI)$$

dans laquelle m et $R^{2'}$ possèdent la signification ci-dessus et Q' représente un groupe partant éliminable Y, au cas où Q signifie le reste

$$\begin{array}{c} R^{1'} \\ / \\ N- \\ \backslash \\ H \end{array}$$

ou Q' représente le reste

$$\begin{array}{c} R^{1'} \\ / \\ N- \\ \backslash \\ H \end{array}$$

au cas où Q signifie un groupe partant éliminable Y, ou

d) pour la préparation de composés de la formule générale Ic

$$\begin{array}{c} \qquad\qquad\qquad R^{1'} \\ \qquad\qquad\qquad | \\ R^3-Z-(CH_2)_{n'}-N-(CH_2)_{m'}-R^{2'} \end{array} \qquad (Ic)$$

dans laquelle $R^{1'}$, $R^{2'}$, $R^3$ et Z possèdent la signification ci-dessus et n' et m' la signification indiquée pour n et m, cependant que, au cas où $R^{2'}$ représente un reste $NR^5R^6$, n' et m' ensemble ne peuvent pas être 4, on fait réagir des composés de la formule générale VII

$$R^3\text{-}Z\text{-}H \qquad (VII)$$

dans laquelle $R^3$ et Z possèdent la signification ci-dessus, avec des composés de la formule générale VIII

$$Y-(CH_2)_{n'}-\overset{\displaystyle R^{1'}}{\underset{|}{N}}-(CH_2)_{m'}-R^{2'} \qquad (VIII)$$

dans laquelle n', m', $R^{1'}$, $R^{2'}$ et Y possèdent la signification ci-dessus, ou

e) pour la préparation de composés de la formule générale Id

$$R^3-Z-(CH_2)_n-\overset{\displaystyle R^{1'}}{\underset{|}{N}}-(CH_2)_m-NH_2 \qquad (Id)$$

dans laquelle n, m, $R^{1'}$, $R^3$ et Z possèdent la signification ci-dessus, on transforme, dans des composés de la formule générale IX

$$R^3-Z-(CH_2)_n-\overset{\displaystyle R^{1'}}{\underset{|}{N}}-(CH_2)_m-A \qquad (IX)$$

dans laquelle n, m, $R^{1'}$, $R^3$ et Z possèdent la signification ci-dessus et A signifie un reste d'azide ou de phtalimide, le reste A en groupe $NH_2$, ou

f) pour la préparation de composés de la formule générale le

$$R^3-O-(CH_2)_n-\overset{\displaystyle H}{\underset{|}{N}}-(CH_2)_m-R^{2''} \qquad (Ie)$$

dans laquelle n, m et $R^3$ possèdent la signification ci-dessus et $R^{2''}$ possède la signification indiquée pour $R^2$ à l'exception de restes contenant un groupe benzylique le cas échéant substitué, on élimine de composés de la formule générale X

$$R^3-O-(CH_2)_n-\overset{\displaystyle B}{\underset{|}{N}}-(CH_2)_m-R^{2''} \qquad (X)$$

dans laquelle n, m, $R^{2''}$ et $R^3$ possèdent la signification ci-dessus et B représente un groupe éliminable par hydrogénolyse, le groupe B par hydrogénolyse, et, au cas où on le souhaite, on introduit dans des composés obtenus de la formule générale I, dans laquelle $R^1$ signifie de l'hydrogène, un reste alkyle $R^{1'}$ avec 1 à 4 atomes de carbone ou, dans des composés obtenus de la formule I, dans laquelle $R^1$ est un alkyle avec 1 à 4 atomes de carbone et $R^2$ contient une fonction NH libre, un reste alkyle $R^{5'}$ avec 1 à 4 atomes de carbone ou un groupe phényl-$C_1$-$C_3$-alkyle $R^{5'}$ le cas échéant substitué par 1 à 3 substituants du groupe comportant les groupes alkyles avec 1 à 4 atomes de carbone, les groupes alkoxy avec 1 à 4 atomes de carbone, les halogènes, le trifluorométhyle et les groupes alkylènedioxy avec 1 à 2 atomes de carbone et, au cas où on le souhaite, on transforme des composés libres de la formule I dans leurs sels d'addition d'acide ou les sels d'addition d'acide dans les composés libres de la formule I.

**2.** Procédé pour la préparation de composés selon la revendication 1, caractérisé en ce que $R^3$ représente un reste d'hydrocarbure monocyclique ou bicyclique avec 10 ou 11 atomes de carbone du loupe 1-méthyl-4-isopropylcyclohex-3-yle (= menthyle) de la formule a, 2-(6,6-diméthylbicyclo[3.1.1]hept-2-yl)éthyle (= dihydronopyle) de la formule b, 6,6-diméthylbicyclo[3.1.1]hept-2-ylméthyle (= myrtanyle) de la formule c, 1,3,3-triméthylbicyclo[2.2.1]hept-2-yle (= fenchyle) de la formule d et 1,7,7-triméthylbicyclo[2.2.1]hept-2-yle (= bornyle) de la formule e.

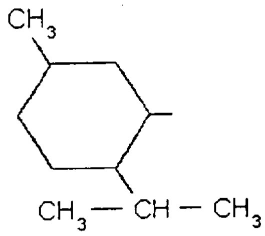

a

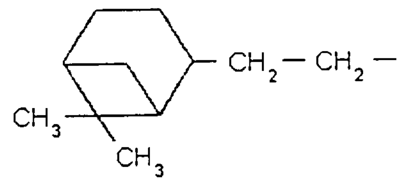

b

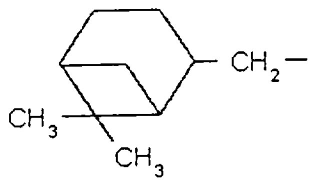

c

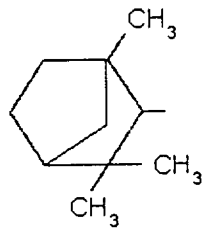

d

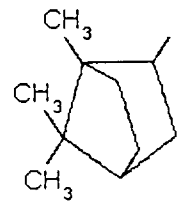

e

**3.** Procédé pour la préparation de composés selon la revendication 2, caractérisé en ce que $R^3$ représente un reste 1-méthyl-4-isopropylcyclohex-3-yle ou un reste 2-(6,6-diméthylbicyclo[3.1.1]hept-2-yl)éthyle.

**4.** Procédé pour la préparation de composés selon la revendication 3, caractérisé en ce que le reste $R^3$ représente un reste 1-méthyl-4-isopropylcyclohex-3-yle qui existe de manière prépondérante dans la configuration 1R, 3R, 4S ou la configuration 1S, 3S, 4R.

**5.** Procédé pour la préparation de composés selon l'une des revendications précédentes, caractérisé en ce que $R^2$ représente un groupe

$R^5$
/
-N .
\
$R^6$

**6.** Procédé pour la préparation de composés selon la revendication 5, caractérisé en ce que $R^5$ représente un alkyle avec 1 à 4 atomes de carbone ou un groupe phényl-$C_1$-$C_2$-alkyle substitué le cas échéant dans le cycle phénylique par 1 à 3 substituants du groupe comportant les groupes alkyles avec 1 à 4 atomes de carbone, les groupes alkoxy avec 1 à 4 atomes de carbone, les halogènes, le trifluorométhyle et le groupe $C_1$-$C_2$-alkylènedioxy et $R^6$ un groupe phényl-$C_1$-$C_2$-alkyle substitué le cas échéant dans le cycle phénylique par 1 à 3 substituants du groupe comportant les groupes alkyles avec 1 à 4 atomes de carbone, les groupes alkoxy avec 1 à 4 atomes de carbone, les halogènes, le trifluorométhyle et le groupe $C_1$-$C_2$-alkylènedioxy, ou $R^5$ et $R^6$ représentent ensemble avec l'atome d'azote, auquel ils sont liés, un hétérocycle saturé à 5 ou 6 pièces qui contient, le cas échéant, de l'oxygène comme second hétéroatome.